# EUROPEAN PATENT SPECIFICATION

(11) **EP 3 169 781 B1**
(45) Date of publication and mention of the grant of the patent: **08.04.2020**
(21) Application number: 15747649.0
(22) Date of filing: 15.07.2015
(51) Int. Cl.: C12N 15/10

(54) **COMPOSITIONS AND METHODS FOR NUCLEIC ACID ASSEMBLY**
ZUSAMMENSETZUNGEN UND VERFAHREN ZUR NUKLEINSÄUREASSEMBLIERUNG
COMPOSITIONS ET MÉTHODES D'ASSEMBLAGE D'ACIDES NUCLÉIQUES

(30) Priority: 15.07.2014 US 201462024650 P
(43) Date of publication of application: 24.05.2017
(73) Proprietor: Life Technologies Corporation, Carlsbad, CA 92008 (US)
(72) Inventor: LIANG, Xiquan, Escondido, California 92026 (US); BAEK, Chang-Ho, San Diego, California 92130 (US); PENG, Lansha, Poway, California 92064 (US); KATZEN, Federico, San Marcos, California 92078 (US)
(74) Representative: Ludwig, Christine Andrea
(86) International application number: PCT/US2015/040606
(87) International publication number: WO 2016/011174

(56) References cited:
- WO-A1-90/15065
- WO-A2-2009/103027
- WO-A2-2014/153188
- GIBSON DANIEL G: "Enzymatic assembly of overlapping DNA fragments", METHODS IN ENZYMOLOGY, ACADEM. PRESS, USA, vol. 498, 1 January 2011 (2011-01-01), pages 349-361, XP009179862, ISSN: 1557-7988

## Description

### FIELD

The present disclosure generally relates to compositions and methods for the assembly of nucleic acid molecules into larger nucleic acid molecules. Provided are compositions and methods for seamless connection of nucleic acid molecules, in many instances, with high sequence fidelity.

### BACKGROUND

As genetic engineering has developed, a need for the generation of larger and larger nucleic acid molecules has also developed. In many instances, nucleic acid assembly methods involve the production of sub-assemblies (e.g., chemically synthesized oligonucleotides), followed by the generation of larger (e.g., annealing of oligonucleotides to form double-stranded nucleic acid molecules) and larger assemblies (e.g., ligation of double-stranded nucleic acid molecules).

The present disclosure generally relates to compositions and methods for efficient assembly of nucleic acid molecules.

### SUMMARY

The present disclosure relates, in part, to compositions and methods for efficient assembly of nucleic acid molecules and the invention is set out in the appended claims. Three aspects, that may be used in combination or separately, are as follows:
1. The use of nuclease resistant regions near the termini (*e.g.*, within 12, 15, 20, 30, 40, or 50 base pairs) of nucleic acid segments to limit digestion of these nucleic acid segments during the formation of single-stranded regions (*e.g*., single-stranded regions designed for hybridization to other nucleic acid segments).
2. The reconstitution of functional nucleic acid elements (*e.g.*, selectable marker, origins or replication, etc.) for the purpose of selecting for correctly assembled nucleic acid molecules.
3. The stopping/inhibition of assembly reaction processes that can affect the stability of nucleic acid molecules prepared during the assembly process.

In some aspects, the invention relates to a method for covalently linking two double-stranded nucleic acid segments having a region of sequence complementarity at their termini, these method comprising: (a) incubating the two double-stranded nucleic acid segments with an exonuclease) under conditions that allow for digestion of termini of the two double-stranded nucleic acid segments to form complementary single-stranded regions on each double-stranded nucleic acid segment and hybridization of the complementary single-stranded regions, wherein each of the two double-stranded nucleic acid segments comprises an exonuclease resistant region within 30 nucleotides of the end of the complementary terminus, wherein the exonuclease resistant region contains a phosphorothioate bond, and (b) covalently connecting at least one strand of the hybridized termini formed in (a) resulting in the linkage of the two double-stranded nucleic acid segments, and wherein the junction between the exonuclease resistant region and the region of hybridization is determined by the region of sequence complementarity between the two double-stranded nucleic acid segments.

Steps (a) and (b), referred to above, may be performed in the same tube and/or at the same time. Further, the two or more nucleic acid segments may be simultaneously contacted with one or more exonuclease and one or more molecule with ligase activity (*e.g*., ligase, topoisomerase, etc.) in step (a). In such instances, the two or more nucleic acid segments may be contacted with the one or more nuclease first, followed by contacting with the one or more molecule ligase activity or the two or more nucleic acid segments with the one or more nuclease and the one or more molecule ligase activity at the same time.

The disclosure also includes compositions and methods in which three or more (e.g., four, five, eight, ten twelve, fifteen, etc.) nucleic acid segments are covalently linked to each other. Further, some of these nucleic acid segments may not contain a nuclease (e.g., exonuclease) resistant region, some may contain a single nuclease resistant region and some may contain two nuclease resistant regions. In most cases, nucleases resistant regions, when present will be within 30 base pairs of a terminus of the nucleic acid segment in which they are present.

In many instances, nucleic acid molecules prepared by methods of the invention will be replicable. Further, many of these replicable nucleic acid molecules will be circular (*e*.*g*., plasmids). Replicable nucleic acid molecules, regardless of whether they are circular, will generally be formed from the assembly of two or more (*e.g*., three, four, five, eight, ten, twelve, etc.) nucleic acid segments. In some instances, methods of the invention employ selection based upon the reconstitution of one or more (*e.g*., two, three, four, etc.) selection marker or one or more (*e.g*., two, three, four, etc.) origin of replication resulting from the linking of different nucleic acid segments. Further selection may result from the formation of a circular nucleic acid molecule, in instances where circularity is required for replication.

The disclosure also relates, in part, to compositions and methods for storing assembled nucleic acid molecules (*e.g*., nucleic acid molecules assembled by method disclosed herein). Stabilization of nucleic acid molecules is often facilitated by the inhibition of nucleic acid assembly activities (*e.g*., nuclease activities). Thus, the disclosure includes methods for the stabilization of nucleic acid molecules associated with the inhibition or elimination of activities (*e.g.*, enzymatic activities) associated with the assembly process. One example is that methods of the disclosure include those involving the partial or full inactivated one or more enzyme contacting assembled nucleic acid molecules. This may be accomplished by the use of enzymatic inhibitors, pH changes, as well as other means.

In some instances, inhibition of enzymatic activity will be mediated by heating. While the temperatures required to inactivate enzymes differ with the particular enzyme or enzymes in the mixture, typically, heating will be to a temperature greater than 65°C *(e.g.,* 70°C, 75°C, 80°C, or 85°C) for at least 10 minutes *(e.g.,* 15 minutes, 20 minutes, 25 minutes, 30 minutes, etc.).

In many instances, after the partial or full inactivated one or more enzyme contacting assembled nucleic acid molecules, the assembled nucleic acid molecules will be stored at a temperature equal to or below 4°C (*e.g.,* -20°C, -30°C, -60°C, or -70°C) for at least 24 hours (*e.g*., 36 hours, two days, five days, seven days, two weeks, three weeks, one month, three months, six months, nine months, one year).

The invention also includes a methods for assembling a double-stranded nucleic acid molecules, these methods comprising: (a) incubating a first double-stranded nucleic acid segment with an exonuclease under conditions that allow for partial digestion of at least one terminus of the first double-stranded nucleic acid segment to form a single-stranded region, wherein the first double-stranded nucleic acid segment contains an exonuclease resistant region within 30 nucleotides of the at least one terminus, wherein the exonuclease resistant region contains a phosphorothioate bond, (b) preparing a reaction mixture containing the digested first nucleic acid segment formed in (a) with an undigested second double-stranded nucleic acid segment under conditions that allow for the hybridization of the single-stranded region of the first nucleic acid segment with a terminus of the undigested second nucleic acid segment with sequence complementarity to form hybridized termini, and (c) covalently connecting at least one strand of the hybridized termini formed in (b), and and wherein the junction between the exonuclease resistant region and the region of hybridization is determined by the region of sequence complementarity between the two double-stranded nucleic acid segments. The second nucleic acid segment of (b) may or may not contain a nuclease resistant region. In many instances, the at least one terminus of the second nucleic acid segment of (b) will contain a single-stranded region with sequence complementarity to the single-stranded region of the first nucleic acid molecules formed in step (a). Further, the nuclease of (a) may is an exonuclease and, more specifically, a 5' to 3' exonuclease or 3" to 5' exonuclease. Additionally, two or more nucleases are present in step (a). Further, the nuclease(s) present may retain partial or full functionality in step (b) or may be partially or fully inactivated.

The invention also includes a methods for assembling a nucleic acid molecules, these methods comprising: (a) incubating two or more double-stranded nucleic acid segments with an exonuclease under conditions that allow for partial digestion of at least one terminus of each of the two or more double-stranded nucleic acid segments to generate single-stranded termini, wherein at least two of the two or more double-stranded nucleic acid segments contain an exonuclease resistant region within 30 nucleotides of at least one of their termini, wherein the exonuclease resistant region contains a phosphorothioate bond, (b) preparing a reaction mixture containing the digested nucleic acid segments prepared in (a) with one or more undigested nucleic acid segment under conditions that allow for the hybridization of termini with sequence complementarity, wherein at least one of the one or more undigested nucleic acid segment has region of sequence complementarity with at least one single-stranded terminus formed in (a), and (c) covalently connecting at least one strand of the hybridized termini formed in (b)and wherein the junction between the exonuclease resistant region and the region of hybridization is determined bv the region of sequence complementarity between the two double-stranded nucleic acid segments.

### BRIEF DESCRIPTION OF THE DRAWINGS

For a more complete understanding of the principles disclosed herein, and the advantages thereof, reference is now made to the following descriptions taken in conjunction with the accompanying drawings, in which:

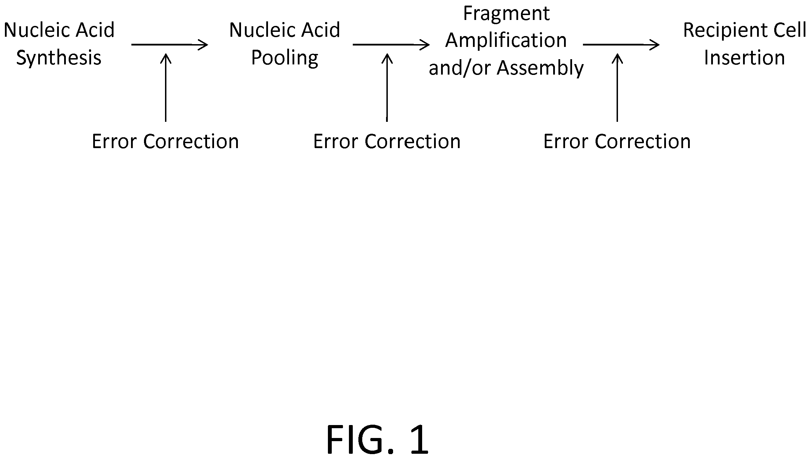

FIG. 1 is a block diagram that shows some components of exemplary work flows. Error correction may be performed at multiple steps within work flows.

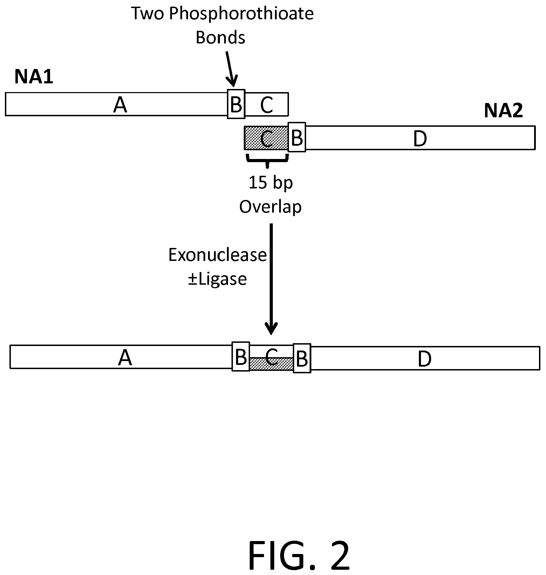

FIG. 2 is a schematic showing two double stranded nucleic acid segments (NA1 and NA2), represented as A-B-C and C-B-D. Region B (a nuclease resistant region), as shown in the diagram, contains two phosphorothioate bonds. Region C in both nucleic acid segments, as shown in the diagram, is fifteen base pairs in length and share sequence complementarity with each other.

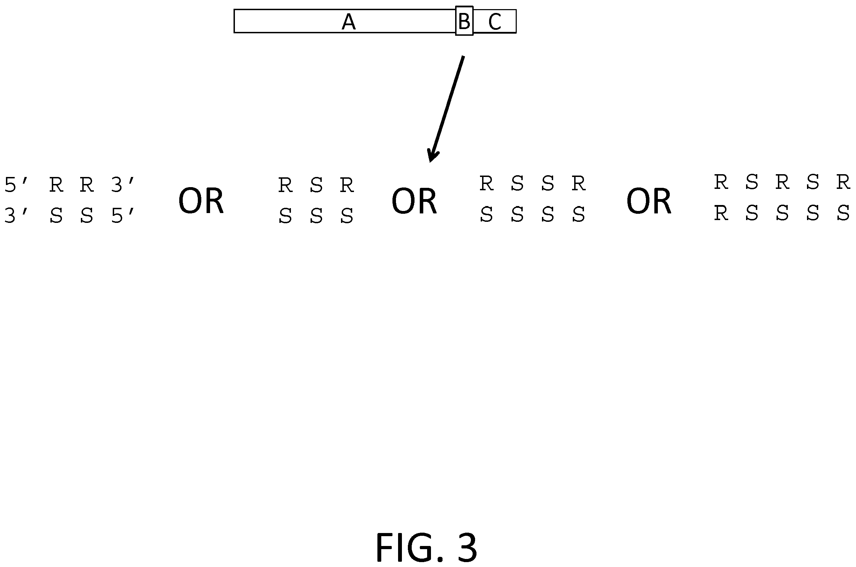

FIG. 3 shows variations of Region B (a nuclease resistant region). R represents a resistant base and S represents a sensitive base. Four variations of Region B are also shown with R and S bases on different strands and having a length of between two and four base pairs.

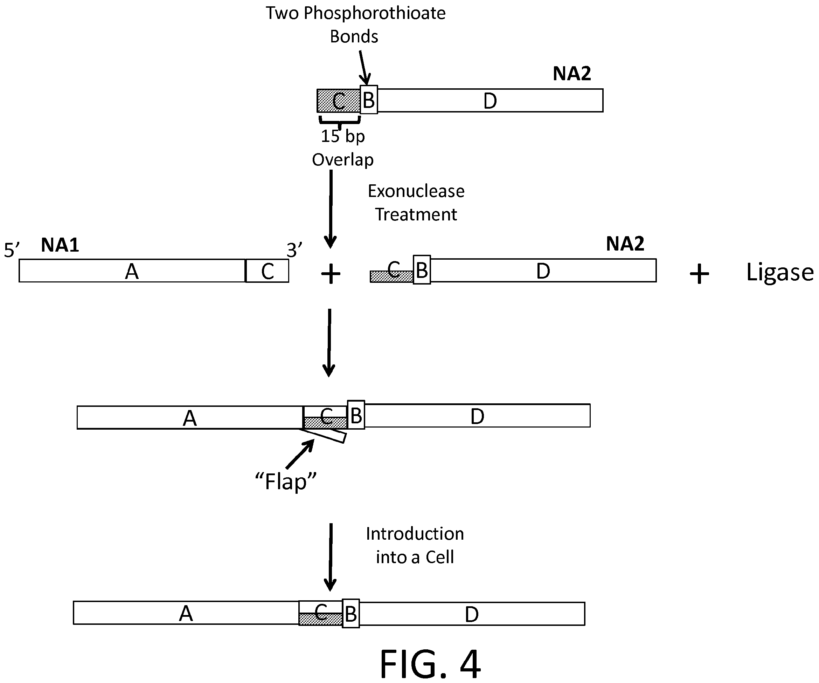

FIG. 4 shows the joining of two nucleic acid segments. One nucleic acid segment (NA1) has no nuclease resistant bases. The other nucleic acid segment (NA2) has a nuclease resistant region (Region B) that contains two phosphorothioate bonds. NA2, but not NA1, is treated with an exonuclease under conditions designed to generate a 15 base pair single-stranded region with sequence complementarity to one terminus of NA1. The result is that, with many of the connected nucleic acid segments, a "flap forms with one strand.

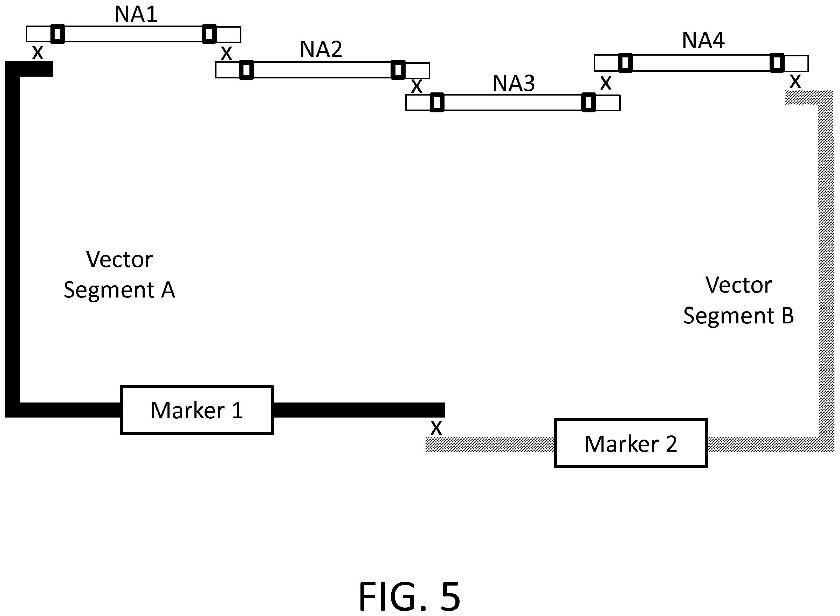

FIG. 5 is a schematic showing six double stranded nucleic acid segments. The two nucleic acid segments shown in black and grey each contain a marker (Marker 1 and Marker 2). The other four nucleic acid segments (numbered 1 through 4) have termini similar to those represented in FIG 2. "X" designations mark regions of sequence homology.

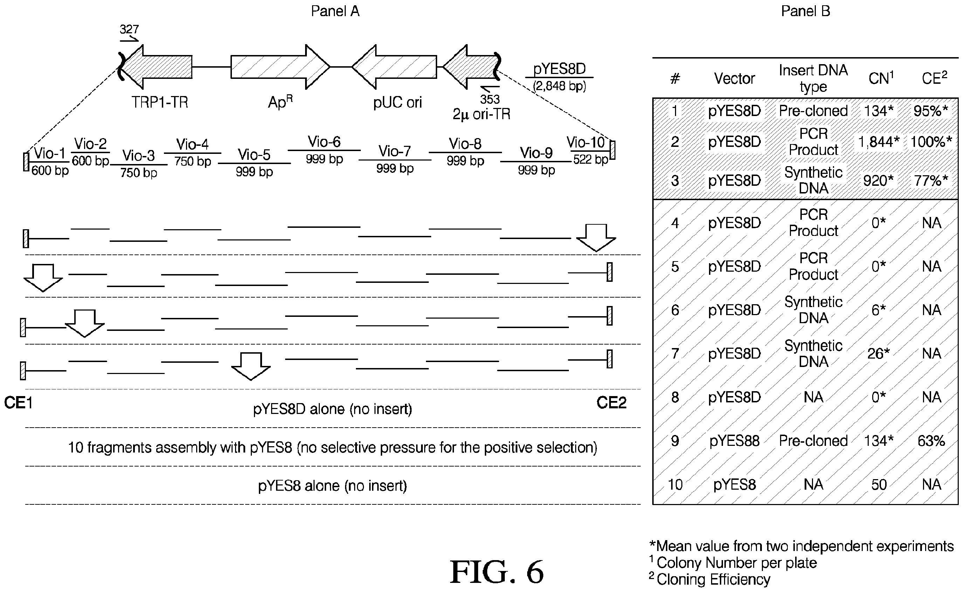

FIG. 6 is a schematic showing the assembly of 10 DNA fragments for violacein synthesis genes (8-kb total insert size) using the positive-selection vector pYES8D in yeast. **Panel A:** Test complete assembly sets using three different types of insert fragments: pre-cloned, PCR- amplified, and synthetic DNA fragments. **Panel B:** Control assembly sets: missing one insert fragment (white downward arrows) at different positions, pYES8D with no insert, complete set but no positive selection, and pYES8 alone. Complement element 1 (CE1) for the TRP1-TR was added to the forward primer for the Vio-1 fragment. CE2 for the 2µ ori-TR was added to the reverse primer for the Vio-10 fragment. Results for colony number and cloning efficiency are summarized in the table at right panel. NA, not applicable.

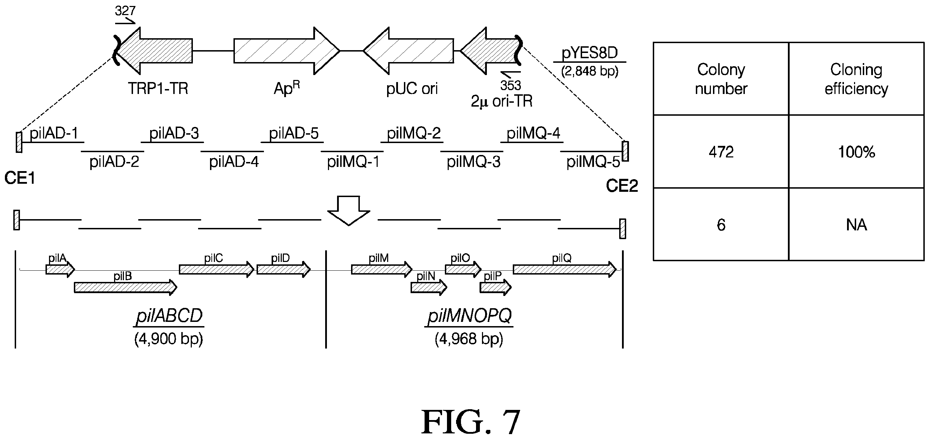

FIG. 7 shows a schematic of an assembly of ten DNA fragments for *V. cholerae pilABCD*/*pilMNOPQ* region (9.9-kb total insert size) using the positive-selection vector pYES8D in yeast. An assembly set missing one insert fragment at pilMQ-1 position was tested as negative control (white downward arrow). Complement element 1 (CE1) for the TRP1-TR was added to the forward primer for the pilAD-1 fragment. CE2 for the 2µ ori-TR was added to the reverse primer for the piIMQ-5 fragment. Results for colony number and cloning efficiency are summarized in the table. NA, not applicable.

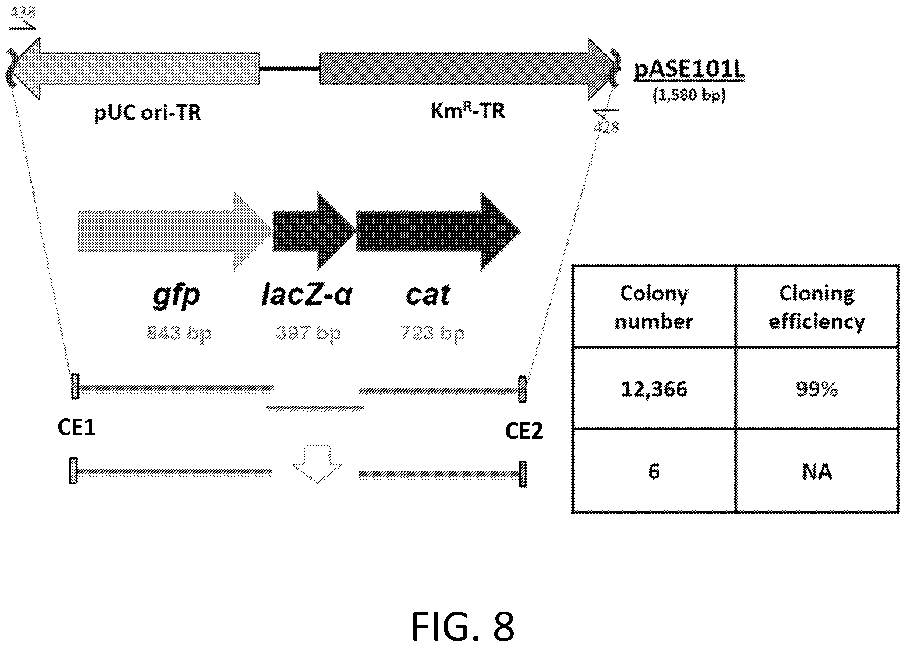

FIG. 8 shows a schematic of a gene assembly using the positive-selection vector pASE101 in *E. coli* using three reporter genes. In particular, the assembly of three reporter DNA fragments (2-kb total insert size) using the positive-selection vector pASE101L in *E. coli* is shown. An assembly set missing one insert fragment at *lacZ-*α position was tested as negative control (white downward arrow). Complement element 1 (CE1) for the truncated pUC ori (pUC ori-TR) was added to the forward primer for the gfp gene fragment. CE2 for the truncated Km^{R} (Km^{R}-TR) was added to the reverse primer for the cat gene fragment. Results for colony number and cloning efficiency are summarized in the table. NA, not applicable.

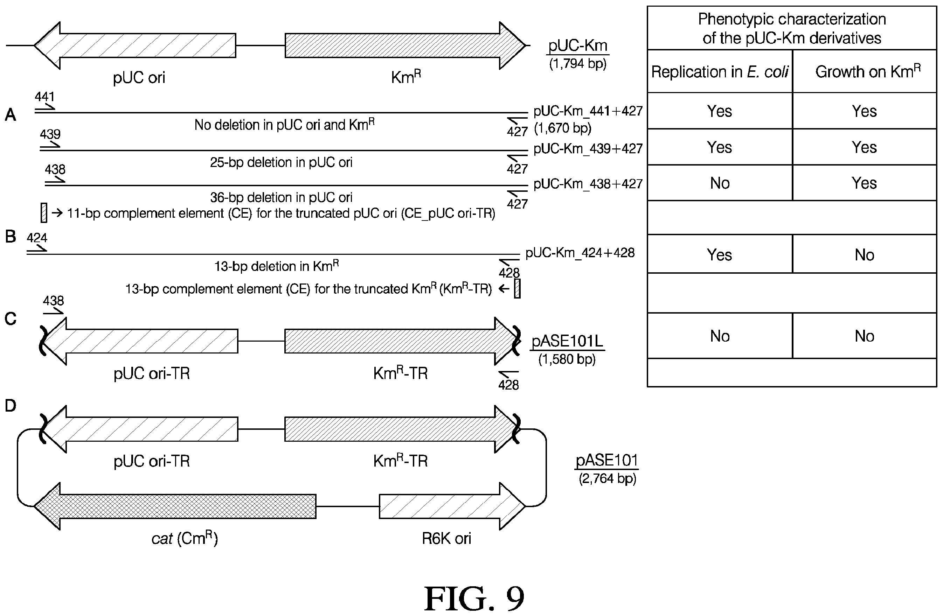

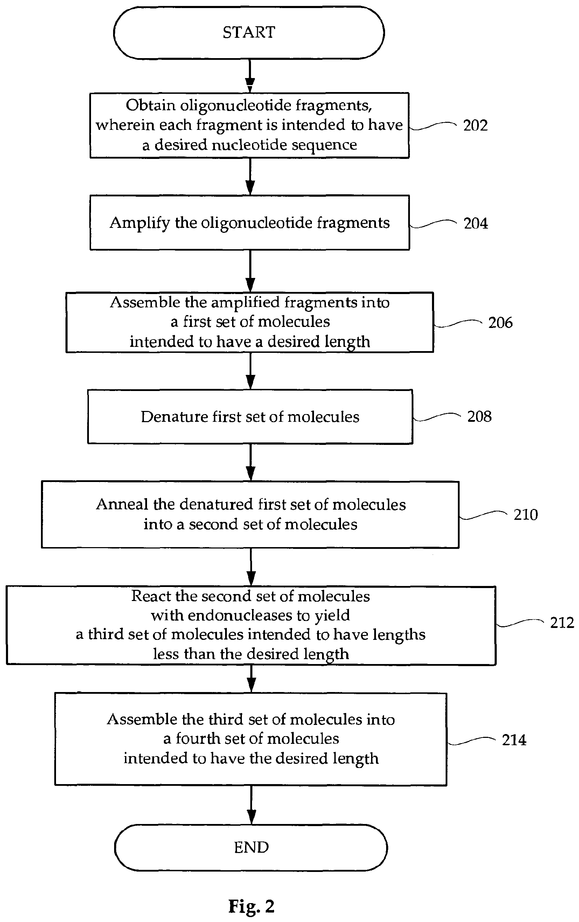

FIG. 9 shows the construction of a positive-selection vector pASE101 for nucleic acid assembly in *E. coli.* **Panel A:** PCR-amplified pUC-Km derivatives to identify complement elements (CE) for the truncated pUC ori (pUC ori-TR). **Panel B:** PCR·amplified pUC·Km derivative to identify CE for the truncated Km^{R}(Km^{R}·TR). **Panel C:** PCR-amplified positive-selection vector pASE101L. **Panel D:** Construction of pASE101 to propagate pASE101L in *E. coli.* PCR products were self-ligated and introduced into E. *coli* strain TOP10 or DH10B-T1 by transformation to test phenotype. Phenotypes of the constructs are summarized in the table. The forward/reverse primer set for each construct are shown as the numbered half arrows.
FIG. 10 is a flow chart of an exemplary process for synthesis of error-minimized nucleic acids.

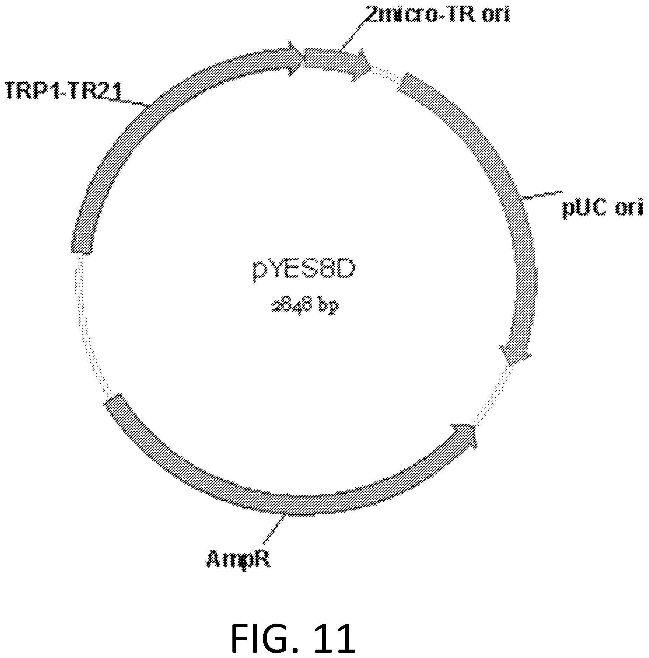

FIG. 11 is a vector map of pYES8D.

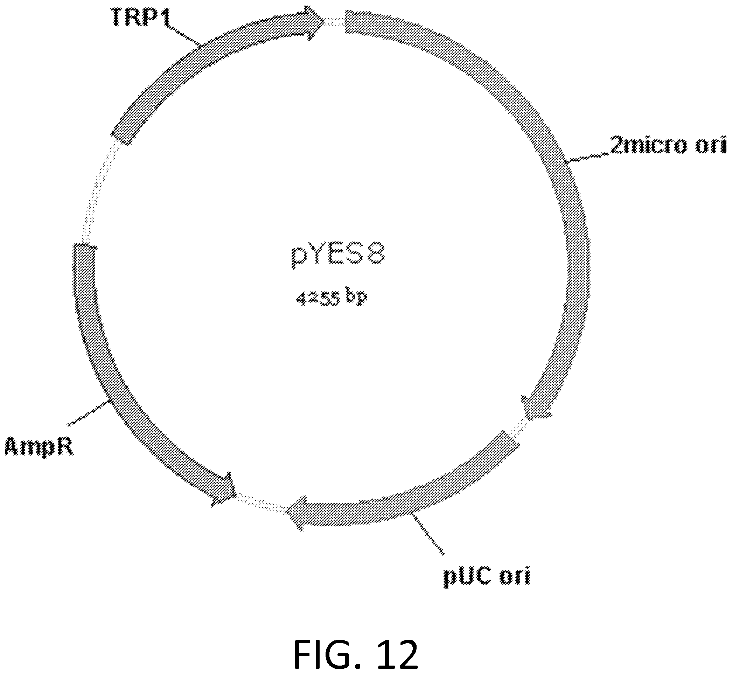

FIG. 12 is a vector map of pYES8.

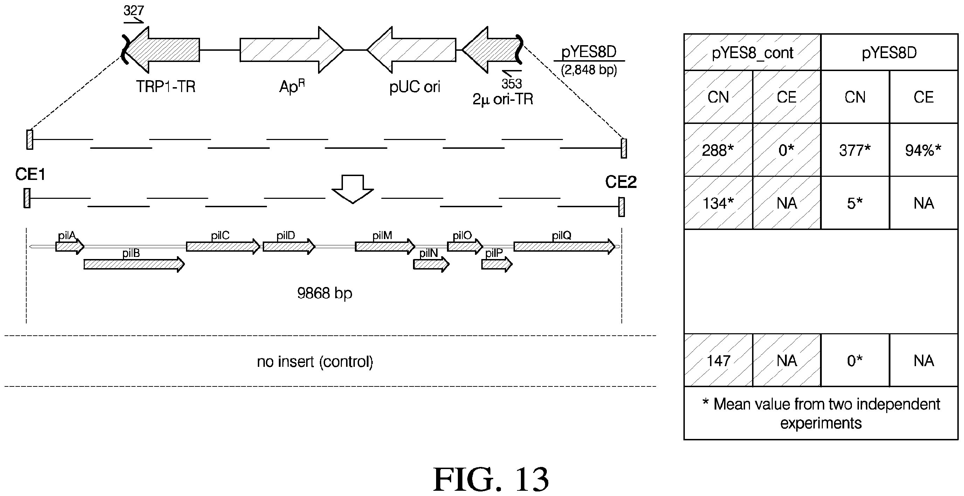

FIG. 13 shows an assembly of 10 DNA fragments for *V. cholerae* pilABCD/pilMNOPQ region (9.9-kb total insert size) using the positive-selection vector pYES8D in yeast. Two assembly sets, missing one insert fragment at pilMQ-1 position (white downward arrow) and no inserts, were tested as control experiments. The complementing sequences for the TRP1-TR (CE1) were added to the forward primer for the pilAD-1 fragment. The complementing sequences for the 2µ ori-TR (CE2) were added to the reverse primer for the pilMQ-5 fragment. Results for colony number and cloning efficiency are summarized in the table. NA, not applicable.

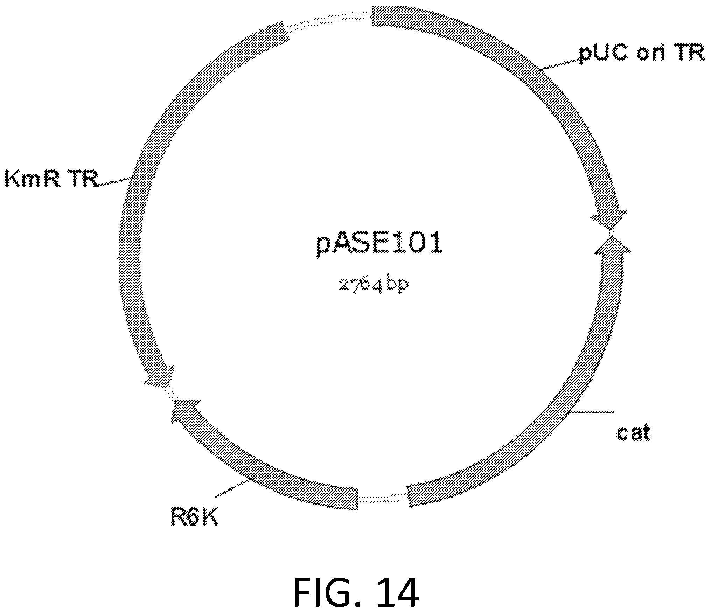

FIG. 14 is a vector map of pASE101.

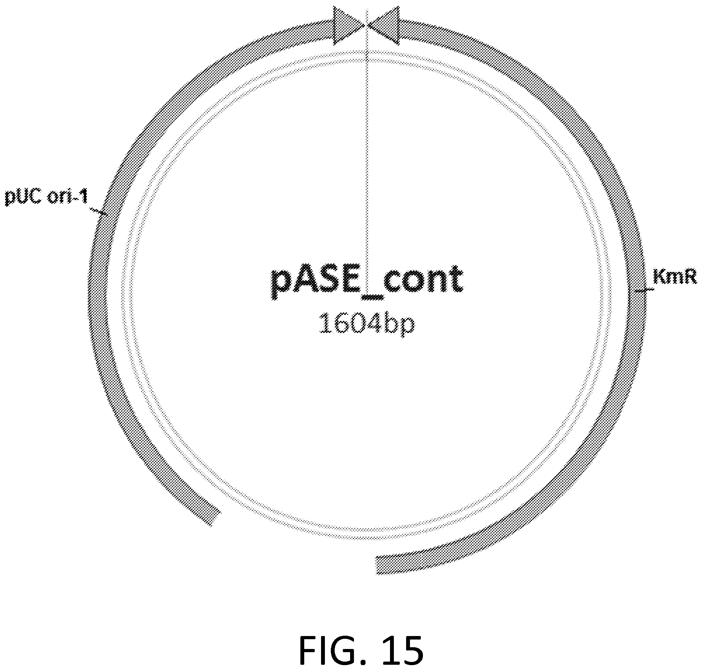

FIG. 15 is a vector map of pASE_cont.

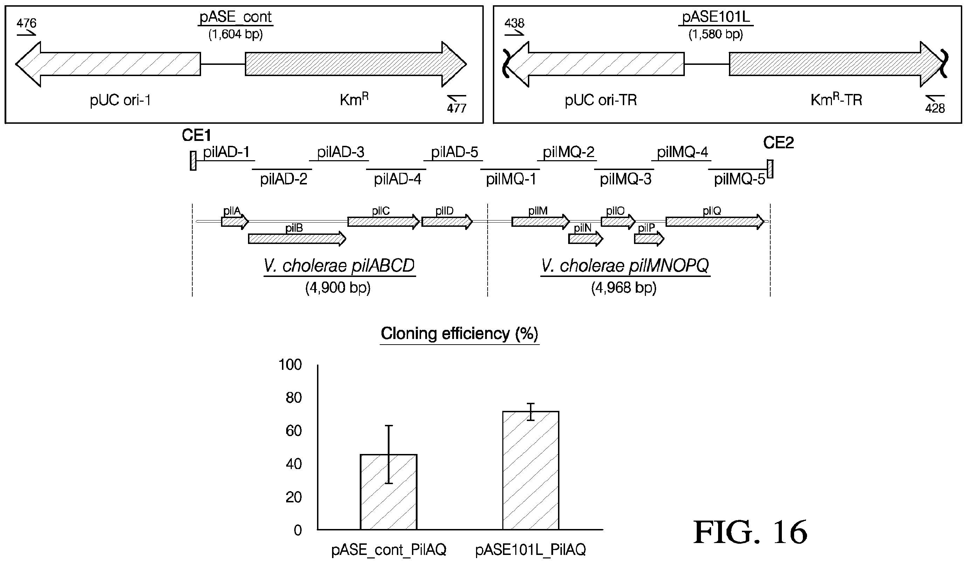

FIG. 16 shows ten fragment assembly into pASE101 and pASE_cont.

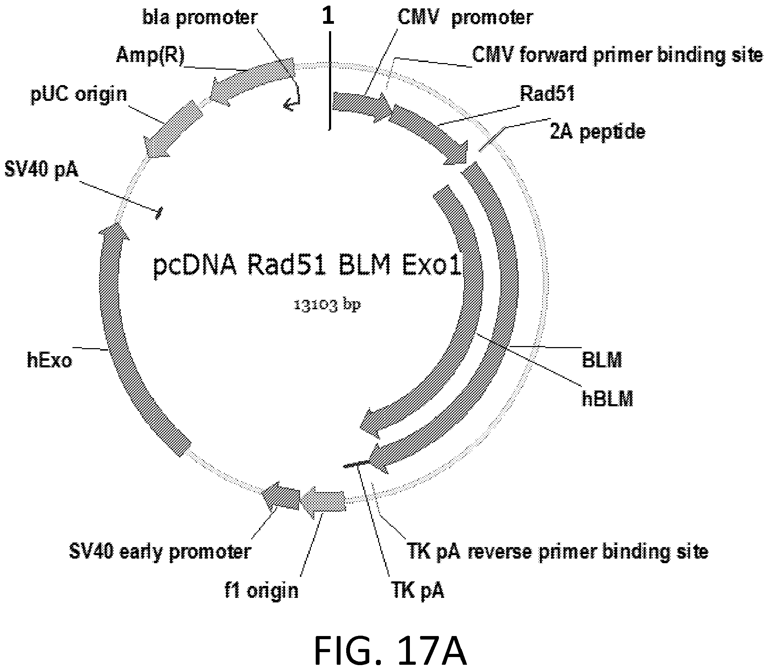

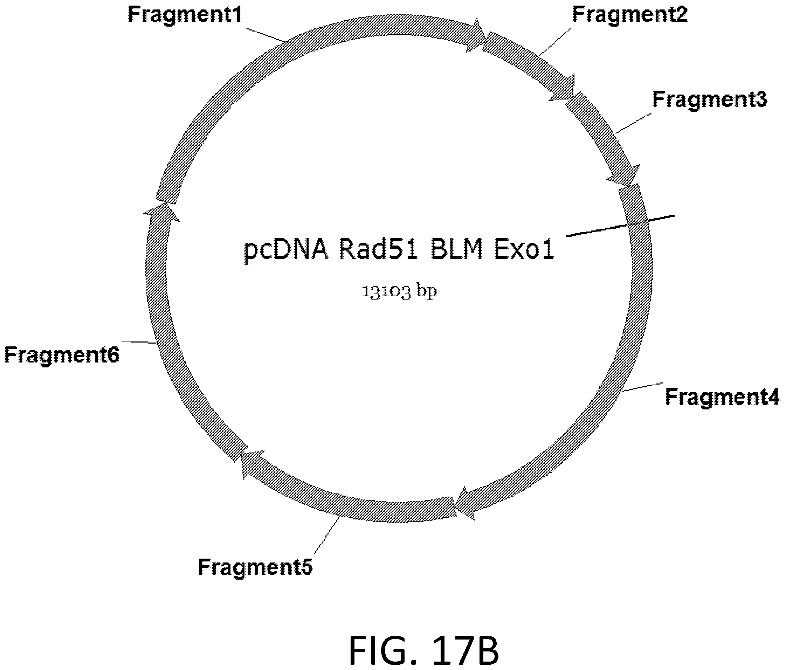

FIGs. 17A-17B show vector maps for pcDNA Rad51 BLM Exo1. The vector contains 13,103 base pairs and was assembled from six fragments/segments using methods of the invention. The nucleotide sequence of this vector is set out in Table 14. Phosphorothioate bonds were located in the termini of the fragments along the lines of FIGs. 2-5.

### DETAILED DESCRIPTION

### DEFINITIONS:

As used herein the term "sequence fidelity" refers to the level of sequence identity of a nucleic acid molecule as compared to a reference sequence. Full identity being 100% identical over the full length of the nucleic acid molecules being scored for sequence identity. Sequence fidelity can be measure in a number of ways, for example, by the comparison of the actual nucleotide sequence of a nucleic acid molecule to a desired nucleotide sequence (e.g., a nucleotide sequence that one wishes to be used to generate a nucleic acid molecule). Another way sequence fidelity can be measured is by comparison of sequences of two nucleic acid molecules in a reaction mixture. In many instances, the difference on a per base basis will be, on average, the same.

As used herein the term "exonuclease" refers to enzymes that cleaves nucleotides one from the end (exo) of a polynucleotide chain. Typically, their enzymatic mechanism involves hydrolyzing reactions that breaks phosphodiester bonds at either the 3' or the 5' end occurs. Exemplary exonucleases include *Escherichia coli* exonuclease I, *Escherichia coli* exonuclease III (3' to 5'), *Escherichia coli* exonuclease VII, *Escherichia coli* exonuclease VIII, bacteriophage lambda exonuclease (5' to 3'), exonuclease T (3' to 5'), bacteriophage T5 Exonuclease, and bacteriophage T7 exonuclease (5' to 3').

As used herein the term "error correction" refers to changes is the nucleotide sequence of a nucleic acid molecule to alter a defect. These defects can be mismatches, insertions, and/or substitutions. Defects can occur when a nucleic acid molecule that is being generated (e.g., by chemical or enzymatic synthesis) is intended to contain a particular base at a location but a different base is present at that location. One error correction workflow is set out in FIG. 10.

As used herein the term "selectable marker" refers to a nucleic acid segment that allows one to select for or against a nucleic acid molecule or a cell that contains it, often under particular conditions. Examples of selectable markers include but are not limited to: (1) nucleic acid segments that encode products which provide resistance against otherwise toxic compounds (*e*.*g*., antibiotics); (2) nucleic acid segments that encode products which are otherwise lacking in the recipient cell (*e*.*g*., tRNA genes, auxotrophic markers); (3) nucleic acid segments that encode products which suppress the activity of a gene product; (4) nucleic acid segments that encode products which can be readily identified (*e*.*g*., phenotypic markers such as (P-galactosidase, green fluorescent protein (GFP), yellow flourescent protein (YFP), red fluorescent protein (RFP), cyan fluorescent protein (CFP), and cell surface proteins); (5) nucleic acid segments that bind products which are otherwise detrimental to cell survival and/or function; (6) nucleic acid segments that bind products that modify a substrate (*e*.*g*., restriction endonucleases); (7) nucleic acid segments that can be used to isolate or identify a desired molecule (*e*.*g*., specific protein binding sites); (8) nucleic acid segments, which when absent, directly or indirectly confer resistance or sensitivity to particular compounds; and/or (9) nucleic acid segments that encode products which either are toxic (*e*.*g*., Diphtheria toxin) or convert a relatively non-toxic compound to a toxic compound (*e*.*g*., Herpes simplex thymidine kinase, cytosine deaminase) in recipient cells.

A "counter selectable" marker (also referred to herein a "negative selectable marker") or marker gene as used herein refers to any gene or functional variant thereof that allows for selection of wanted vectors, clones, cells or organisms by eliminating unwanted elements. These markers are often toxic or otherwise inhibitory to replication under certain conditions which often involve exposure to a specific substrates or shift in growth conditions. Counter selectable marker genes are often incorporated into genetic modification schemes in order to select for rare recombination or cloning events that require the removal of the marker or to selectively eliminate plasmids or cells from a given population. One example of a negative selectable marker system widely used in bacterial cloning methods is the CcdA/CCdB Type II Toxin-antitoxin system.

### OVERVIEW:

The disclosure relates, in part, to compositions and methods for the preparation of nucleic acid molecules. While the disclosure has numerous aspects and variations associated with it, some of these aspects and variations of applicability of the technology may be represented with the exemplary work flow shown in FIG. 1.

FIG.1 shows a work flow including nucleic acid synthesis (e.g., chemical or enzymatic synthesis), pooling of synthesized nucleic acid molecules, amplification of pooled nucleic acid molecules, assembly of nucleic acid molecules (amplified and non-amplified nucleic acid molecules), and insertion of assembled nucleic acid molecules into recipient cells. Further indicated are locations in the work flow where error correction may be employed. As one skilled in the art would understand, error correction, when performed, may be employed at one or more locations in the work flow and multiple rounds of error correction may be employed at each point in the work flow where it is performed.

Multiple variations of the work flow represented in FIG. 1 may be used. For example, in instances where nucleic acid molecules are generated for *in vitro* transcription, recipient cell insertion may be omitted. As another example, sequencing of pre-assembly components of and/or assembled nucleic acid molecules may be used instead of or in additional to error correction of assembled nucleic acid molecules. Further, nucleic acid molecules determined to have the desired nucleotide sequence may be selected for, for example, insertion into recipient cells.

In one aspect, methods are provided for the production of nucleic acid molecules having high "sequence fidelity". This high sequence fidelity can be achieved by, for example, one two or all three of the following: accurate nucleic acid synthesis, error correction, and sequence verification.

Described herein are a number of technologies with applicability to work flows such as those shown in FIG. 1, as well as other work flows. In one aspect, the disclosure is directed to method for the generation for nucleic acid molecules with high sequence fidelity as compared to nucleic acid molecules which are sought.

### NUCLEIC ACID ASSEMBLY:

One exemplary embodiment of assembly technology described herein is set out in FIG. 2. FIG. 2 schematically shows exemplary assembly methods through which two nucleic acid segments (NA1 and NA2) are connected. In this exemplification, each nucleic acid segment has a region of sequence complementarity (Region C) and a region containing two phosphorothioate bonds (Region B) on the same strand or different strands but typically on different strands (*e*.*g*., within from about 4 to about 40 nucleotides of either from 3' or 5' terminus). When exposed to an exonuclease (*e*.*g*., a 5' or 3' exonuclease), one strand of Region C is "chewed back" up to Region B, generating termini capable of hybridizing with each other under suitable conditions (*e*.*g*., temperature, pH, ionic strength, etc.). Upon hybridization, a ligase (or a ligase activity) seals the nicks in each strand resulting in each strand resulting in the formation of a ligated nucleic acid molecule containing no nicks.

Nucleic acid segments such as those used in the work flow of FIG. 2 will typically contain a chemical modification that renders termini of nucleic acid strands resistant to nuclease activity (*e*.*g*., endonuclease digestion). One example of such a chemical modification, phosphorothioate bonds, is shown in FIG. 2. Other chemical modifications include methylphosphonates, 2' methoxy ribonucleotides, locked nucleotides (LNAs), and 3' terminal phosphoroamidates.

Only one terminus of each nucleic acid segment represented in FIG. 2 is shown as containing chemical modifications. In many instances, both termini will be chemically modified (similar to as shown for nucleic acid segments 1 through 4 in FIG. 5).

Numerous parameters may be designed and chosen to assemble, for example, different numbers of segments and nucleic acid segments of different length. Parameters may also be altered that result in increased efficiency of nucleic acid assembly for particular applications.

Using the schematic representation of FIG. 2 for reference, physical parameters such as the total lengths of NA1 and/or NA2, the lengths of Regions A and/or D, the lengths of one or both Region C, and the number of bases in one or both Region B may be varied. One chemical parameter that may be varied is the type of types of nuclease resistant bases incorporated into Region B. Other parameters that may be altered are the concentration of nucleic acid segments for assembly, the units of activity of enzymes (*e*.*g*., exonuclease, ligase, etc.) in the reactions mixture(s), pH, salt concentration, temperature, etc.

With respect to lengths of Regions A and/or D, when a nucleic acid molecule is longer than a certain length, the termini act as though they are, for purposes of association with other nucleic acid molecules, in effect different molecules. This, and other factors associated with long nucleic acid molecules (*e*.*g*., fragility), means that nucleic acid segment length is one factor for optimization with respect to assembly efficiency.

In some aspects of the invention, nucleic acid segment length will vary from about 20 base pairs to about 5,000 base pairs, from about 100 base pairs to about 5,000 base pairs, from about 150 base pairs to about 5,000 base pairs, from about 200 base pairs to about 5,000 base pairs, from about 250 base pairs to about 5,000 base pairs, from about 300 base pairs to about 5,000 base pairs, from about 350 base pairs to about 5,000 base pairs, from about 400 base pairs to about 5,000 base pairs, from about 500 base pairs to about 5,000 base pairs, from about 700 base pairs to about 5,000 base pairs, from about 800 base pairs to about 5,000 base pairs, from about 1,000 base pairs to about 5,000 base pairs, from about 100 base pairs to about 4,000 base pairs, from about 150 base pairs to about 4,000 base pairs, from about 200 base pairs to about 4,000 base pairs, from about 300 base pairs to about 4,000 base pairs, from about 500 base pairs to about 4,000 base pairs, from about 50 base pairs to about 3,000 base pairs, from about 100 base pairs to about 3,000 base pairs, from about 200 base pairs to about 3,000 base pairs, from about 250 base pairs to about 3,000 base pairs, from about 300 base pairs to about 3,000 base pairs, from about 400 base pairs to about 3,000 base pairs, from about 600 base pairs to about 3,000 base pairs, from about 800 base pairs to about 3,000 base pairs, from about 100 base pairs to about 2,000 base pairs, from about 200 base pairs to about 2,000 base pairs, from about 300 base pairs to about 1,500 base pairs, etc.

Nucleic acid segments used for assembly may be derived from a number of sources, for example, they may be cloned, derived from polymerase chain reactions, or chemically synthesized. Chemically synthesized nucleic acids tend to be of less than 100 nucleotides in length. PCR and cloning can be used to generate much longer nucleic acids. Further, the percentage of erroneous bases present in nucleic acids (*e*.*g*., nucleic acid segment) is, to some extent, tied to the method by which it is made. Typically, chemically synthesized nucleic acids have the highest error rate.

The length of the "hybridization" region, Region C, may also vary. The lengths of Region C may vary on each nucleic acid segment. FIG. 2 shows Region C being 15 base pairs in length on each nucleic acid segment. The lengths of Region C on each nucleic acid segment may vary with factor such as AT/CG content (due to A:T having two hydrogen bonds and C:G having three hydrogen bonds), the number of nucleic acid segments being assembled, the lengths of the nucleic acid segments, and the incubation conditions (*e*.*g*., salt concentration, pH, temperature, etc.).

Typically, Region C will be, independently, on one or both segments in ranges of from about 1 to about 100 base pairs, from about 2 to about 100 base pairs, from about 10 to about 100 base pairs, from about 15 to about 100 base pairs, from about 20 to about 100 base pairs, from about 5 to about 80 base pairs, from about 10 to about 80 base pairs, from about 20 to about 80 base pairs, from about 30 to about 80 base pairs, from about 40 to about 80 base pairs, from about 25 to about 65 base pairs, from about 35 to about 65 base pairs, from about 1 to about 50 base pairs, from about 2 to about 50 base pairs, from about 3 to about 50 base pairs, from about 5 to about 50 base pairs, from about 6 to about 50 base pairs, from about 7 to about 50 base pairs, from about 8 to about 50 base pairs, from about 10 to about 50 base pairs, from about 12 to about 50 base pairs, from about 13 to about 50 base pairs, from about 14 to about 50 base pairs, from about 15 to about 50 base pairs, from about 18 to about 50 base pairs, from about 20 to about 50 base pairs, from about 1 to about 35 base pairs, from about 5 to about 30 base pairs, from about 5 to about 25 base pairs, from about 5 to about 20 base pairs, from about 5 to about 18 base pairs, from about 8 to about 50 base pairs, from about 8 to about 35 base pairs, from about 8 to about 30 base pairs, from about 8 to about 25 base pairs, from about 8 to about 20 base pairs, from about 10 to about 40 base pairs, from about 10 to about 35 base pairs, from about 10 to about 30 base pairs, from about 10 to about 25 base pairs, from about 10 to about 20 base pairs, etc.

The disclosure includes compositions and methods for nucleic acid assembly where the length or Region C varies with the sequence of this region. In particular, the disclosure includes reaction mixtures where nucleic acid segments with higher amount of As and Ts in Region C have a longer Region C than nucleic acid segments with a higher amount of Cs and Gs. As an example, Region C of a nucleic acid segment with 60% C and G and 40% A and T may be 12 base pairs in length. Region C of a nucleic acid segment with 60% A and T and 40% C and G may be 18 base pairs in length. Further, both of these nucleic acid segments may be assembled in the same reaction mixture.

Table 1 shows an exemplary relationship between the A/T:C/G content and length of Region C. Region C may also be of different lengths when present at both termini of a nucleic acid segment.

| **Table 1**: Exemplary Region C (Hybridization Region) Lengths and A/T:C/G Content | | | |
|---|---|---|---|
| **Number of A/T Base Pairs** | **% A/T** | **Length of Region C (Base Pairs)** | **% Δ in Length** |
| 5 | 33.3% | 9 | 40% |
| 6 | 40% | 12 | 20% |
| 7 or 8 | 46.7%/53.3% | 15 | NA |
| 9 | 60% | 18 | 20% |
| 10 | 66.7% | 21 | 40% |

The disclosure thus includes methods for assembling two or more nucleic acid segments, wherein one nucleic acid segment comprises at least one terminus with sequence homology to a second nucleic acid segment (*e.g.*, Region C), wherein the region of homology varies in length as a function of the A/T:C/G ratio, with longer regions of sequence homology being present where the termini have higher A/T: C/G ratios. In some instances, one or both nucleic acid segment with sequence homology at their termini will contain an exonuclease resistant region (*e*.*g*., Region B).

In many instances, Regions C will be designed such that the two regions share 100% sequence complementarity after nuclease digestion. In some instances, sequence complementarity will be below 100% (*e*.*g*., greater than 75%, greater than 80%, greater than 85%, greater than 90%, greater than 95%, between 75% and 99%, between 75% and 95%, between 75% and 90%, between 75% and 85%, between 80% and 99%, between 80% and 95%, between 85% and 99%, between 85% and 95%, etc.).

Further, incubation conditions may be adjusted such that there is, on average, partial or complete nuclease digestion of one strand of Region C. Also, conditions may be adjusted such that either the 3' strand or the 5' strand is digested. This may be determined by the choice of nuclease used (*e.g.*, exonuclease). In particular, one or more 3'-exonuclease or 5' exonuclease may be used. For example, two or more exonucleases may be used to digest termini of nucleic acid segments.

The length, number and spacing of nuclease resistant bases in Region B may also vary. In some instances, Region B will be bounded by nuclease resistant bases. In other instances, Region B will contain non-resistant bases abutting Region C. This may be useful instances where one seeks to add one or more bases (*e.g*., restriction sites) to final assembly products that may or may not be translated. With reference to FIG. 2, the junction between Regions B and C will generally be determined by the overlap region (Region C) between nucleic acid 1 (NA1) and nucleic acid 2 (NA2).

Nuclease resistant bases will normally be in only one strand of nucleic acid segments to be joined but may be present in both strands.

The length of Region B may be as short as one base pair or substantially longer than one base pair. In some instances, the length of Region B will be from about one to about twenty base pairs, from about one to about fifteen base pairs, from about one to about ten base pairs, from about one to about six base pairs, from about one to about four base pairs, from about one to about two base pairs, from about two to about twenty base pairs, from about two to about ten base pairs, from about two to about five base pairs, from about three to about twenty base pairs, from about three to about ten base pairs, from about three to about five base pairs, etc.

The number of nuclease resistant bases in Region B may also vary. For example, the number of bases may be from about one to about ten, from about two to about ten, from about three to about ten, from about four to about ten, from about five to about ten, from about two to about five, from about two to about four, etc.

Other parameters that may be varied include the concentration of nucleic acid segments present and the ratio of these segments. In many instances, the nucleic acid segment concentration will be adjusted in combination with the concentration of nuclease and enzyme with ligase activity. Further, the ratio of nucleic acid segments to each other will often be essentially 1:1 but ratios may vary for particular applications. For example, when hybridization termini are AT rich (*e*.*g*., greater than 50%, 55%, 60%, 65% AT), these nucleic acid segments may be present in a higher ratio than nucleic acid segments with non-AT rich hybridization termini.

Nucleic acid segments such as those represented in FIG. 2 may be generated by polymerase chain reaction (PCR) using primers containing nuclease resistant modifications. Such nucleic acid segments may also be generated by other methods such as chemical synthesis.

FIG. 3 shows some exemplary spacing of nuclease resistant bases in Region B. The far left shows two nuclease resistant bases (R) in one strand and two nuclease sensitive bases (S) in the other strand. The far left shows a Region B that is five base pairs in lengths with interspersed nuclease resistant bases in one strand. A single nuclease resistant base in the other strand and this base is located in Region B abutting Region A. One advantage of having a nuclease resistant base at this location is that provides nuclease resistance for the inhibition of digestion of Region B into Region A by exonucleases.

In some embodiments, two or more nucleic acid segments may be digested with exonucleases together or separately, then combined for assembly. In such instances, the same or different exonuclease may be used to digest termini or each fragment. Similarly, digestion reaction conditions may be the same or different the nucleic acid segments.

If desired, amplification of these nucleic acid molecules (*e.g.,* polymerase chain reaction) may also be employed to generate nucleic acid molecules without phosphorothioate bonds.

FIG. 4 shows a variation of the process shown in FIG. 2, where only one of the two nucleic acid segments to be joined at their termini is susceptible to nuclease action. In such instances, a blunt end may be joined to a "sticky" end through "strand invasion". Strand invasion results in the formation of a "flap", which is a single stranded region that protrudes from the connected nucleic acid segments. Strand invasion mechanisms are set out in U.S. Patent No. 7,528, 241. A ligase or a molecule with ligase activity (e.g., a topoisomerase) may be used to connect the strand the recessed strand of the "invading" terminus to the 3' strand of the blunt terminus of NA1.

In many instances of embodiments shown in FIG. 2, elimination of the "flap" will be performed after introduction into a cell by cellular nucleic acid repair mechanisms. Also, ligation of both strands may also occur intracellularly. In such instances, the two nucleic acid segments would not be covalently bound to each other until after introduction into a cell.

FIG. 5 is a schematic showing the assembly of six nucleic acid segments using methods of the invention. In this representation, two vector segments (Vector Segment A and Vector Segment B) are joined to four nucleic acid segments numbered 1 through 4. While FIG. 5 is directed to the assembly of a closed, circular vector, assemblies may be linear nucleic acid molecules. Compositions and methods are also provide for the preparation of linear nucleic acid molecules (*e.g*., linear vectors, sub-components of a larger nucleic acid molecule, nucleic acid molecules suitable for homologous recombination, etc.).

When a replicable, circular vector is generated, two types of selection are employed in the workflow of FIG. 5. One selection is based upon the formation of a circular nucleic acid molecule. An origin of replication, for example, may be used that allows for replication of a nucleic acid molecule when that molecule is circular. Thus, vector replication only occurs when circular nucleic acid molecules are formed. The assembly scheme in FIG. 5 is designed to result in the assembly of a circular nucleic acid molecule only when suitable termini are joined, resulting in the formation of a nucleic acid molecule containing Vector Segment A, Vector Segment B and nucleic acid segments 1 through 4. Of course, other combinations of the six nucleic acid segments can form from spurious connections between nucleic acid segments. In such cases, replicable nucleic acid molecules may be screened by methods such as gel electrophoresis and nucleotide sequencing to identify correct assemblies.

A second type of selection involves the use of selectable markers. Vector Segment A and Vector Segment B shown in FIG. 5 each contain a selectable marker. Any number of selectable markers and/or vector segments may be used. If two selective agents are used (*e.g*., ampicillin and puromycin), then only nucleic acid molecules containing both selectable markers (*e.g.*, ampicillin resistance and puromycin resistance) will confer a resistant phenotype on cells. Thus, compositions and method of the disclosure include the presence and use of multiple selectable markers and resistance cassettes. These selectable markers may be present in assembled constructs produced using methods of the disclosure.

The disclosure further includes methods involving multiple selection methods for obtaining assembled nucleic acid molecules containing desired nucleic acid segments. In one example, the disclosure includes methods for selecting assembled nucleic acid molecules through a combination of the generation of replicable vectors (*e.g*., recircularized vectors) and one or more selectable marker.

In some instances, vector segments may be distinguished from other nucleic acid segments in that they contain components in that they will generally contain components (*e.g*., functional components) normally found on. Examples of such components include origins or replication, long terminal repeats, selectable markers, promoters and antidote coding sequences (*e.g., ccd*A coding sequences for counter-acting toxic effects of *ccd*B)*.* However, all nucleic acid segments assembled by methods described herein may contain such components. For example, when nucleic acid segments are assembled to form an operon, the assembled nucleic acid segments will often contain promoter and terminator sequences. Further, in some instances when a vector is assembled, the only segments that will be assembled will be vector segments.

The disclosure thus includes methods for the assembly of nucleic acid segments where some of the nucleic acid segments contain selectable markers or have functionalities that are otherwise required for replication (*e.g*., contain an origin of replication). As noted above, the number of nucleic acid segments assembled by methods of the invention may vary greatly. For example, the number of nucleic acid fragments/segments that may be assembled by methods of the invention include from about two to about fifty, from about three to about fifty, from about four to about fifty, from about two to about five, from about two to about ten, from about two to about fifteen, from about two to about twenty, from about three to about five, from about three to about ten, from about three to about twenty, from about four to about six, from about four to about ten, from about four to about fifteen, from about four to about twenty, from about five to about ten, from about five to about twenty, from about five to about thirty, from about five to about forty, from about eight to about fifteen, etc.

Further, the number of nucleic acid segments that do not contain components that confer selective or other replication related functionality may also vary. In general, the number of "non-selective" assembly components will be greater than the number of "selective" assembly components and the ratio of these two components may vary from about 2:1 to about 1:1, from about 2:1 to about 1.1:1, from about 3:1 to about 1.1:1, from about 5:1 to about 1.1:1, from about 6:1 to about 1.1:1, from about 7:1 to about 1.1:1, from about 8:1 to about 1.1:1, from about 10:1 to about 1.1:1, from about 15:1 to about 1.1:1, from about 20:1 to about 1.1:1, from about 10:1 to about 2:1, from about 10:1 to about 3:1, from about 10:1 to about 4:1, from about 10:1 to about 5:1, from about 10:1 to about 6:1, etc.

In the representation of FIG. 5, the two vector segments contain no nuclease resistant bases at either of their termini and nucleic acid segments 1 through 4 contain nuclease resistant bases at both of their termini. As one skilled in the art would understand, some termini may contain nuclease resistant bases and some may not. Some factors that will determine will often determine which termini contain nuclease resistant bases include ease of or difficulty in making of chemically modified nucleic acid molecules, assembly efficiency of the particular system, and "downstream" work-flow issues associated with the inclusion of modified bases in products nucleic acid molecules.

The six nucleic acid segments represented in FIG. 5 may be exposed to one or more nuclease prior to contact with each other or while in contact with each other. Further, groups of the nucleic acid segments may be exposed to one or more nuclease or some of the nucleic acid segments may be exposed to one or more nuclease followed by incubation of undigested nucleic acid segments and nuclease digested nucleic acid segments in the presence of one or more nuclease. Thus, the disclosure includes, for example, workflows in which nucleic acid segments containing one or more nuclease resistant bases near one or both termini are contacted with one or more nuclease, then contacted with undigested nucleic acid segments. The undigested nucleic acid segments may have 5' or 3' overhangs at one or both termini or may be blunt ended at both termini.

6 shows a nucleic acid assembly scheme for the assembly of ten nucleic acid segments and a pYES8D vector segment. In this experiment eleven fragments with a total size of almost 13,000 base pairs were assembled. Correct assembly of these eleven nucleic acid segments results in the reconstitution of two vector components: TRP1 (yeast tryptophan auxotrophic marker) and the 2µ origin of replication. Also present on the vector back bone shown in FIG. 6 are a full length pUC origin of replication and a full length ampicillin resistance marker.

The right hand side of FIG. 6 shows data associated with assembly experiments. As can be seen the highest cloning efficiency was seen with PCR amplified nucleic acid segments. PCR generated and pre-cloned nucleic acids tend to be of higher purity than chemically synthesized ones. This may be one reason for the high cloning efficiency seen PCR amplified nucleic acid segments. FIG. 6A shows the experimental assembly and FIG. 6B shows a series of control assemblies. Assemblies missing one fragment at different positions (white blank arrow) gave zero or very low colony output indicating that every single fragment is important for successful assembly. Assembly of the truncated vector pYES8D with no insert showed no colony output, whereas assembly of the pYES8 (no positive selection) vector alone gave some false-positive background colonies. Thus this zero background from pYES8D-based DNA assembly may contribute to the higher cloning efficiency (95%) for pre-cloned DNA than pYES8-based assembly (63%).

Correctly assembled nucleic acid molecules resulting from the work flow shown in FIG. 6 are capable of replicating in both *Escherichia coli* and *Saccharomyces cerevisiae.* Thus, initial cloning may be done in *E. coli* in the presence of ampicillin, followed by transfer to *S. cerevisiae* for selection of full length, correctly assembled vectors. Alternatively, initial cloning may be done in *S. cerevisiae* for selection of full length, correctly assembled vectors, followed by transfer to *E. coli* if desired.

The disclosure thus provides compositions and methods for the preparation of shuttle vectors. These shuttle vectors may be screened for full length, correctly assembly in one organism (*e.g*., a eukaryotic cell), followed by transfer to another organism (*e.g*., a prokaryotic cell).

The disclosure also provides compositions and methods for the assembly of nucleic acid segments involving the reconstitution of one or more selectable markers and/or one or more origin of replication. In many instances, two functional components required for cell survival will be reconstituted in methods of the disclosure.

Compositions and methods of the disclosure are also useful for the preparation of nucleic acid molecules that encode counter-selectable markers (*e.g.*, *ccd*B)*.* Such vectors may be generated in a number of different ways. Vectors with counter-selectable markers may be generated by introducing assembled nucleic acid molecules into a cell that is not susceptible to the marker. Two types of such cells are ones that are not naturally susceptible to the marker (*e.g.,* introduction of a *ccd*B counter-selectable marker into a yeast cell) or one that encodes an antidote or is otherwise resistant to the counter-selectable marker product (*e.g., ccd*A and *ccd*B)*.*

FIG. 7 shows a work flow using compositions and methods of the disclosure. pYES8D is employed to assemble a 9.9kb region of the *Vibrio cholera* genome. As can be seen from the numerical data on the right, high efficiency assembly and cloning were achieved.

FIG. 8 shows a workflow for the assembly of and *E. coli* vector containing (1) an origin of replication, (2) a kanamycin resistance marker, (3) a green fluorescent protein gene fragment, (4) a lacZ-α fragment, and (5) a chloramphenicol resistance marker fragment.

FIG. 9 shows a work flow for full assembly of an *E. coli* vector containing two origins of replication, one functional and the other truncated. Also present are two selectable markers, one function and the other truncated. Vectors such as this may be used to produce vector fragments suitable for use in assembly reactions. The disclosure thus includes compositions and methods for: (1) generating vectors suitable for use in assembly reactions and (2) vectors containing truncated functional elements for use in assembly reactions. The second item relates to method for producing truncated vector fragments for use in assembly reaction.

### ERROR IDENTIFICATION AND CORRECTION:

Errors may find their way into nucleic acid molecules in a number of ways. Examples of such ways include chemical synthesis errors, amplification/polymerase mediated errors (especially when non-proof reading polymerases are used), and assembly mediated errors (usually occurring at nucleic acid segment junctions).

Two ways to lower the number of errors in assembled nucleic acid molecules is by (1) selection of nucleic acid segments for assembly with corrects sequences and (2) correction of errors in nucleic acid segments, partially assembled sub-assemblies nucleic acid molecules, or fully assembled nucleic acid molecules.

In many instances, errors are incorporated into nucleic acid molecules regardless of the method by which the nucleic acid molecules are generated. Even when nucleic acid segments known to have correct sequences are used for assembly, errors can find their way into the final assembly products. Thus, in many instances, error reduction will be desirable. Error correction can be achieved by any number of means.

One method is by individually sequencing nucleic acid segments (*e.g*., chemically synthesized nucleic acid segments), followed by assembly of only nucleic acid segments determined to have correct sequences. This may be done by the selection of a single nucleic acid segment for amplification, then sequencing of the amplification products to determine if any errors are present. Thus, the disclosure also includes selection methods for the reduction of sequence errors. Methods for amplifying and sequence verifying nucleic acid molecules are set out in U.S. Patent No. 8,173,368, Similar methods are set out in Matzas et al., Nature Biotechnology, 28:1291-1294 (2010).

Another way to reduce the number of sequence errors is by error correction. An exemplary error correction workflow is set out in FIG. 10, which shows a process for synthesis of error-minimized nucleic acid molecules. In the first step, nucleic acid molecules of a length smaller than that of the full-length desired nucleotide sequence (*i.e.,* "nucleic acid molecule fragments" of the full-length desired nucleotide sequence) are obtained. Each nucleic acid molecule is intended to have a desired nucleotide sequence that comprises a part of the full length desired nucleotide sequence. Each nucleic acid molecule may also be intended to have a desired nucleotide sequence that comprises an adapter primer for PCR amplification of the nucleic acid molecule, a tethering sequence for attachment of the nucleic acid molecule to a DNA microchip, or any other nucleotide sequence determined by any experimental purpose or other intention. The nucleic acid molecules may be obtained in any of one or more ways, for example, through synthesis, purchase, etc.

In the optional second step, the nucleic acid molecules are amplified to obtain more of each nucleic acid molecule. The amplification may be accomplished by any method, for example, by PCR. Introduction of additional errors into the nucleotide sequences of any of the nucleic acid molecules may occur during amplification.

In the third step, the amplified nucleic acid molecules are assembled into a first set of molecules intended to have a desired length, which may be the intended full length of the desired nucleotide sequence. Assembly of amplified nucleic acid molecules into full-length molecules may be accomplished in any way, for example, by using a PCR-based method.

In the fourth step, the first set of full-length molecules is denatured. Denaturation renders single-stranded molecules from double-stranded molecules. Denaturation may be accomplished by any means. In some examples, denaturation is accomplished by heating the molecules.

In the fifth step, the denatured molecules are annealed. Annealing renders a second set of full-length, double-stranded molecules from single-stranded molecules. Annealing may be accomplished by any means. In some examples, annealing is accomplished by cooling the molecules.

In the sixth step, the second set of full-length molecules are reacted with one or more endonucleases to yield a third set of molecules intended to have lengths less than the length of the complete desired gene sequence. The endonucleases cut one or more of the molecules in the second set into shorter molecules. The cuts maybe accomplished by any means. Cuts at the sites of any nucleotide sequence errors are particularly desirable, in that assembly of pieces of one or more molecules that have been cut at error sites offers the possibility of removal of the cut errors in the final step of the process. In an exemple, the molecules are cut with T7 endonuclease I, *E. coli* endonuclease V, and Mung Bean endonuclease in the presence of manganese. In this example, the endonucleases are intended to introduce blunt cuts in the molecules at the sites of any sequence errors, as well as at random sites where there is no sequence error.

In the last step, the third set of molecules is assembled into a fourth set of molecules, whose length is intended to be the full length of the desired nucleotide sequence. Because of the late-stage error correction enabled by the provided method, the set of molecules is expected to have many fewer nucleotide sequence errors than can be provided by methods in the prior art.

The process set out above and in FIG. 10 is also set out in U.S. Patent No. 7,704,690.

Another process for effectuating error correction in chemically synthesized nucleic acid molecules is by a commercial process referred to as ERRASE™ (Novici Biotech). Error correction methods and reagent suitable for use in error correction processes are set out in U.S. Patents Nos. 7,838,210 and 7,833,759, U.S. Patent Publication No. 2008/0145913 A1 (mismatch endonucleases), and PCT Publication WO 2011/102802 A1.

Exemplary mismatch endonucleases include endonuclease VII (encoded by the T4 gene 49), RES I endonuclease, CEL I endonuclease, and SP endonuclease or methyl-directed endonucleases such as MutH, MutS or MutL. The skilled person will recognize that other methods of error correction may be practiced in certain examples of the disclosure such as those described, for example, in U.S. Patent Publication Nos. 2006/0127920 AA, 2007/0231805 AA, 2010/0216648 A1, 2011/0124049 A1 or U.S. Patent No. 7,820,412.

One error correction methods involves the following steps. The first step is to denature DNA contained in a reaction buffer (*e.g.*, 200 mM Tris-HCl (pH 8.3), 250 mM KCl, 100 mM MgCl₂, 5 mM NAD, and 0.1% TRITON® X-100) at 98°C for 2 minutes, followed by cooling to 4°C for 5 minutes, then warming the solution to 37°C for 5 minutes, followed by storage at 4°C. At a later time, T7endonuclease I and DNA ligase are added the solution 37°C for 1hour. The reaction is stopped by the addition EDTA. A similar process is set out in Huang et al., Electrophoresis 33:788 796 (2012).

Once nucleic acid segments are assembled, their sequences may be confirmed by sequence analysis. Sequence analysis may be used to confirm that "junction" sequences are correct and that no other nucleotide sequence "errors" are located within assembled nucleic acid molecules.

A number of nucleic acid sequences methods are known in the art and include Maxam-Gilbert sequencing, chain-termination sequencing (*e.g*., Sanger sequencing), pyrosequencing, sequencing by synthesis and sequencing by ligation.

The disclosure thus includes compositions and methods for the assembly of nucleic acid molecules with high sequence fidelity. High sequence fidelity can be achieved by several means, including sequencing of nucleic acid segments prior to assembly or partially assembled nucleic acid molecules, sequencing of fully assembled nucleic acid molecules to identify ones with correct sequences, and/or error correction.

### HIGH ORDER ASSEMBLY:

Large nucleic acid molecules are relatively fragile and, thus, shear readily. One method for stabilizing such molecules is by maintaining them intracellularly. Thus, in some aspects, the disclosure involves the assembly and/or maintenance of large nucleic acid molecules in host cells. Large nucleic acid molecules will typically be 20kb or larger (*e*.*g*., larger than 25kb, larger than 35kb, larger than 50kb, larger than 70kb, larger than 85kb, larger than 100kb, larger than 200kb, larger than 500kb, larger than 700kb, larger than 900kb, etc.).

Methods for producing and even analyzing large nucleic acid molecules are known in the art. For example, Karas et al., "Assembly of eukaryotic algal chromosomes in yeast, Journal of Biological Engineering 7:30 (2013) shows the assembly of an algal chromosome in yeast and pulse-field gel analysis of such large nucleic acid molecules.

As suggested above, one group of organisms known to perform homologous recombination fairly efficient is yeasts. Thus, host cells used in the practice of the disclosure may be yeast cells (*e.g., Saccharomyces cerevisiae, Schizosaccharomyces pombe, Pichia, pastoris,* etc.).

Yeast hosts are particularly suitable for manipulation of donor genomic material because of their unique set of genetic manipulation tools. The natural capacities of yeast cells, and decades of research have created a rich set of tools for manipulating DNA in yeast. These advantages are well known in the art. For example, yeast, with their rich genetic systems, can assemble and re-assemble nucleotide sequences by homologous recombination, a capability not shared by many readily available organisms. Yeast cells can be used to clone larger pieces of DNA, for example, entire cellular, organelle, and viral genomes that are not able to be cloned in other organisms. Thus, in some examples, the disclosure employs the enormous capacity of yeast genetics generate large nucleic acid molecules (*e.g*., synthetic genomics) by using yeast as host cells for assembly and maintenance.

Exemplary of the yeast host cells are yeast strain VL6-48N, developed for high transformation efficiency parent strain: VL6-48 (ATCC Number MYA-3666TM)), the W303a strain, the MaV203 strain (Thermo Fisher Scientific, cat. no. 11281-011), and recombination-deficient yeast strains, such as the RAD54 gene-deficient strain, VL6-48-Δ54G (MATαhis3-Δ200 trpl-Δ1 ura3-52 lys2 ade2-101 met14 rad54-Δ1:: kanMX), which can decrease the occurrence of a variety of recombination events in yeast artificial chromosomes (YACs).

### SAMPLE PREPRARATION AND STORAGE:

In some instances, enzymes associated with nucleic acid assembly reactions interfere with nucleic acid molecule stability. As a result, some assembly protocols call for the transformation of cells within a short time period (*e.g.*, less than one hour) after assembly has been performed. This is not always convenient and, in some cases (*e.g*., high-throughput applications), may not be practical. The disclosure thus provides compositions and methods for stabilizing partially and/or fully assembled nucleic acid molecules.

This aspect of methods of the disclosure involves the use of conditions for inhibiting enzymatic reactions employed in the assembly of nucleic acid segments. One enzyme that may be inhibited is exonuclease. Exonucleases, as well as other enzymes (*e.g.*, polymerases and ligases), may be inhibited by (1) the addition of an inhibitor, a proteinase, and/or an antibody with binding affinity for a reaction component (*e.g*., an exonuclease) and/or (2) physical means such as alteration of pH, metal ion concentration, heating, or salt concentration. Also, compositions and methods of the disclosure may involve a combination of inhibition methods. One goal of such methods is to reduce the activity of enzymatic function to a desired level, including essentially complete inactivation (*i.e*., unidentifiable levels of activity).

In terms of reduction of exonuclease activity, the level of inhibition will typically be measured under conditions and at a temperature (*e.g.,* 37°C) where the particular enzyme exhibits high levels of activity. This provides a benchmark for comparison. Exemplary reaction conditions include 67 mM glycine-KOH, 2.5 mM MgCl₂, 50 µg/ml BSA, pH 9.4, 37°C (Lambda Exonuclease); and 67 mM glycine-KOH, 6.7 mM MgCl₂, 10 mM β-mercaptoethanol, pH 9.5, 37°C (*E. coli* Exonuclease I). Typically, the goal will be to achieve a reduction in enzymatic activity of at least 80% (*e.g.,* at least 85%, at least 90%, at least 95%, at least 97%, at least 98%, from about 80% to about 99%, from about 80% to about 98%, from about 80% to about 97%, from about 80% to about 95%, from about 80% to about 93%, from about 85% to about 99%, from about 85% to about 98%, from about 85% to about 97%, from about 85% to about 95%, from about 90% to about 99%, from about 90% to about 98%, from about 90% to about 97%, from about 90% to about 96%, from about 90% to about 95%, from about 90% to about 94%, from about 90% to about 93%, etc.) as compared to benchmark conditions.

Methods for identifying degradation of nucleic acid molecules include transformation efficiency and gel electrophoresis. With gel electrophoresis, a portion of a reaction mixture may be run on a gel and the amount of "smearing" may be determined. The level of smearing may then be used to calculate the amount (*e.g.*, percentage) of the nucleic acids present that have been damaged. Thus, in some aspects, assays that may be used for determining whether a sample has been stabilized by methods for the disclosure involve the measurement of degradation of nucleic acid molecules in reaction mixtures maintained under defined storage conditions (*e.g.*, -20°C for 2 weeks, -20°C for 4 weeks, -20°C for 8 weeks, -20°C for 12 weeks, -20°C for 20 weeks, -20°C for 24 weeks, -20°C for 30 weeks, -20°C for 36 weeks, -20°C for 40 weeks, -20°C for 48 weeks, -20°C for 52 weeks, -70°C for 2 weeks, -70°C for 4 weeks, -70°C for 8 weeks, -70°C for 12 weeks, -70°C for 20 weeks, -70°C for 24 weeks, -70°C for 30 weeks, -70°C for 36 weeks, -70°C for 40 weeks, -70°C for 48 weeks, -70°C for 52 weeks, etc.).

Enzymatic reactions normally follow a trend of decreasing as the temperature decreases from the optimum temperature for the particular enzyme catalyzing the reaction. Further, enzymatic reactions continue to occur even when reactions mixtures are frozen. Also, the lower the temperature after a sample is frozen, the lower the enzymatic reaction rate. Thus, enzymatic reaction rates are expected to be lower at -70°C than at -20°C. The benchmark temperature referenced above is used for convenience because assaying of enzymatic activity under common laboratory sample storage conditions (*e.g.,* -20°C) is generally more difficult than under optimal reaction conditions. Also, high levels of enzymatic activities typical associated with optimal reaction conditions (or reactions conditions close thereto) provide sufficient activity to accurately measure the effects of inhibitory conditions.

Exonuclease inhibitors that may be used in the practice of the disclosure include 8-oxoguanine, mononucleotides, nucleoside 5'-monophsophates, 6-mercaptopurine ribonucleoside 5'-monophsophate, sodium fluoride, fludarabine (9-β-D-arabinofuranosyl-2-fluoroadenine 5'-monophosphate)-terminated DNA, and nucleic acid binding proteins (*e.g*., poly(U)-binding protein. Exonuclease inhibitors may inhibit specific exonucleases, groups of exonucleases (*e.g.,* 3' to 5' exonucleases, 5' to 3' exonucleases, etc.), or essentially all exonucleases.

As noted above, pH may also be altered to inhibit enzymatic activities (*e.g*., exonuclease activity). Many exonucleases, for example, exhibit significant nuclease activity at pHs in ranges of 7.5 to 9.5. A shifting of the pH away from the optimum for the particular exonuclease or exonucleases used will generally decrease enzymatic activities. Further, the farther the pH is shifted from the optimum pH, the less enzymatic activity is expected. Also, pH may be shifted higher or lower. In instances, where the removal of RNA is desired pH may be shifted higher because RNA, but generally not DNA, is hydrolyzed under basic conditions.

In many instances, pH shifts will be greater than one pH unit from the optimum pH of at least one of the exonucleases present in a nucleic acid segments assembly reaction mixture. Thus, if the optimum pH for a particular enzyme is 7.5, then the pH would be shifted to at least either pH 6.5 or 8.5. pH shifts will typically be in the ranges of from about 1 to about 7 pH units, from about 1.5 to about 7 pH units, from about 2 to about 7 pH units, from about 2.5 to about 7 pH units, from about 3 to about 7 pH units, from about 3.5 to about 7 pH units, from about 4 to about 7 pH units, from about 4.5 to about 7 pH units, from about 5 to about 7 pH units, from about 1 to about 6 pH units, from about 1.5 to about 6 pH units, from about 2 to about 6 pH units, from about 2.5 to about 6 pH units, from about 3 to about 6 pH units, from about 3.5 to about 6 pH units, from about 4 to about 6 pH units, from about 4.5 to about 6 pH units, from about 5 to about 6 pH units, from about 1 to about 5 pH units, from about 1.5 to about 5 pH units, from about 2 to about 5 pH units, from about 2.5 to about 5 pH units, from about 3 to about 5 pH units, from about 3.5 to about 5 pH units, from about 4 to about 5 pH units, from about 4.5 to about 5 pH units, etc.

Many enzymes, including exonucleases, require divalent metal ions (e.g., magnesium, manganese, and calcium) for enzymatic activity. Removal or sequestration of divalent metal ions may also be used to inhibit enzymatic activities. For example, divalent metal ion sequestration may occur by the addition of a chelating agent such as EDTA, EGTA, 1,2-bis(o-aminophenoxy)ethane-N,N,N',N'-tetraacetic acid (BAPTA). Many chelating agents have higher affinity for some metal ions than other metal ions. For example, EGTA is more selective for calcium ions than magnesium ions.

Final divalent metal ion concentrations in exonuclease reaction mixtures, for example, tend to be in the range of 2 to 7 mM. Sequestration agents, when used, will typically be present in an amount to binding greater than 95% of the total amount of divalent metal ion present. The stoichiometry will often be determined by the affinity of the sequestration agent for the divalent metal ion, the amount of divalent metal ion present, the amount of sequestration agent present, the amount of ions present that compete for the sequestration agent, and other reaction mixture conditions. Typically, sequestration agents will be present in an amount that is at least equal to the divalent metal ion (1:1) but may be present in a greater amount (e.g., from about 5:1 to about 1:1, from about 4:1 to about 1:1, from about 3:1 to about 1:1, from about 5:1 to about 1:1, from about 5:1 to about 1:1, from about 5:1 to about 1:1, from about 2:1 to about 1:1, from about 1.5:1 to about 1:1, from about 1.25:1 to about 1:1, from about 5:1 to about 1.1:1, from about 2.5:1 to about 1.1:1, from about 5:1 to about 1.5:1, from about 2.5: 1 to about 1.5:1, from about 5:1 to about 2:1, from about 4:1 to about 2:1, from about 5:1 to about 1.5:1, etc.). In many instances, the amount of sequestration agent will be adjusted to achieve a reduction in enzymatic activity of at least 80% under the selected benchmark conditions.

One method of inhibiting thermolabile enzymes (e.g., exonucleases, ligases and polymerases) is by heating aqueous reaction mixtures (e.g., aqueous reaction mixtures) containing these enzymes for a sufficient period of time to allow for enzymatic inactivation. In most instances, this will result in irreversible inactivation by denaturation of enzyme(s) present in the reaction mixtures. Suitable heating conditions will vary with the thermal properties of particular enzymes present but will generally be greater than 60°C (*e*.*g*., from about 60°C to about 95°C, from about 65°C to about 95°C, from about 70°C to about 95°C, from about 75°C to about 95°C, from about 80°C to about 95°C, from about 60°C to about 90°C, from about 60°C to about 85°C, from about 60°C to about 80°C, from about 60°C to about 75°C, from about 65°C to about 90°C, from about 60°C to about 95°C, from about 65°C to about 85°C, from about 70°C to about 95°C, from about 70°C to about 90°C, etc.) for at least 5 minutes (e.g., from about 5 min. to about 30 min., from about 5 min. to about 20 min., from about 5 min. to about 15 min., from about 5 min. to about 10 min., from about 10 min. to about 30 min., from about 10 min. to about 25 min., from about 10 min. to about 20 min., etc.).

One advantage of heating to inactivate exonucleases is that, in many instances, it will not be necessary to open containers (*e.g*., tubes) or add reagents as part of the inactivation step. This is especially useful when high-throughput methods are used.

Another way in which assembly reactions may be inhibited is through degradation of one or more assembly reaction components (*e.g.*, an exonuclease). This may be done, for example, using a one or more proteinase. Exemplary proteinases include serine endopeptidases (*e.g.,* Proteinase K of *Tritirachium album limber*) and aspartate proteinases (*e.g.*, pepsin and cathepsin D), threonine proteases, cysteine proteases, glutamic acid proteases, and metalloproteases. Thus, the disclosure includes methods in which assembled nucleic acid molecules are exposed to one or more proteinase for a time sufficient to inhibit assembly reaction components.

Inhibition of assembly reaction components may be measure in a number of ways. One way is by measure the reduction in one or more assembly reaction activity (*e.g.*, exonuclease or ligase activity). For example, when inhibition of exonuclease activity is measured, the amount of reduction of activity is discussed above but will often be greater than 75%. Further, this reduction in activity may be measured in units, with, for example, a decrease in activity of at least 75 units as compared to a control.

Exonuclease units may be defined as the amount of enzyme that will catalyze the release of 10 nanomole of acid-soluble nucleotide in 30 minutes at 37°C in a total reaction volume of 50 µl, with the reaction mixture containing 67 mM Glycine-KOH, 6.7 mM MgCl₂, 10 mM β-ME, pH 9.5 at 25°C and 0.17 mg/ml single-stranded [³H]-DNA.

Methods for assessing exonuclease activity based on the preferential binding of single-stranded DNA over double-stranded DNA to graphene oxide are set out, for example, in Lee et al., "A simple fluorometric assay for DNA exonuclease activity based on graphene oxide, "Analyst 137:2024-2026 (2012).

Another way in which assembly reactions can be inhibited is through the use of antibodies with binding affinity for assembly reaction components (*e.g*., ligase and exonuclease). A number of antibodies with binding affinity for, for examples, ligases and exonucleases are commercially available from companies such as abcam (1 Kendall Square, Suite B2304, Cambridge, MA 02139), including Anti-DNA Ligase III antibody [6G9] (ab587), Anti-DNA Ligase I antibody [10H5] (ab615), Anti-DNA Ligase IV antibody (ab26039), and Anti-Exonuclease 1 antibody (ab106303).

More than one *(e.g.,* two, three or four) enzyme (*e.g.,* exonuclease) inhibition method may be used in the practice of the disclosure. For example, a pH shift may be use in conjunction with heating. When a thermostable enzyme is used, heat based inactivation will generally not be used.

The disclosure thus provides compositions and methods for stabilizing assembled nucleic acid molecules present in reaction mixtures. These reaction mixtures will generally contain components (*e.g.*, enzymes) that can cause damage to the nucleic acid molecules present therein. Nucleic acid molecules in reaction mixtures prepared using methods of the disclosure will typically show little (less than 5% of the total nucleic acid molecules present) or no degradation upon storage at -20°C for 8 weeks, -20°C for 12 weeks, -20°C for 24 weeks, -70°C for 12 weeks, -70°C for 24 weeks, -70°C for 36 weeks, or -70°C for 52 weeks.

### KITS:

The disclosure also provides kits for the assembly and storage of nucleic acid molecules. As part of these kits, materials and instruction are provided for both the assembly of nucleic acid molecules and the preparation of reaction mixtures for storage.

Kits of the disclosure will often contain one or more of the following components:
1. One or more exonuclease,
2. One or more polymerase,
3. One or more ligase,
4. One or more partial vector (*e.g.,* one or more nucleic acid segment containing an origin of replication and/or a selectable marker) or complete vector,
5. One or more enzymatic (*e.g.,* an exonuclease) inhibitor (*e.g.,* a solution with a pH above 9 or below 6.5, a sequestration agent, and, optionally, one or more of the following
6. One or more non-vector nucleic acid segments in may
7. Instructions for how to prepare and store samples (*e*.*g*., direction the addition of one or more inhibitory compound and/or heating of the sample, followed by storage at low temperature (*e.g.*, -20°C or below).

### Examples

### Example 1: Seamless Cloning Using Phosphorothioate Chemistry

There is increasing demand for large, high-fidelity, synthetic DNA constructs. However, the most commonly synthesized genes range in size from 600 to 1,200 bp. Further seamless assembly is required to obtain large nucleic acid (*e.g*., DNA) constructs. A seamless, sequence-independent nucleic acid assembly method, based on phosphorothioate chemistry, is set out in this example. Some features of methods set out in this example are:
1. The use of phosphorothioate chemistry stops the "chew back" reaction of exonuclease at a specified location, allowing the generation of controllable overhangs and correct assembly.
2. Synthetic DNA fragments are generated by PCR using a pair of phosphorothioate end primers, followed by one-step reaction using, for example, the GeneArt® Seamless Cloning and Assembly Kit (Life Technologies Corporation, now part of Thermo Fisher Scientific, cat. no. A13288).
3. Data indicate that the efficiency of cloning ten 1 kb PCR fragments is around 98%, with about 2000 colonies, although the efficiency of cloning ten synthetic strings reduces to about 64%.
4. DNA sequencing analysis confirms the integrity of the DNA conjunctions.
5. Optimization of assembly reactions can be achieved by the alteration of factors such as PCR conditions, length of overhangs, amount of DNAs, and incubation times. In brief, these are highly efficient *in vitro* assembly methods applicable, for example, to gene synthesis.

**Introduction:** Long synthetic DNA fragments (*e.g.,* >10 kb), commonly used for the construction of large genes and multi gene pathways, are often challenging to assemble. Traditional restriction-based ligation methods are sequence-specific and often generate "scars".

Homologous recombination-based methods, such as those employed by the GeneArt® Seamless Cloning and Assembly Kit (Life Technologies Corporation, now part of Thermo Fisher Scientific, cat. no. A13288), utilize exonuclease to generate single-stranded DNA overhangs for joining of overlapping fragments. However, the "chew back" reaction is often difficult to control, which leads to non-specific annealing amongst DNA fragments and decreases the efficiency of large DNA assembly.

In this example, a highly efficiency DNA assembly methods is described, which utilizes phosphorothioate chemistry in conjunction with GeneArt® Seamless Cloning and Assembly Enzyme Mix (cat. no. A14606). These methods allow for one-step assembly of, for example, ten 1 kb PCR fragments, as well as repetitive DNA fragments.

### Material and Methods.

**Materials:** Phusion DNA polymerase (NEB), GeneArt® Seamless Cloning and Assembly Kit (Life Technologies Corporation, now part of Thermo Fisher Scientific, cat. no. A13288), AccuPrime™ Pfx DNA polymerase (Thermo Fisher, cat. no. 12344-032), T4 DNA ligase (Thermo Fisher, cat. no. 15224-090), PureLink™ Quick PCR purification kit (Thermo Fisher, cat. no. K3100-1), pType-IIs recipient vector (vector map can be viewed at www.lifetechnologies.com), One-Shot TOP10 Chemically Competent Cells (Thermo Fisher, cat. no. C4040-10), BigDye terminator v3.1 cycle sequencing kit (Thermo Fisher, cat. no. 4337457), E-gel (Thermo Fisher cat. no. G5018-8), synthetic DNAs and the trimers of Tal assembly repeats are synthesized by GeneArt® (Thermo Fisher).

### Methods:

**Oligo Design:** Two adjacent PCR fragments share 15 bases of homology at each end (FIG. 2). Two consecutive oligonucleotides modified by a phosphorothioate (PS) linkage were added to positions 16 and 17 accounting from the 5' end. Typically, the phosphorothioate primer is approximately 20-30 nucleotides long. For assembly of repetitive DNA fragments, the adjacent DNA fragments can have a 12-bp overlap at their ends, in which the two PS bonds are positioned at nucleotides 13 and 14, counting from the 5' end.

The following phosphorothioate primers were used for DNA amplification and assembly:

| **Table 2:** *Mycoplasma genitalium* |
|---|
| Frag1-F2-5kb: TGC TGG AGT GAA CGC ZEG GCC GAG CGC AAA G (SEQ ID NO: 1) |
| Frag1-R-5kb: GCA AGA AAA CTA TCC OEA CCG CC (SEQ ID NO: 2) |
| Frag2-F-5kb: GGA TAG TTT TCT TGC EEC CCT AAT C (SEQ ID NO: 3) |
| Frag2-R-5kb: CGT CTG GGA CTG GGT EEA TCA G (SEQ ID NO: 4) |
| Frag3-F-5kb: ACC CAG TCC CAG ACG FFG CCG C (SEQ ID NO: 5) |
| Frag3-R-5kb: CAG ATG TGC GGC GAG ZZG CGT GAC TAC (SEQ ID NO: 6) |
| Frag4-F-5kb: CTC GCC GCA CAT CTG FFC TTC AGC (SEQ ID NO: 7) |
| Frag4-R-5kb: CGC AGT GGA AGA TAG FZC TGA TTG (SEQ ID NO: 8) |
| Frag5-F-5kb: CTA TCT TCC ACT GCG FET TGA A (SEQ ID NO: 9) |
| Frag5-R-10kb: AGT GCA GTT GGT GGA EZT GTT GAT G (SEQ ID NO: 10) |
| Frag6-F-10kb: TCC ACC AAC TGC ACT FEG AGA TTG (SEQ ID NO: 11) |
| Frag6-R-10kb: AGC AAG GTG AGA TTG FFA CTA GGA TTG (SEQ ID NO: 12) |
| Frag7-F-10kb: CAA TCT CAC CTT GCT EZG CTT TAG C (SEQ ID NO: 13) |
| Frag7-R-10kb: TCT TGC CCT AGC AGT ZEG TCA TAC CAA C (SEQ ID NO: 14) |
| Frag8-F-10kb: ACT GCT AGG GCA AGA FOC ACC ACC AAA TAG (SEQ ID NO: 15) |
| Fmg8-R-10kb: CTT TAG ATG GTG AGA OFG TTT ATG CAG G (SEQ ID NO: 16) |
| Frag9-F-10kb: TCT CAC CAT CTA AAG ZFA CGA TCC (SEQ ID NO: 17) |
| Frag9-R-10kb: CTG TTG GGT TAG ATC FFA TGG CG (SEQ ID NO: 18) |
| Frag10-F-10kb: GAT CTA ACC CAA CAG ZFG GTT C (SEQ ID NO: 19) |
| Frag10-R-10kb: CAC ATG CCT CCC TTT ZOC ACT TTT ATT G (SEQ ID NO: 20) |
| pLP-F-10kb: AAA GGG AGG CAT GTG FEC AAA AGG (SEQ ID NO: 21) |
| pLP-R2: GCC CAG CGT TCA GGC OEC GAT ATC ACC C (SEQ ID NO: 22) |
| DNA IUPAC 1-Letter Codes: |
| F = phosphorothioate-A base; O = phosphorothioate-C base; E = phosphorothioate-G base; Z = phosphorothioate-T base. |

| **Table 3:** Synthetic Strings |
|---|
| String1-F2 GGC CTA AAA GAC TCT FFC AAA ATA GCA AAT TTC G (SEQ ID NO: 23) |
| String 1-R: CCC ATT AGG CCA TTT OFG CAG (SEQ ID NO: 24) |
| String2-F: AAA TGG CCT AAT GGG ZZA CGA TGC TTT GTT CTT G (SEQ ID NO: 25) |
| String2-R: ACC TCT CCA ATA ATT ZET TCC AAG TAA CCA TCT TCA C (SEQ ID NO: 26) |
| String3-F: AAT TAT TGG AGA GGT ZET GTT GCT GAA GGT G (SEQ ID NO: 27) |
| String3-R: GCT TCA CCC ACA AAG OOA ATC TAG CAC (SEQ ID NO: 28) |
| String4-F: CTT TGT GGG TGA AGC ZEA TAG AGG TGA TG (SEQ ID NO: 29) |
| String4-R: TCT GGT CAT CTC TCA FOA ACA AAT CAC CC (SEQ ID NO: 30) |
| String5-F: TGA GAG ATG ACC AGA ZZT GGG TGC TAA ATT GCC (SEQ ID NO: 31) |
| String5-R: GTT CAG CAG TTC TCT ZOT TCT ATC ACC AG (SEQ ID NO: 32) |
| String6-F: AGA GAA CTG CTG AAC FFT TAC AAT TGG C (SEQ ID NO: 33) |
| String6-R: TTC TAG CCA AGG TTC OFA CAT GGA GGC (SEQ ID NO: 34) |
| String7-F: GAA CCT TGG CTA GAA EFT GTG AAA GAT TAT TGG (SEQ ID NO: 35) |
| String7-R: AAC CAG AAA GGC TCT OFT AGT AGG (SEQ ID NO: 36) |
| String8-F: AGA GCC TTT CTG GTT OZC CAT CTT TGA C (SEQ ID NO: 37) |
| String8-R: CTC AAA GCC GAA TCT EFT GGC AAT ACC TTG (SEQ ID NO: 38) |
| String9-F: AGA TTC GGC TTT GAG FEA TAA GTG TAG ATC (SEQ ID NO: 39) |
| String9-R: CCA ATA AGA CAG TAA OOA GAA GTC AAT T (SEQ ID NO: 40) |
| String 10-F: TTA CTG TCT TAT TGG ZOA CCA ATG TTG CC (SEQ ID NO: 41) |
| String 10-R: CAC ATG CTA TAG AAC OOG AAC GAC CGA GC (SEQ ID NO: 42) |
| pLP-F-string: GTT CTA TAG CAT GTG FEC AAA AGG CCA GC (SEQ ID NO: 43) |
| pLP-R2-string: AGA GTC TTT TAG GCC EOG ATA TCA CCC CTA (SEQ ID NO: 44) |

| **Table 4:** Tal Trimers |
|---|
| Tal-F1f: CGC GGA ACC TGA OOC CCG AAC (SEQ ID NO: 45) |
| Tal-F1r: CAG TCC GTG AGC OZG GCA CAG C (SEQ ID NO: 46) |
| Tal-F2f: gctcacggactgFOccccg (SEQ ID NO: 47) |
| Tal-F2r: TCA GCC CGT GAG OOT GGC AC (SEQ ID NO: 48) |
| Tal-F3f: ctcacgggctgaOOcccg (SEQ ID NO: 49) |
| Tal-F3r: CGG GGG TCA AAC OET GAG CCT G (SEQ ID NO: 50) |
| Tal-F4f: gtttgacccccgFFcagg (SEQ ID NO: 51) |
| Tal-F4r1plus4: TGT GAG GCC GTG FEC CTG GC (SEQ ID NO: 52) |
| V-Tal-F1plus4: CAC GGC CTC ACA ZET GAG CAA AAG G (SEQ ID NO: 53) |
| V-Tal-R: TCA GGT TCC GCG FZA TCA CCC CTA (SEQ ID NO: 54) |

**Assembly Method:** Ten 1 kb DNA fragments from either *M. genitalium, V. cholerae* or *C*. *violaceum* were PCR-amplified using phosphorothioate primers in the presence of either Phusion® DNA polymerase or AccuPrime™ Pfx DNA polymerase. To assemble synthetic DNA strings, synthetic DNAs were PCR-amplified using phosphorothioate primers. Linearized of pType IIs vector was also prepared by PCR amplification using phosphorothioate primers accordingly. PCR fragments were purified using standard PCR column. If the DNA concentration is too low (below 50 ng/µl), the DNA fragments can be mixed and concentrated using a Speed Vac. The DNA fragments were resuspended in 7 µl water. In a 10 µl assembly reaction, 75 ng of linear vector, 75 ng each of 10 PCR fragments, 2 µl of 5x reaction buffer, and 1 µl of 10 x enzyme mix were added. The reaction was initiated by the addition of enzyme mix, followed by incubation at room temperature for 1 hour. 3 µl of reaction mix was transformed into TOP10 competent cells and then incubated on ice for 30 minutes, followed by heat shock at 42°C for 30 seconds. Upon incubation on ice for 2 minutes, 250 µl of SOC medium was added to the transform reaction and incubated at 37°C for 1 hour. One hundred µl of cell suspension was spread on LB+Amp plates and incubated at 37°C overnight. Colonies were randomly picked and subjected to plasmid DNA isolation, followed by analysis of both restriction enzyme digestion and sequencing.

**Results and Discussion:** Because the phosphorothioate bonds stop the chew back reaction catalyzed by exonucleases at a specified location and generate perfect overhangs for homologous recombination, it was expected that the efficiency for DNA assembly would be higher than for assembly reactions using molecules not having phosphorothioate bonds, especially for large fragment assembly. To examine this, two sets of ten 1 kb fragments were designed that are PCR-amplified from either *M. genitalium* and *V. cholerae* using phosphorothioate primers, respectively. The DNA fragments share 15 bp homology at their ends. The assembly of ten 1 kb fragments plus linear vector was performed in triplicate as described above. The DNA fragments of *M. genitalium* also harbor a functional *LacZ* gene which was intentionally split into two adjacent fragments so that blue colonies were produced on X-gal plates once the DNA fragments were assembled correctly. As depicted in Table 5, about 2000 colonies per transformation were obtained. The cloning efficiency was more than 98% based on the calculation of percentage of blue colonies. To confirm the identity of the construct, 11 blue colonies were picked. Plasmid DNA was isolated from each of these colonies for restriction digestion analysis and sequencing analysis. Digestion of the 11 plasmids with *Bgl*II all generated three expected sizes of DNA fragments, which are 640bp, 2003bp and 8743bp (data not shown), respectively. Sequencing of three individual plasmids reveals that all three constructs had the correct sequences at the 11 junctions connecting the fragments and vector. Similar results were observed with the second set of ten 1 kb DNA fragments that were amplified from *V. cholerae.* As shown in Table 6, around 2000 CFU were obtained in two individual experiments. Ten colonies were randomly picked and subjected to restriction analysis with *Nco*I*.* Upon digestion, all ten clonal isolates showed the expected sizes of DNA fragments, which are 1263bp and 10396bp (data not shown), respectively.

| **Table 5:** One step assembly of 10 x 1 kb plus vector using phosphorothioate primers | | | |
|---|---|---|---|
| No. of White Colonies | 30 | 18 | 54 |
| No. of Blue Colonies | 1884 | 2172 | 1962 |
| % of Blue Colonies | 98.4% | 99.2% | 97.3% |
| AVG | 98.3%±0.9 | | |

| **Table 6:** Assembly of ten 1 kb fragments from *V. cholerae* | | |
|---|---|---|
| Exp# | 1 | 2 |
| CFU/rxn | 2280 | 1980 |
| CE | 100% | 100% |

Next this method was evaluated on the assembly of ten synthetic DNA fragments (strings). The synthetic strings were produced by GeneArt (Thermo Fisher) and PCR amplified using phosphorothioate primers. The quality of the PCR products was fair as some of the DNA fragments had minor truncated products. Average of 248 colonies was observed in the triplicate experiments (Table 7). Restriction digestion analysis with *Xmn*I produced three expected sizes of DNA fragments of 1563bp, 2317bp and 6kb (data not shown), suggesting that the efficiency of assembly is around 60%.

| **Table 7:** Assembly of ten synthetic strings using PS primers | | | |
|---|---|---|---|
| | Synthetic Strings | | |
| | 1 | 2 | 3 |
| CFU/rxn | 225 | 186 | 333 |
| Avg | 248±130 | | |
| CE | 60% | | |

The feasibility of using this PS approach for assembly of repetitive DNA fragments was also examiner. Tal repeat trimers having more than 90% homology were obtained from GeneArt® (Thermo Fisher). To minimize the cross-reactivity, the length of overlap was reduced from 15 bp to 12 bp. Four trimers of Tal repeats were PCR amplified using phosphorothioate primers and assembly simultaneously to produce a Tal effector containing 12 repeats. Around 28,000 colonies were observed. Five colonies were randomly picked for DNA sequencing. The results indicated that 4 out of 5 contained all four trimers of Tal repeats.

In conclusion, a robust assembly method was developed using phosphorothioate chemistry. Since T7exo hydrolyzes double stranded DNA from 5' to 3', it generates a 5' phosphate at a specified phosphorothioate nucleotide. Upon annealing to a complimentary strand, the double stranded DNA contains a nick bounded by 3'-OH and 5'-P termini. Ligase may be used to seal the gaps.

### Example 2: Positive Selection Assembly and Cloning

**Summary:** Here, a technique based on positive-selection vectors is presented. The strategy relies on vectors with a truncated and inactive replication origin and selection marker, whose short missing sequences are provided in *trans* during the cloning procedure. The approach i) provides selective survivability on the assembly products that have correct assembled outermost fragments and ii) reduces background colony growth due to recircularized vectors.

### Materials and Methods

**Strains:** Chemically or electro competent *Escherichia coli* strains, DH10B-T1 and TOP10, were obtained from Thermo Fisher Scientific. *E. coli* strain S17-1::λ*-pir* (de Lorenzo and Timmis, Analysis and construction of stable phenotypes in gram-negative bacteria with Tn5- and Tn10-derived minitransposons, Methods Enzymol. 235:386-405 (1994)) was used to maintain the positive-selection vector pASE101. Chemically competent yeast MaV203 strain (a part of the GeneArt® High-Order Genetic Assembly System kit) was obtained from Thermo Fisher Scientific. *E. coli* strains were grown in LB medium appropriate antibiotics: ampicillin (Ap, 50 µg/ml), kanamycin (Km, 25 µg/ml), and chloramphenicol (Cm, 20 µg/ml). Yeast MaV203 transformants were grown on CSM-Trp medium.

**Oligonucleotides, synthetic DNAs, and plasmids:** Oligonucleotides used in this study are listed in Table 8. Synthetic DNA strings were obtained from Thermo Fisher Scientific (GeneArt, Germany). A subset of these synthetic DNA fragments were cloned into pCR®-Blunt II-TOPO® (Thermo Fisher Scientific) Vector as indicated below. These pre-cloned DNA fragments were used as templates to produce PCR-amplified inserts. Then those three different types of DNA, synthetic, pre-cloned, and PCR-amplified, were used for DNA assembly tests. All DNA fragments for assembly test were listed in Table 9.

A 4,255-bp DNA fragment was amplified from pYES3/CT (Thermo Fisher Scientific) using a primer set (CH316 & CH371) and circularized by self-ligation to generate pYES8. A 2,848-bp linear positive-selection vector pYES8D for *in vivo* DNA assembly in yeast was PCR amplified from pYES8 using a primer set (CH327 and CH353), and was also circularized by self-ligation to maintain in *E. coli.* Three DNA fragments, 2micron ori-TR_pUC ori (1045 bp, CH353 & CH397) and TRP1-TR (871 bp, CH399 & CH401) from pYES8D and Km^{R} (1006 bp, CH396 & CH400) from pCR®-Blunt II-TOPO® Vector (ThermoFisher), were assembled using GeneArt® Seamless PLUS Cloning and Assembly Kit (ThermoFisher) to generate pYES10. A 1815 bp DNA fragment harboring pUC ori and ApR gene from pYES8 was amplified by PCR (CH423 & CH418) and self-ligated to produce pUC-Ap. A 1794 bp DNA fragment harboring pUC ori and ApR gene from pYES10 was amplified by PCR using a primer set (CH423 & CH418) and self-ligated to produce pUC-Km. A 1581 bp DNA fragment harboring truncated pUC ori (pUC ori-TR) and Km^{R} (Km^{R}-TR) was amplified from pUC-Km by PCR using a primer set (CH428 & CH438) and assembled with a 1223 bp PCR-amplified (CH450 & CH451) synthetic DNA fragment using GeneArt® Seamless PLUS Cloning and Assembly Kit (ThermoFisher) to generate pASE101. This vector can be maintained only in an *E. coli* strain harboring pir gene such as S17-1::λ*-pir.* A linear 1581 bp positive selection vector pASE101L was amplified from pASE101 by PCR using a primer set (CH428 & CH438). A linear 1603 bp control vector pASE_cont harboring functional pUC ori and Km^{R} was amplified from pASE101 by PCR using a primer set (CH476 & CH477). Phosphorothiate version of pASE101L and pASE_cont were amplified using phosphorothioate primer sets, CHPT1 & CHPT2 and CHPT3 & CHPT4.

**DNA assembly:** For *in vivo* assembly in yeast, the protocol for GeneArt® High-Order Genetic Assembly System (ThermoFisher) was followed using a modified amount of vector (50 ng) and inserts (50 ng each). For *in vitro* assembly and cloning in *E*. *coli,* both GeneArt® Seamless Cloning and Assembly Kit and GeneArt® Seamless PLUS Cloning and Assembly Kit (ThermoFisher) were used following the manufacturer's protocol using vector (75 ng) and inserts (75 ng each).

### Results and Conclusions

**Positive selection in *Saccharomyces cerevisiae:*** A map and sequence of the 2848 bp vector pYES8D is shown in FIG. 11 and Table 10. The plasmid encodes i) the β-lactamase gene and the replication origin (ori) from pUC19, ii) an inactive *S. cerevisiae* trp1 gene (Braus et al., The role of the TRP1 gene in yeast tryptophan biosynthesis, J. Biol. Chem. 263:7868-75 (1988)), and iii) a truncated ori from the yeast 2µ episome (Ludwig and Bruschi, The 2-micron plasmid as a nonselectable, stable, high copy number yeast vector, Plasmid 25:81-95 (1991)). Whereas the trp1 gene misses the last 21 bp of the otherwise active wild type open reading frame, the truncated 2µ ori lacks 10 bp (AGATAAACAT) (SEQ ID NO: 55) sufficient to provide full functionality (positions 1358 to 1367 of nucleotide sequence of pYES8, the wild-type counterpart (FIG. 12 and Table 11). The plasmid is PCR amplified with divergent oligonucleotides that anneal in between the inactive elements described above (position 2684 of its nucleotide sequence, Table 10) resulting in a linear vector ready to be used for cloning in yeast.

In a first cloning example 10 different fragments accounting for a total of 9868 bp were PCR amplified from *Vibrio cholerae's* genomic DNA and mixed with the linearized vector above (FIGs. 13 and 6). Adjacent fragments share 30 bp of homology at their corresponding ends for recombination. It is important to note that the first and 10th fragment contain the missing sequences of the truncated trp1 and 2µ ori at their 5' and 3' ends respectively plus 30 additional nucleotides required for recombination into the linearized vector. These additional sequences were added to the corresponding PCR primers resulting in 71 and 60mer oligonucleotides respectively (pilAD-1 and PilMQ-5 in Table 8).

The fragments and vector were transformed into competent MaV203 yeast cells, which were subsequently plated onto CSM-Trp agar plates as indicated in materials and methods. The cells are unable to grow on media lacking tryptophan, unless they are complemented by a plasmid harboring an active trp1 gene.

A series of control experiments were performed. First, a linear plasmid with intact and functional trp1 and 2µ ori elements, pYES8 (FIG. 12) was used instead of pYES8D. This plasmid is not subjected to positive selection, as it does not require complementing sequences for selection, replication and maintenance. Second, a DNA array lacking fragment number 6 was used instead of the otherwise complete 10-fragment set. In this case, a construct could not be assembled, as the necessary co-linearity for homologous recombination is broken. Finally, a vector only control was included for background growth assessment.

The results showed that the positive selection vector pYES8D promoted the recombination of the expected construct with an efficiency of 94%. In other words, 94 out of 100 colonies contained the right clone. In the absence of the positive selection feature, no correct clone could be obtained, despite the fact that a comparable number of colonies appeared on the plates. Lastly, the negative control experiments (no fragment number 6 and no insert controls) produced a significantly reduced number of colonies only if the positive control vector was employed.

In a second example, 10 synthetic DNA fragments were *in vitro* synthesized employing standard gene synthesis procedures (FIG. 6). In this case, the homology between adjacent fragments and between the outermost fragments and the vector was introduced during the gene synthesis procedure. Three different DNA sources were employed. First, the fragments were used as they were received from the DNA synthesis provider (FIG. 6, synthetic DNA). Second, the fragments were individually cloned into a carrier vector and then released by restriction endonuclease digestion procedures (FIG. 6, pre-cloned). And third the fragments were PCR amplified from the pre-cloned constructs above (FIG. 6, PCR product). Again, a series of negative controls were used where either the first, second, fourth, or tenth fragment was excluded from the assembly procedure. Additional experiments included the pYES8 vector as described above, and two no-insert control reactions.

The results showed that with the use of the positive selection vector pYES8D, the expected final construct could be obtained with cloning efficiencies ranging from 77 to 100%. Without positive selection, the expected clone was obtained at a significantly lower rate (compare assemblies 1 and 9 in FIG. 6). Negative controls showed considerably lower colony counts.

In conclusion, the positive selection vector approach in yeast significantly reduces the downstream screening effort compared with standard selection procedures, shortening the hands-on and overall time required to obtain the expected clone.

**Positive selection in *Escherichia coli*:** In this second example, the performance of the positive selection approach is shown in the context of *E. coli* cloning. In this case the vector, pASE101 (FIG. 14 and Table 12) harbors truncated non-functional pUC ori (pUC ori-TR) and kanamycin resistance elements (Km^{R}-TR), with 11-bp and 13-bp deletions respectively (compare sequences in FIG. 15 and Table 13). The vector pASE101 harbors a functional chloramphenicol resistance gene and the R6K ori, which restrict its propagation in *pir*+ *E. coli* strains such as S17-1::λ*-pir.* Therefore, standard *E. coli* K12, W, or B strains are non-viable in the presence of selection pressure. A subfragment of the vector encompassing only pUC ori-TR and Km^{R}-TR was PCR amplified using phosphorothioated oligonucleotides, as described in the materials and method section (FIGs. 9 and 16). This fragment was used as an acceptor for the cloning reactions described below.

A similar 10-fragment array as that one described in the previous section was employed as a source of inserts. In this particular case the fragments were PCR amplified using oligonucleotides harboring phosphorothioate bonds as described in materials and methods. The construct was assembled using the GeneART Seamless Assembly kit (Thermo Fisher Scientific), and transformed into TOP10 cells (Thermo Fisher Scientific). As a control, a similar construct was assembled using the vector pASE_cont (FIG. 16), which encodes functional pUC ori and kanamycin resistant markers (no positive selection).

The results show that the positive control vector strategy significantly increases the cloning efficiency compared with the approach where no positive selection is employed (cloning efficiencies of 71 and 45% respectively).

In conclusion, the positive selection approach can be applied to the most common *E*. *coli-based* cloning complementing and boosting the performance of otherwise standard cloning methodologies.

| **Table 8:** Oligonucleotides used in this study. | | | |
|---|---|---|---|
| Name | Sequence (5' to 3') | Relevant DNA fragment or construct | SEQ ID NO: |
| CH312 | | Vio-1 | 56 |
| CH313 | TCTTATTGGTCACCAATGTTGCCAGAC | Vio-10 | 57 |
| CH314 | | Vio-10 | 58 |
| CH316 | | pYES8 | 59 |
| CH317 | | pYES8 | 60 |
| CH327 | | pYES8D | 61 |
| CH353 | AAAAAATGTAGAGGTCGAGTTTAG | pYES8D, pYES10 | 62 |
| CH361 | TAAAAGACTCTAACAAAATAGC | Vio-1 | 63 |
| CH362 | ATGGGTTACGATGCTTTGTTC | Vio-2 | 64 |
| CH363 | TAATTTGTTCCAAGTAACCATC | Vio-2 | 65 |
| CH364 | TTGGAGAGGTTGTGTTGCTG | Vio-3 | 66 |
| CH365 | CCACAAAGCCAATCTAGCAC | Vio-3 | 67 |
| CH366 | GTGAAGCTGATAGAGGTGATG | Vio-4 | 68 |
| CH367 | TCATCTCTCAACAACAAATC | Vio-4 | 69 |
| CH368 | CCAGATTTGGGTGCTAAATTG | Vio-5 | 70 |
| CH369 | TCTCTTCTTCTATCACCAGAAC | Vio-5 | 71 |
| CH370 | ACTGCTGAACAATTACAATTG | Vio-6 | 72 |
| CH371 | GGTTCCAACATGGAGGCTTG | Vio-6 | 73 |
| CH372 | TTGGCTAGAAGATGTGAAAG | Vio-7 | 74 |
| CH373 | AAGGCTCTCATAGTAGGTTC | Vio-7 | 75 |
| CH374 | TCTGGTTCTCCATCTTTGAC | Vio-8 | 76 |
| CH375 | CTTTCGAATCTGATGGCAATAC | Vio-8 | 77 |
| CH376 | GCTTTGAGAGATAAGTGTAG | Vio-9 | 78 |
| CH377 | CAGTAACCAGAAGTCAATTG | Vio-9 | 79 |
| CH396 | | pYFS10 | 80 |
| CH397 | | pYFS10 | 81 |
| CH399 | GAAAAGTGCCACCTGACGT | pYES10 | 82 |
| CH428 | TCAAGAAGGCGATAGAAGG | pASE101 | 83 |
| CH438 | TTTTTTCTGCGCGTAATCTG | pASE101 | 84 |
| CH476 | | pASE_cont | 85 |
| CH477 | | pASE_cont | 86 |
| CH400 | | pYFS10 | 87 |
| CH401 | | pYES10 | 88 |
| | | | |
| CH450 | | pASE101 | 89 |
| CH451 | | pASE101 | 90 |
| CH452 | | PilAD-1 | 91 |
| CH453 | TCGGGCACCGAACTCCCCGAAG | PilAD-1 | 92 |
| CH454 | GTTAGCGCTTCGGGGAGTTC | PilAD-2 | 93 |
| CH455 | CGGCTGCACTTGCACTTGG | PilAD-2 | 94 |
| CH456 | TCTGGGATTAACCAAGTGCAAG | PilAD-3 | 95 |
| CH457 | TGCGCATCGCCTTGGAAAGTG | PilAD-3 | 96 |
| CH458 | CGGGCACGCCACTTTCCAAG | PilAD-4 | 97 |
| CH459 | TAGATCACCACATTGAGAAAG | PilAD-4 | 98 |
| CH460 | TGTCGGTAGCTTTCTCAATGTG | PilAD-5 | 99 |
| CH461 | | PilAD-5 | 100 |
| CH462 | | PilMQ-1 | 101 |
| CH463 | TGGACGTCGATACGCACGGCTAG | PilMQ-1 | 102 |
| CH464 | CAACTCGCTAGCCGTGCGTATC | PilMQ-2 | 103 |
| CH465 | TGGGGTTAAAAATTGGAAGGAG | PilMQ-2 | 104 |
| CH466 | TTTAAAGTCTCCTTCCAATTTTTAAC | PilMQ-3 | 105 |
| CH467 | GCCTTAACCTTGACCACACTC | PilMQ-3 | 106 |
| CH468 | GCCGGCAGGGAGTGTGGTCAAG | PilMQ-4 | 107 |
| CH469 | TCAGACAACATGTTCACGTTG | PilMQ-4 | 108 |
| CH470 | CGGCGGTGAAGGCAACGTGAAC | PilMQ-5 | 109 |
| CH471 | | PilMQ-5 | 110 |
| CH472 | | PilAD-1 (E.coli) | 111 |
| CH473 | | PilMQ-5 (E.coli) | 112 |
| CH478 | | VioAE-1 | 113 |
| CH479 | CAGGCTAAAACGCGCACCTG | VioAE-1 | 114 |
| CH480 | CAGGTGCGCGTTTTAGCCTG | VioAE-2 | 115 |
| CH481 | CAGACCGTCACCACGATCCG | VioAE-2 | 116 |
| CH482 | CGGATCGTGGTGACGGTCTG | VioAE-3 | 117 |
| CH483 | CTCGATACGATGCGGGATATC | VioAE-3 | 118 |
| CH484 | GATATCCCGCATCGTATCGAG | VioAE-4 | 119 |
| CH485 | CACGGTTGGTCAGCTCATTC | VioAE-4 | 120 |
| CH486 | GAATGAGCTGACCAACCGTG | VioAE-5 | 121 |
| CH487 | CAGCGGACGGAAATCCTCC | VioAE-5 | 122 |
| CH488 | GGAGGATTTCCGTCCGCTG | VioAE-6 | 123 |
| CH489 | TTACCTCCTTAAAGATCTTC | VioAE-6 | 124 |
| CH490 | GAAGATCTTTAAGGAGGTAA | VioAE-7 | 125 |
| CH491 | CGACGGTTTCGAACCAAAC | VioAE-7 | 126 |
| CH492 | GTTTGGTTCGAAACCGTCG | VioAE-8 | 127 |
| CH493 | | VioAE-8 | 128 |
| CHPT1 | TCA GAA GAA CTC GTC FFG AAG GCG | pASE_cont (PT) | 129 |
| CHPT2 | CTT GAG ATC CTT TTT ZZC TGC GCG | pASE_cont (PT) | 130 |
| CHPT3 | TCA AGA AGG CGA TAG FFG GCG ATG | pASE101L (PT) | 131 |
| CHPT4 | | pASE101L (PT) | 132 |
| CHPT5 | | PilAD-1 (PT) | 133 |
| CHPT6 | | PilAD-1 (PT) | 134 |
| CHPT7 | GAGTTCGGTGCCCGAEECGCTGCTTGAG | PilAD-2 (PT) | 135 |
| CHPT8 | | PilAD-2 (PT) | 136 |
| CHPT9 | | PilAD-3 (PT) | 137 |
| CHPT10 | | PilAD-3 (PT) | 138 |
| CHPT11 | | PilAD-4 (PT) | 139 |
| CHPT12 | | PilAD-4 (PT) | 140 |
| CHPT13 | | PilAD-5 (PT) | 141 |
| CHPT14 | | PilAD-5 (PT) | 142 |
| CHPT15 | | PilMQ-1 (PT) | 143 |
| CHPT16 | | PilMQ-1 (PT) | 144 |
| CHPT17 | | PilMQ-2 (PT) | 145 |
| CHPT18 | | PilMQ-2 (PT) | 146 |
| CHPT19 | | PilMQ-3 (PT) | 147 |
| CHPT20 | | PilMQ-3 (PT) | 148 |
| CHPT21 | | PilMQ-4 (PT) | 149 |
| CHPT22 | | PilMQ-4 (PT) | 150 |
| CHPT23 | | PilMQ-5 (PT) | 151 |
| CHPT24 | | PilAD-1 (PT) | 152 |
| PT, phosphorothioate; F, PT-deoxyadenine; O, PT-deoxycytosine; E, PT-deoxyguanidine; Z, PT-deoxythymidine | | | |

| **Table 9:** DNA fragments for assembly test in this study. | | | | | |
|---|---|---|---|---|---|
| DNA Fragment | Host | Size | DNA type | Primer set | Source |
| Vio-1 | Yeast | 600 bp | Synthetic | NA | *C. violaceum* |
| Vio-2 | Yeast | 600 bp | Synthetic | NA | *C. violaceum* |
| Vio-3 | Yeast | 750 bp | Synthetic | NA | *C. violaceum* |
| Vio-4 | Yeasti | 750 bp | Synthetic | NA | *C. violaceum* |
| Vio-5 | Yeast | 999 bp | Synthetic | NA | *C. violaceum* |
| Vio-6 | Yeast | 999 bp | Synthetic | NA | *C. violaceum* |
| Vio-7 | Yeast | 999 bp | Synthetic | NA | *C. violaceum* |
| Vio-8 | Yeast | 999 bp | Synthetic | NA | *C. violaceum* |
| Vio-9 | Yeast | 999 bp | Synthetic | NA | *C. violaceum* |
| Vio-10 | Yeast | 588 bp | Synthetic | NA | *C. violaceum* |
| Vio-1 (BamHI/XhoI) | Yeast | 589 bp | Pre-cloned | NA | *C. violaceum* |
| Vio-2 (BamHI/XhoI) | Yeast | 589 bp | Pre-cloned | NA | *C. violaceum* |
| Vio-3 (BamHI/XhoI) | Yeast | 739 bp | Pre-cloned | NA | *C. violaceum* |
| Vio-4 (BamHI/XhoI) | Yeasti | 988 bp | Pre-cloned | NA | *C. violaceum* |
| Vio-5 (BamHI/XhoI) | Yeast | 989 bp | Pre-cloned | NA | *C. violaceum* |
| Vio-6 (BamHI/XhoI) | Yeast | 989 bp | Pre-cloned | NA | *C. violaceum* |
| Vio-7 (BamHI/XhoI) | Yeast | 989 bp | Pre-cloned | NA | *C. violaceum* |
| Vio-8 (BamHI/XhoI) | Yeast | 989 bp | Pre-cloned | NA | *C. violaceum* |
| Vio-9 (BamHI/XhoI) | Yeast | 989 bp | Pre-cloned | NA | *C. violaceum* |
| Vio-10 (BamHI/XhoI) | Yeast | 577 bp | Pre-cloned | NA | *C. violaceum* |
| Vio-1 | Yeast | 580 bp | PCR amplified | CH312 & CH361 | *C. violaceum* |
| Vio-2 | Yeast | 680 bp | PCR amplified | CH362 & CH363 | *C. violaceum* |
| Vio-3 | Yeast | 730 bp | PCR amplified | CH364 & CH365 | *C. violaceum* |
| Vio-4 | Yeast | 730 bp | PCR amplified | CH366 & CH367 | *C. violaceum* |
| Vio-5 | Yeast | 980 bp | PCR amplified | CH368 & CH369 | *C. violaceum* |
| Vio-6 | Yeast | 980 bp | PCR amplified | CH370 & CH371 | *C. violaceum* |
| Vio-7 | Yeast | 980 bp | PCR amplified | CH372 & CH373 | *C. violaceum* |
| Vio-8 | Yeast | 980 bp | PCR amplified | CH374 & CH375 | *C. violaceum* |
| Vio-9 | Yeast | 980 bp | PCR amplified | CH376 & CH377 | *C. violaceum* |
| Vio-10 | Yeast | 568 bp | PCR amplified | CH313 & CH357 | *C. violaceum* |
| PilAD-1 | Yeast | 1051 bp | PCR amplified | CH452 & CH453 | *V. cholerae* |
| PilAD-2 | Yeast/E.coli | 1029 bp | PCR amplified | CH454 & CH455 | *V. cholerae* |
| PilAD-3 | Yeast/E.coli | 1030 bp | PCR amplified | CH456 & CH457 | *V. cholerae* |
| PilAD-4 | Yeast/E.coli | 1030 bp | PCR amplified | CH458 & CH459 | *V. cholerae* |
| PilAD-5 | Yeast/E.coli | 945 bp | PCR amplified | CH460 & CH461 | *V. cholerae* |
| PilMQ-1 | Yeast/E.coli | 1015 bp | PCR amplified | CH462 & CH463 | *V. cholerae* |
| PilMQ-2 | Yeast/E.coli | 1030 bp | PCR amplified | CH464 & CH465 | *V. cholerae* |
| PilMQ-3 | Yeast/E.coli | 1030 bp | PCR amplified | CH466 & CH467 | *V. cholerae* |
| PilMQ-4 | Yeast/E.coli | 1030 bp | PCR amplified | CH468 & CH469 | *V. cholerae* |
| PilMQ-5 | Yeast | 1041 bp | PCR amplified | CH470 & CH471 | *V. cholerae* |
| PilAD-1 (normal) | *E. coli* | 1026 bp | PCR amplified | CH472 & CH453 | *V. cholerae* |
| PilMQ-5 (normal) | *E. coli* | 1011 bp | PCR amplified | CH470 & CH473 | *V. cholerae* |
| PilAD-1 (PT) | *E. coli* | 1026 bp | PCR amplified | CHPT5 & CHPT6 | *V. cholerae* |
| PilAD-2 (PT) | *E. coli* | 1015 bp | PCR amplified | CHPT7 & CHPT8 | *V. cholerae* |
| PilAD-3 (PT) | *E. coli* | 1015 bp | PCR amplified | CHPT9 & CHPT 10 | *V. cholerae* |
| PilAD-4 (PT) | *E. coli* | 1015 bp | PCR amplified | CHPT11 & CHPT12 | *V. cholerae* |
| PilAD-5 (PT) | *E. coli* | 930 bp | PCR amplified | CHPT13 & CHPT14 | *V. cholerae* |
| PilMQ-1 (PT) | *E. coli* | 1000 bp | PCR amplified | CHPT15 & CHPT 16 | *V. cholerae* |
| PilMQ-2 (PT) | *E. coli* | 1015 bp | PCR amplified | CHPT17 & CHPT 18 | *V. cholerae* |
| PilMQ-3 (PT) | *E. coli* | 1015 bp | PCR amplified | CHPT19 & CHPT20 | *V. cholerae* |
| PilMQ-4 (PT) | *E. coli* | 1015 bp | PCR amplified | CHPT21 & CHPT22 | *V. cholerae* |
| PilMQ-5 (PT) | *E. coli* | 1011 bp | PCR amplified | CHPT23 & CHPT24 | *V. cholerae* |
| VioAE-1 | *E. coli* | 1031 bp | PCR amplified | CH478 & CH479 | *C. violaceum* |
| VioAE-2 | *E. coli* | 1021 bp | PCR amplified | CH480 & CH481 | *C. violaceum* |
| VioAE-3 | *E. coli* | 1013 bp | PCR amplified | CH482 & CH483 | *C. violaceum* |
| VioAE-4 | *E. coli* | 1027 bp | PCR amplified | CH484 & CH485 | *C. violaceum* |
| VioAE-5 | *E. coli* | 1020 bp | PCR amplified | CH486 & CH487 | *C. violaceum* |
| VioAE-6 | *E. coli* | 1009 bp | PCR amplified | CH488 & CH489 | *C. violaceum* |
| VioAE-7 | *E. coli* | 1028 bp | PCR amplified | CH490 & CH491 | *C. violaceum* |
| VioAE-8 | *E. coli* | 770 bp | PCR amplified | CH492 & CH493 | *C. violaceum* |
| VioAE-14 | *E. coli* | 4031 bp | PCR amplified | CH478 & CH485 | *C. violaceum* |
| VioAE-56 | *E. coli* | 2010 bp | PCR amplified | CH486 & CH489 | *C. violaceum* |
| VioAE-78 | *E. coli* | 1779 bp | PCR amplified | CH490 & CH493 | *C. violaceum* |
| VioAE-58 | *E. coli* | 3769 bp | PCR amplified | CH486 & CH493 | *C. violaceum* |
| PT, phosphorothioate; NA, not applicable | | | | | |

| **Table10:** pYES8D Sequence |
|---|
| |
| |

| **Table 11:** pYES8 Sequence |
|---|
| |
| |
| |

| **Table 12:** pASE101 Sequence |
|---|
| |
| |

| **Table 13:** pASE_Cont Sequence |
|---|
| |
| |

### Example 3: A Simple Method to Terminate GeneArt® Seamless Assembly Reaction Enable High Throughput Applications

The protocol below is directed, in part, to the termination of enzymatic reactions related to nucleic acid assembly. Once nucleic acid segments are fully assembled, the continued action of enzymes (*e.g*., exonucleases) can damage assembled nucleic acid molecules.

A linearized vector and DNA fragments is prepared as instructed in GeneArt® Seamless DNA assembly kit (Life Technologies, Catalog number A14606) manual. Add DNA mix in a volume of 10 µl to a thin-walled PCR tube or a well on a PCR plate. Add 10 µl of GeneArt Seamless DNA assembly enzyme mix, mix by pipetting up and down or flicking the tube. Brief spin down the liquid to the bottom of the tube (DO NOT exceed 5 seconds and 500 rpm). Incubate in a PCR machine with the following protocol if final construct is smaller than 13kb: 30 minutes at 25°C, then 10 minutes at 75°C, hold at 4°C. If final the construct is larger than 13kb, use the following protocol: 30 minutes at 25°C, 75 minutes at 75°C, then 60 minutes at 25°C, hold at 4°C. The reaction mixture can be stored at 25°C or lower temperature for up to 48 hours until transformation.

| **Table 14:** Nucleotide sequence of pcDNA Rad51 BLM Exol Vector | Element |
|---|---|
| Fragment 1: | CMV Promoter |
| | |
| Fragment 2: | Rad51 |
| | |
| Fragment 3: | Rad51 |
| | |
| | 2A Peptide |
| | BLM |
| Fragment 4: | |
| | |
| | BLM |
| | BLM |
| | |
| Fragment 5: | TK PolyA |
| | |
| | F1 Origin |
| | SV40 Promoter |

| **Table 14:**Nucleotide sequence of pcDNA Rad51 BLM Exol Vector | Element |
|---|---|
| | |
| Fragment 6: | hExo |
| | |

| **Table 14:** Nucleotide sequence of pcDNA Rad51 BLM Exol Vector | Element |
|---|---|
| | pUC Origin |
| | AmpR |

Table 14 shows the nucleotide sequence of the pcDNA Rad51 BLM Exol vector. Also, indicated in Table 14 are the nucleotide sequences of a number of vector elements. As shown in FIG. 17A-7B and in Table 14, a number of the vector element are partially encoded by different fragments/segments to are assembled to generate a replicable vector.

Examples of apparatuses, systems and methods for providing a simplified workflow for nucleic acid sequencing are described in this specification. The section headings used herein are for organizational purposes only and are not to be construed as limiting the described subject matter in any way.

While the foregoing examples have been described in some detail for purposes of clarity and understanding, it will be clear to one skilled in the art from a reading of this disclosure that various changes in form and detail can be made. For example, all the techniques, apparatuses, systems and methods described above can be used in various combinations.

## Claims

1. A method for covalently linking two double-stranded nucleic acid segments having a region of sequence complementarity at their termini, the method comprising:
(a) incubating the two double-stranded nucleic acid segments with an exonuclease under conditions that allow for digestion of termini of the two double-stranded nucleic acid segments to form complementary single-stranded regions on each double-stranded nucleic acid segment and hybridization of the complementary single-stranded regions, wherein each of the two double-stranded nucleic acid segments comprises an exonuclease resistant region within 30 nucleotides of the end of the complementary terminus, wherein the exonuclease resistant region contains a phosphorothioate bond, and
(b) covalently connecting at least one strand of the hybridized termini formed in (a) resulting in the linkage of the two double-stranded nucleic acid segments,
and wherein the junction between the exonuclease resistant region and the region of hybridization is determined by the region of sequence complementarity between the two double-stranded nucleic acid segments.

2. The method of claim 1, wherein steps (a) and (b) occur in the same tube or in the same reaction mixture.

3. The method of claim 1, wherein the two or more double-stranded nucleic acid segments are simultaneously contacted with an exonuclease and a ligase in step (a).

4. The method of claim 1, wherein a replicable nucleic acid molecule is formed.

5. The method of claim 1, wherein the two or more double-stranded nucleic acid segments are covalently linked to form a circular double-stranded nucleic acid molecule.

6. The method of claim 5, where the circular double-stranded nucleic acid molecule contains one or more selection marker or origin of replication that is reconstituted by the linking of different double-stranded nucleic acid segments.

7. The method of claim 3, wherein one or more enzyme contacting the double-stranded nucleic acid segments is partially or fully inactivated.

8. The method of claim 7, wherein the one or more enzyme is inactivated by heating.

9. A method for assembling a double-stranded nucleic acid molecule, the method comprising:
(a) incubating a first double-stranded nucleic acid segment with an exonuclease under conditions that allow for partial digestion of at least one terminus of the first double-stranded nucleic acid segment to form a single-stranded region, wherein the first double-stranded nucleic acid segment contains an exonuclease resistant region within 30 nucleotides of the at least one terminus, wherein the exonuclease resistant region contains a phosphorothioate bond,
(b) preparing a reaction mixture containing the digested first nucleic acid segment formed in (a) with an undigested second double-stranded nucleic acid segment under conditions that allow for the hybridization of the single-stranded region of the first nucleic acid segment with a terminus of the undigested second nucleic acid segment with sequence complementarity to form hybridized termini, and
(c) covalently connecting at least one strand of the hybridized termini formed in (b),
and wherein the junction between the exonuclease resistant region and the region of hybridization is determined by the region of sequence complementarity between the two double-stranded nucleic acid segments.

10. The method of claim 9, wherein the second double-stranded nucleic acid segment of (b) contains no exonuclease resistant regions.

11. The method of claim 9, wherein at least one terminus of the second double-stranded nucleic acid segment of (b) contains a single-stranded region with sequence complementarity to the single-stranded region of the digested first nucleic acid segment formed in step (a).

12. The method of claim 9, wherein the exonuclease is a 5' to 3' exonuclease.

13. The method of claim 9, wherein a functional exonuclease is present in step (b).

14. A method for assembling a nucleic acid molecule, the method comprising:
(a) incubating two or more double-stranded nucleic acid segments with an exonuclease under conditions that allow for partial digestion of at least one terminus of each of the two or more double-stranded nucleic acid segments to generate single-stranded termini, wherein at least two of the two or more double-stranded nucleic acid segments contain an exonuclease resistant region within 30 nucleotides of at least one of their termini, wherein the exonuclease resistant region contains a phosphorothioate bond,
(b) preparing a reaction mixture containing the digested nucleic acid segments prepared in (a) with one or more undigested double-stranded nucleic acid segment under conditions that allow for the hybridization of termini with sequence complementarity,
wherein at least one of the one or more undigested double-stranded nucleic acid segment has region of sequence complementarity with at least one single-stranded terminus formed in (a), and
(c) covalently connecting at least one strand of the hybridized termini formed in (b),
and wherein the junction between the exonuclease resistant region and the region of hybridization is determined by the region of sequence complementarity between the two double-stranded nucleic acid segments.

15. The method of claim 1 or claim 14, wherein the two or more double-stranded nucleic acid segments are covalently linked to one or more additional double-stranded nucleic acid segments that do not contain exonuclease resistant regions.

## Patentansprüche

1. Verfahren zum kovalenten Verknüpfen von zwei doppelsträngigen Nukleinsäuresegmenten, die einen Sequenzkomplementaritätsbereich an ihren Termini aufweisen, wobei das Verfahren Folgendes umfasst:
(a) Inkubieren der beiden doppelsträngigen Nukleinsäuresegmente mit einer Exonuklease, unter Bedingungen, die einen Verdau von Termini der beiden doppelsträngigen Nukleinsäuresegmente, um komplementäre einzelsträngige Bereiche auf jedem doppelsträngigen Nukleinsäuresegment auszubilden, und ein Hybridisieren der komplementären einzelsträngigen Bereiche ermöglichen, wobei jedes der beiden doppelsträngigen Nukleinsäuresegmente einen exonukleaseresistenten Bereich innerhalb von 30 Nukleotiden des Endes des komplementären Terminus umfasst, wobei der exonukleaseresistente Bereich eine Phosphorothioatbindung enthält, und
(b) kovalentes Verbinden wenigstens eines Strangs der in (a) ausgebildeten hybridisierten Termini, was zu der Verknüpfung der beiden doppelsträngigen Nukleinsäuresegmente führt, und wobei der Verbindungspunkt zwischen dem exonukleaseresistenten Bereich und dem Bereich der Hybridisierung durch den Sequenzkomplementaritätsbereich zwischen den beiden doppelsträngigen Nukleinsäuresegmenten bestimmt wird.

2. Verfahren nach Anspruch 1, wobei die Schritte (a) und (b) sich in demselben Röhrchen oder in demselben Reaktionsgemisch ereignen.

3. Verfahren nach Anspruch 1, wobei die zwei oder mehr doppelsträngigen Nukleinsäuresegmente gleichzeitig mit einer Exonuklease und einer Ligase in Schritt (a) in Berührung gebracht werden.

4. Verfahren nach Anspruch 1, wobei ein replizierbares Nukleinsäuremolekül ausgebildet wird.

5. Verfahren nach Anspruch 1, wobei die zwei oder mehr doppelsträngigen Nukleinsäuresegmente kovalent verknüpft sind, um ein kreisförmiges doppelsträngiges Nukleinsäuremolekül auszubilden.

6. Verfahren nach Anspruch 5, wobei das kreisförmige doppelsträngige Nukleinsäuremolekül einen oder mehrere Selektionsmarker oder Replikationsstartpunkte enthält, die durch das Verknüpfen verschiedener doppelsträngiger Nukleinsäuresegmente wiederhergestellt werden.

7. Verfahren nach Anspruch 3, wobei ein oder mehrere die doppelsträngigen Nukleinsäuresegmente berührende Enzyme teilweise oder vollständig inaktiviert sind.

8. Verfahren nach Anspruch 7, wobei die einen oder mehreren Enzyme durch Erhitzen inaktiviert sind.

9. Verfahren zum Zusammensetzen eines doppelsträngigen Nukleinsäuremoleküls, wobei das Verfahren Folgendes umfasst:
(a) Inkubieren eines ersten doppelsträngigen Nukleinsäuresegments mit einer Exonuklease, unter Bedingungen, die den teilweisen Verdau wenigstens eines Terminus des ersten doppelsträngigen Nukleinsäuresegments ermöglichen, um einen einzelsträngigen Bereich auszubilden, wobei das erste doppelsträngige Nukleinsäuresegment einen exonukleaseresistenten Bereich innerhalb von 30 Nukleotiden des wenigstens einen Terminus umfasst, wobei der exonukleaseresistente Bereich eine Phosphorothioatbindung enthält,
(b) Herstellen eines Reaktionsgemischs, das das in (a) ausgebildete verdaute erste Nukleinsäuresegment enthält mit einem unverdauten zweiten doppelsträngigen Nukleinsäuresegment, unter Bedingungen, die die Hybridisierung des einzelsträngigen Bereichs des ersten Nukleinsäuresegments mit einem Terminus des unverdauten zweiten Nukleinsäuresegments mit Sequenzkomplementarität ermöglichen, um hybridisierte Termini auszubilden, und
(c) kovalentes Verbinden wenigstens eines Strangs der in (b) ausgebildeten hybridisierten Termini und wobei der Verbindungspunkt zwischen dem exonukleaseresistenten Bereich und dem Bereich der Hybridisierung durch den Sequenzkomplementaritätsbereich zwischen den beiden doppelsträngigen Nukleinsäuresegmenten bestimmt wird.

10. Verfahren nach Anspruch 9, wobei das zweite doppelsträngige Nukleinsäuresegment von (b) keine exonukleaseresistenten Bereiche enthält.

11. Verfahren nach Anspruch 9, wobei wenigstens ein Terminus des zweiten doppelsträngigen Nukleinsäuresegments von (b) einen einzelsträngigen Bereich mit Sequenzkomplementarität zu dem einzelsträngigen Bereich des in Schritt (a) ausgebildeten verdauten ersten Nukleinsäuresegments enthält.

12. Verfahren nach Anspruch 9, wobei die Exonuklease eine 5'- bis 3'-Exonuklease ist.

13. Verfahren nach Anspruch 9, wobei in Schritt (b) eine funktionelle Exonuklease vorhanden ist.

14. Verfahren zum Zusammensetzen eines Nukleinsäuremoleküls, wobei das Verfahren Folgendes umfasst:
(a) Inkubieren von zwei oder mehr doppelsträngigen Nukleinsäuresegmenten mit einer Exonuklease, unter Bedingungen, die einen teilweisen Verdau wenigstens eines Terminus jedes der zwei oder mehr doppelsträngigen Nukleinsäuresegmente ermöglichen, um einzelsträngige Termini zu erzeugen, wobei wenigstens zwei der zwei oder mehr doppelsträngigen Nukleinsäuresegmente einen exonukleaseresistenten Bereich innerhalb von 30 Nukleotiden wenigstens eines ihrer Termini enthalten, wobei der exonukleaseresistente Bereich eine Phosphorothioatbindung enthält,
(b) Herstellen eines Reaktionsgemisches, das die in (a) hergestellten verdauten Nukleinsäuresegmente enthält, mit einem oder mehreren unverdauten doppelsträngigen Nukleinsäuresegmenten, unter Bedingungen, die die Hybridisierung von Termini mit Sequenzkomplementarität ermöglichen, wobei wenigstens eines der einen oder der mehreren unverdauten doppelsträngigen Nukleinsäuresegmente einen Sequenzkomplementaritätsbereich mit wenigstens einem in (a) ausgebildeten einzelsträngigen Terminus aufweist, und
(c) kovalentes Verbinden wenigstens eines Strangs der in (b) ausgebildeten hybridisierten Termini und wobei der Verbindungspunkt zwischen dem exonukleaseresistenten Bereich und dem Bereich der Hybridisierung durch den Sequenzkomplementaritätsbereich zwischen den beiden doppelsträngigen Nukleinsäuresegmenten bestimmt wird.

15. Verfahren nach Anspruch 1 oder 14, wobei die zwei oder mehr doppelsträngigen Nukleinsäuresegmente kovalent mit einem oder mehreren zusätzlichen doppelsträngigen Nukleinsäuresegmenten, die keine exonukleaseresistenten Bereiche enthalten, verknüpft sind.

## Revendications

1. Procédé pour lier de façon covalente deux segments d'acide nucléique bicaténaire ayant une région de complémentarité de séquence à leurs extrémités, le procédé consistant à :
(a) incuber les deux segments d'acide nucléique bicaténaire avec une exonucléase dans des conditions permettant la digestion des extrémités des deux segments d'acide nucléique bicaténaire pour former des régions complémentaires monocaténaires sur chaque segment d'acide nucléique bicaténaire et l'hybridation des régions monocaténaires complémentaires, dans lequel chacun des deux segments d'acide nucléique bicaténaire comprend une région résistante à l'exonucléase dans 30 nucléotides de l'extrémité de l'extrémité terminale complémentaire, dans lequel la région résistante à l'exonucléase contient une liaison phosphorothioate, et
(b) relier de manière covalente au moins un brin des extrémités hybrides formées en (a), entraînant la liaison des deux segments d'acide nucléique bicaténaire, et dans lequel la jonction entre la région résistante à l'exonucléase et la région d'hybridation est déterminée par la région de complémentarité de séquence entre les deux segments d'acide nucléique bicaténaire.

2. Procédé selon la revendication 1, dans lequel les étapes (a) et (b) se déroulent dans le même tube ou dans le même mélange réactionnel.

3. Procédé selon la revendication 1, dans lequel les deux ou plus segments d'acide nucléique bicaténaire sont simultanément mis en contact avec une exonucléase et une ligase dans l'étape (a).

4. Procédé selon la revendication 1, dans lequel une molécule d'acide nucléique réplicable est formée.

5. Procédé selon la revendication 1, dans lequel les deux ou plus segments d'acide nucléique bicaténaire sont liés par covalence pour former une molécule circulaire d'acide nucléique bicaténaire.

6. Procédé selon la revendication 5, dans lequel la molécule circulaire d'acide nucléique bicaténaire contient un ou plusieurs marqueurs de sélection ou origine de réplication reconstitué(es) par liaison de différents segments d'acide nucléique bicaténaire.

7. Procédé selon la revendication 3, dans lequel une ou plusieurs enzymes en contact avec les segments d'acide nucléique bicaténaire est partiellement ou totalement inactivée.

8. Procédé selon la revendication 7, dans lequel une ou plusieurs enzymes est inactivée par chauffage.

9. Procédé d'assemblage d'une molécule d'acide nucléique bicaténaire, le procédé consistant à :
(a) incuber un premier segment d'acide nucléique bicaténaire avec une exonucléase dans des conditions permettant la digestion partielle d'au moins une extrémité du premier segment d'acide nucléique bicaténaire pour former une région monocaténaire, dans laquelle le premier le segment d'acide nucléique bloqué contient une région résistante à l'exonucléase dans 30 nucléotides de ladite extrémité, dans laquelle la région résistante à l'exonucléase contient une liaison phosphorothioate,
(b) préparer un mélange réactionnel contenant le premier segment d'acide nucléique digéré formé en (a) avec un second segment d'acide nucléique bicaténaire non digéré dans des conditions qui permettent l'hybridation de la région monocaténaire du premier segment d'acide nucléique avec une extrémité du second segment d'acide nucléique non digéré ayant une complémentarité de séquence pour former des extrémités hybrides, et
(c) relier de manière covalente au moins un brin des extrémités hybrides formées en (b) et dans lequel la jonction entre la région résistante à l'exonucléase et la région d'hybridation est déterminée par la région de complémentarité de séquence entre les deux segments d'acide nucléique bicaténaire.

10. Procédé selon la revendication 9, dans lequel le second segment d'acide nucléique bicaténaire de (b) ne contient pas de régions résistantes à l'exonucléase.

11. Procédé selon la revendication 9, dans lequel au moins une extrémité du second segment d'acide nucléique bicaténaire de (b) contient une région monocaténaire ayant une complémentarité de séquence avec la région monocaténaire du premier segment d'acide nucléique digéré formé à l'étape (a).

12. Procédé selon la revendication 9, dans lequel l'exonucléase est une exonucléase de 5' à 3'.

13. Procédé selon la revendication 9, dans lequel une exonucléase fonctionnelle est présente à l'étape (b).

14. Procédé d'assemblage d'une molécule d'acide nucléique, le procédé consistant à :
(a) incuber deux ou plus segments d'acide nucléique bicaténaire avec une exonucléase dans des conditions permettant la digestion partielle d'au moins une extrémité de chacun des deux segments ou plus d'acide nucléique bicaténaire pour générer des terminaisons monocaténaires, dans lequel au moins deux des deux ou plus segments d'acide nucléique bicaténaire contiennent une région résistante à l'exonucléase dans 30 nucléotides d'au moins l'une de leurs extrémités, la région résistante à l'exonucléase contenant une liaison phosphorothioate,
(b) préparer un mélange réactionnel contenant les segments d'acide nucléique digéré préparé en (a) avec un ou plusieurs segments d'acide nucléique bicaténaire non digéré dans des conditions qui permettent l'hybridation des extrémités ayant une complémentarité de séquence, dans lequel au moins l'une d'une ou plus des segments d'acide nucléique bicaténaire non digéré possède une région de complémentarité de séquence ayant au moins une extrémité monocaténaire formée en (a), et
(c) relier de manière covalente au moins un brin des extrémités hybrides formées en (b) et dans lequel la jonction entre la région résistante à l'exonucléase et la région d'hybridation est déterminée par la région de complémentarité de séquence entre les deux segments d'acide nucléique bicaténaire.

15. Procédé selon la revendication 1 ou la revendication 14, dans lequel les deux ou plus segments d'acide nucléique bicaténaire sont liés par covalence à un ou plus segments supplémentaires d'acide nucléique bicaténaire qui ne contiennent pas de régions résistantes à l'exonucléase.


### SEQUENCE LISTING

<110> LIFE TECHNOLOGIES CORPORATION
<120> COMPOSITIONS AND METHODS FOR NUCLEIC ACID ASSEMBLY
<130> LT00899 PCT
<140>
   <141>
<150> 62/024,650
   <151> 2014-07-15
<160> 162
<170> PatentIn version 3.5
<210> 1
   <211> 31
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-G base
<400> 1
   tgctggagtg aacgctgggc cgagcgcaaa g 31
<210> 2
   <211> 23
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-G base
<400> 2
   gcaagaaaac tatcccgacc gcc 23
<210> 3
   <211> 25
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-G base
<400> 3
   ggatagtttt cttgcggccc taatc 25
<210> 4
   <211> 22
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-G base
<400> 4
   cgtctgggac tgggtggatc ag 22
<210> 5
   <211> 22
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-A base
<400> 5
   acccagtccc agacgaagcc gc 22
<210> 6
   <211> 27
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-T base
<400> 6
   cagatgtgcg gcgagttgcg tgactac 27
<210> 7
   <211> 24
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-A base
<400> 7
   ctcgccgcac atctgaactt cagc 24
<210> 8
   <211> 24
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-T base
<400> 8
   cgcagtggaa gatagatctg attg 24
<210> 9
   <211> 22
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-G base
<400> 9
   ctatcttcca ctgcgagttg aa 22
<210> 10
   <211> 25
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-T base
<400> 10
   agtgcagttg gtggagttgt tgatg 25
<210> 11
   <211> 24
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-G base
<400> 11
   tccaccaact gcactaggag attg 24
<210> 12
   <211> 27
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-A base
<400> 12
   agcaaggtga gattgaaact aggattg 27
<210> 13
   <211> 25
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-T base
<400> 13
   caatctcacc ttgctgtgct ttagc 25
<210> 14
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-G base
<400> 14
   tcttgcccta gcagttggtc ataccaac 28
<210> 15
   <211> 30
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-C base
<400> 15
   actgctaggg caagaaccac caccaaatag 30
<210> 16
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-A base
<400> 16
   ctttagatgg tgagacagtt tatgcagg 28
<210> 17
   <211> 24
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-A base
<400> 17
   tctcaccatc taaagtaacg atcc 24
<210> 18
   <211> 23
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-A base
<400> 18
   ctgttgggtt agatcaaatg gcg 23
<210> 19
   <211> 22
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-A base
<400> 19
   gatctaaccc aacagtaggt tc 22
<210> 20
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-C base
<400> 20
   cacatgcctc ccttttccac ttttattg 28
<210> 21
   <211> 24
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-G base
<400> 21
   aaagggaggc atgtgagcaa aagg 24
<210> 22
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-G base
<400> 22
   gcccagcgtt caggccgcga tatcaccc 28
<210> 23
   <211> 34
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-A base
<400> 23
   ggcctaaaag actctaacaa aatagcaaat ttcg 34
<210> 24
   <211> 21
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-A base
<400> 24
   cccattaggc catttcagca g 21
<210> 25
   <211> 34
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-T base
<400> 25
   aaatggccta atgggttacg atgctttgtt cttg 34
<210> 26
   <211> 37
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-G base
<400> 26
   acctctccaa taatttgttc caagtaacca tcttcac 37
<210> 27
   <211> 31
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-G base
<400> 27
   aattattgga gaggttgtgt tgctgaaggt g 31
<210> 28
   <211> 27
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-C base
<400> 28
   gcttcaccca caaagccaat ctagcac 27
<210> 29
   <211> 29
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-G base
<400> 29
   ctttgtgggt gaagctgata gaggtgatg 29
<210> 30
   <211> 29
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-C base
<400> 30
   tctggtcatc tctcaacaac aaatcaccc 29
<210> 31
   <211> 33
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-T base
<400> 31
   tgagagatga ccagatttgg gtgctaaatt gcc 33
<210> 32
   <211> 29
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-C base
<400> 32
   gttcagcagt tctcttcttc tatcaccag 29
<210> 33
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-A base
<400> 33
   agagaactgc tgaacaatta caattggc 28
<210> 34
   <211> 27
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-A base
<400> 34
   ttctagccaa ggttccaaca tggaggc 27
<210> 35
   <211> 33
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-A base
<400> 35
   gaaccttggc tagaagatgt gaaagattat tgg 33
<210> 36
   <211> 24
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-A base
<400> 36
   aaccagaaag gctctcatag tagg 24
<210> 37
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-T base
<400> 37
   agagcctttc tggttctcca tctttgac 28
<210> 38
   <211> 30
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-A base
<400> 38
   ctcaaagccg aatctgatgg caataccttg 30
<210> 39
   <211> 30
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-G base
<400> 39
   agattcggct ttgagagata agtgtagatc 30
<210> 40
   <211> 28
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-C base
<400> 40
   ccaataagac agtaaccaga agtcaatt 28
<210> 41
   <211> 29
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-T base
<220>
   <221> modified_base
   <222> (17)..(17)
   <223> Phosphorothioate-C base
<400> 41
   ttactgtctt attggtcacc aatgttgcc 29
<210> 42
   <211> 29
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-C base
<220>
   <221> modified_base
   <222> (17)..(17)
   <223> Phosphorothioate-C base
<400> 42
   cacatgctat agaacccgaa cgaccgagc 29
<210> 43
   <211> 29
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-A base
<220>
   <221> modified_base
   <222> (17)..(17)
   <223> Phosphorothioate-G base
<400> 43
   gttctatagc atgtgagcaa aaggccagc 29
<210> 44
   <211> 30
   <212> DNA
   <213> Artificial Sequence
<220>
   <221> source
   <223> /note="Description of Artificial Sequence: Synthetic primer"
<220>
   <221> modified_base
   <222> (16)..(16)
   <223> Phosphorothioate-G base
<220>
   <221> modified_base
   <222> (17)..(17)
   <223> Phosphorothioate-C base
<400> 44
   agagtctttt aggccgcgat atcaccccta 30
<210> 45
   <211> 21
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-C base
<220>
   <221> modified_base
   <222> (14)..(14)
   <223> Phosphorothioate-C base
<400> 45
   cgcggaacct gacccccgaa c 21
<210> 46
   <211> 22
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-C base
<220>
   <221> modified_base
   <222> (14)..(14)
   <223> Phosphorothioate-T base
<400> 46
   cagtccgtga gcctggcaca gc 22
<210> 47
   <211> 19
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-A base
<220>
   <221> modified_base
   <222> (14)..(14)
   <223> Phosphorothioate-C base
<400> 47
   gctcacggac tgacccccg 19
<210> 48
   <211> 20
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-C base
<220>
   <221> modified_base
   <222> (14)..(14)
   <223> Phosphorothioate-C base
<400> 48
   tcagcccgtg agcctggcac 20
<210> 49
   <211> 18
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-C base
<220>
   <221> modified_base
   <222> (14)..(14)
   <223> Phosphorothioate-C base
<400> 49
   ctcacgggct gacccccg 18
<210> 50
   <211> 22
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-C base
<220>
   <221> modified_base
   <222> (14)..(14)
   <223> Phosphorothioate-G base
<400> 50
   cgggggtcaa accgtgagcc tg 22
<210> 51
   <211> 18
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-A base
<220>
   <221> modified_base
   <222> (14)..(14)
   <223> Phosphorothioate-A base
<400> 51
   gtttgacccc cgaacagg 18
<210> 52
   <211> 20
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-A base
<220>
   <221> modified_base
   <222> (14)..(14)
   <223> Phosphorothioate-G base
<400> 52
   tgtgaggccg tgagcctggc 20
<210> 53
   <211> 25
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-T base
<220>
   <221> modified_base
   <222> (14)..(14)
   <223> Phosphorothioate-G base
<400> 53
   cacggcctca catgtgagca aaagg 25
<210> 54
   <211> 24
   <212> DNA
   <213> Artificial Sequence
<220>
   <221> source
   <223> /note="Description of Artificial Sequence: Synthetic primer"
<220>
   <221> modified_base
   <222> (13)..(13)
   <223> Phosphorothioate-A base
<220>
   <221> modified_base
   <222> (14)..(14)
   <223> Phosphorothioate-T base
<400> 54
   tcaggttccg cgatatcacc ccta 24
<210> 55
   <211> 10
   <212> DNA
   <213> Artificial Sequence
<220>
   <221> source
   <223> /note="Description of Artificial Sequence: Synthetic oligonucleotide"
<400> 55
   agataaacat 10
<210> 56
   <211> 30
   <212> DNA
   <213> Artificial Sequence
<220>
   <221> source
   <223> /note="Description of Artificial Sequence: Synthetic oligonucleotide"
<400> 56
   taggccattt cagcagaaat atctggcaag 30
<210> 57
   <211> 27
   <212> DNA
   <213> Artificial Sequence
<220>
   <221> source
   <223> /note="Description of Artificial Sequence: Synthetic oligonucleotide"
<400> 57
   tcttattggt caccaatgtt gccagac 27
<210> 58
   <211> 29
   <212> DNA
   <213> Artificial Sequence
<220>
   <221> source
   <223> /note="Description of Artificial Sequence: Synthetic oligonucleotide"
<400> 58
   ttatctctta gcagcaaaaa cagcatctg 29
<210> 59
   <211> 32
   <212> DNA
   <213> Artificial Sequence
<220>
   <221> source
   <223> /note="Description of Artificial Sequence: Synthetic oligonucleotide"
<400> 59
   ccaaagcttc aggggataac gcaggaaaga ac 32
<210> 60
   <211> 36
   <212> DNA
   <213> Artificial Sequence
<220>
   <221> source
   <223> /note="Description of Artificial Sequence: Synthetic oligonucleotide"
<400> 60
   ttgaagcttt ctgattatca accggggtgg agcttc 36
<210> 61
   <211> 40
   <212> DNA
   <213> Artificial Sequence
<220>
   <221> source
   <223> /note="Description of Artificial Sequence: Synthetic oligonucleotide"
<400> 61
   gaaatttgct attttgttag agtcttttac accatttgtc 40
<210> 62
   <211> 24
   <212> DNA
   <213> Artificial Sequence
<220>
   <221> source
   <223> /note="Description of Artificial Sequence: Synthetic oligonucleotide"
<400> 62
   aaaaaatgta gaggtcgagt ttag 24
<210> 63
   <211> 22
   <212> DNA
   <213> Artificial Sequence
<220>
   <221> source
   <223> /note="Description of Artificial Sequence: Synthetic oligonucleotide"
<400> 63
   taaaagactc taacaaaata gc 22
<210> 64
   <211> 21
   <212> DNA
   <213> Artificial Sequence
<220>
   <221> source
   <223> /note="Description of Artificial Sequence: Synthetic oligonucleotide"
<400> 64
   atgggttacg atgctttgtt c 21
<210> 65
   <211> 22
   <212> DNA
   <213> Artificial Sequence
<220>
   <221> source
   <223> /note="Description of Artificial Sequence: Synthetic oligonucleotide"
<400> 65
   taatttgttc caagtaacca tc 22
<210> 66
   <211> 20
   <212> DNA
   <213> Artificial Sequence
<220>
   <221> source
   <223> /note="Description of Artificial Sequence: Synthetic oligonucleotide"
<400> 66
   ttggagaggt tgtgttgctg 20
<210> 67
   <211> 20
   <212> DNA
   <213> Artificial Sequence
<220>
   <221> source
   <223> /note="Description of Artificial Sequence: Synthetic oligonucleotide"
<400> 67
   ccacaaagcc aatctagcac 20
<210> 68
   <211> 21
   <212> DNA
   <213> Artificial Sequence
<220>
   <221> source
   <223> /note="Description of Artificial Sequence: Synthetic oligonucleotide"
<400> 68
   gtgaagctga tagaggtgat g 21
<210> 69
   <211> 20
   <212> DNA
   <213> Artificial Sequence
<220>
   <221> source
   <223> /note="Description of Artificial Sequence: Synthetic oligonucleotide"
<400> 69
   tcatctctca acaacaaatc 20
<210> 70
   <211> 21
   <212> DNA
   <213> Artificial Sequence
<220>
   <221> source
   <223> /note="Description of Artificial Sequence: Synthetic oligonucleotide"
<400> 70
   ccagatttgg gtgctaaatt g 21
<210> 71
   <211> 22
   <212> DNA
   <213> Artificial Sequence
<220>
   <221> source
   <223> /note="Description of Artificial Sequence: Synthetic oligonucleotide"
<400> 71
   tctcttcttc tatcaccaga ac 22
<210> 72
   <211> 21
   <212> DNA
   <213> Artificial Sequence
<220>
   <221> source
   <223> /note="Description of Artificial Sequence: Synthetic oligonucleotide"
<400> 72
   actgctgaac aattacaatt g 21
<210> 73
   <211> 20
   <212> DNA
   <213> Artificial Sequence
<220>
   <221> source
   <223> /note="Description of Artificial Sequence: Synthetic oligonucleotide"
<400> 73
   ggttccaaca tggaggcttg 20
<210> 74
   <211> 20
   <212> DNA
   <213> Artificial Sequence
<220>
   <221> source
   <223> /note="Description of Artificial Sequence: Synthetic oligonucleotide"
<400> 74
   ttggctagaa gatgtgaaag 20
<210> 75
   <211> 20
   <212> DNA
   <213> Artificial Sequence
<220>
   <221> source
   <223> /note="Description of Artificial Sequence: Synthetic oligonucleotide"
<400> 75
   aaggctctca tagtaggttc 20
<210> 76
   <211> 20
   <212> DNA
   <213> Artificial Sequence
<220>
   <221> source
   <223> /note="Description of Artificial Sequence: Synthetic oligonucleotide"
<400> 76
   tctggttctc catctttgac 20
<210> 77
   <211> 22
   <212> DNA
   <213> Artificial Sequence
<220>
   <221> source
   <223> /note="Description of Artificial Sequence: Synthetic oligonucleotide"
<400> 77
   ctttcgaatc tgatggcaat ac 22
<210> 78
   <211> 20
   <212> DNA
   <213> Artificial Sequence
<220>
   <221> source
   <223> /note="Description of Artificial Sequence: Synthetic oligonucleotide"
<400> 78
   gctttgagag ataagtgtag 20
<210> 79
   <211> 20
   <212> DNA
   <213> Artificial Sequence
<220>
   <221> source
   <223> /note="Description of Artificial Sequence: Synthetic oligonucleotide"
<400> 79
   cagtaaccag aagtcaattg 20
<210> 80
   <211> 41
   <212> DNA
   <213> Artificial Sequence
<220>
   <221> source
   <223> /note="Description of Artificial Sequence: Synthetic oligonucleotide"
<400> 80
   gagtaaactt ggtctgacag tcagaagaac tcgtcaagaa g 41
<210> 81
   <211> 41
   <212> DNA
   <213> Artificial Sequence
<220>
   <221> source
   <223> /note="Description of Artificial Sequence: Synthetic oligonucleotide"
<400> 81
   cttcttgacg agttcttctg actgtcagac caagtttact c 41
<210> 82
   <211> 19
   <212> DNA
   <213> Artificial Sequence
<220>
   <221> source
   <223> /note="Description of Artificial Sequence: Synthetic oligonucleotide"
<400> 82
   gaaaagtgcc acctgacgt 19
<210> 83
   <211> 19
   <212> DNA
   <213> Artificial Sequence
<220>
   <221> source
   <223> /note="Description of Artificial Sequence: Synthetic oligonucleotide"
<400> 83
   tcaagaaggc gatagaagg 19
<210> 84
   <211> 20
   <212> DNA
   <213> Artificial Sequence
<220>
   <221> source
   <223> /note="Description of Artificial Sequence: Synthetic oligonucleotide"
<400> 84
   ttttttctgc gcgtaatctg 20
<210> 85
   <211> 32
   <212> DNA
   <213> Artificial Sequence
<220>
   <221> source
   <223> /note="Description of Artificial Sequence: Synthetic oligonucleotide"
<400> 85
   cttgagatcc tttttttctg cgcgtaatct gc 32
<210> 86
   <211> 37
   <212> DNA
   <213> Artificial Sequence
<220>
   <221> source
   <223> /note="Description of Artificial Sequence: Synthetic oligonucleotide"
<400> 86
   tcagaagaac tcgtcaagaa ggcgatagaa ggcgatg 37
<210> 87
   <211> 48
   <212> DNA
   <213> Artificial Sequence
<220>
   <221> source
   <223> /note="Description of Artificial Sequence: Synthetic oligonucleotide"
<400> 87
   tggtttctta gacgtcaggt ggcacttttc aaccggaatt gccagctg 48
<210> 88
   <211> 64
   <212> DNA
   <213> Artificial Sequence
<220>
   <221> source
   <223> /note="Description of Artificial Sequence: Synthetic oligonucleotide"
<400> 88
   ctaaactcga cctctacatt ttttgaaatt tgctattttg ttagagtctt ttacaccatt 60
   tgtc 64
<210> 89
   <211> 39
   <212> DNA
   <213> Artificial Sequence
<220>
   <221> source
   <223> /note="Description of Artificial Sequence: Synthetic oligonucleotide"
<400> 89
   gccttctatc gccttctttg agctcataca cccaaacag 39
<210> 90
   <211> 39
   <212> DNA
   <213> Artificial Sequence
<220>
   <221> source
   <223> /note="Description of Artificial Sequence: Synthetic oligonucleotide"
<400> 90
   agattacgcg cagaaaaaac aagaattctt actacgcac 39
<210> 91
   <211> 71
   <212> DNA
   <213> Artificial Sequence
<220>
   <221> source
   <223> /note="Description of Artificial Sequence: Synthetic oligonucleotide"
<400> 91
   gtaaaagact ctaacaaaat agcaaatttc gtcaaaaatg ctaagaaata gactttagcc 60
   ttgagatgat g 71
<210> 92
   <211> 22
   <212> DNA
   <213> Artificial Sequence
<220>
   <221> source
   <223> /note="Description of Artificial Sequence: Synthetic oligonucleotide"
<400> 92
   tcgggcaccg aactccccga ag 22
<210> 93
   <211> 20
   <212> DNA
   <213> Artificial Sequence
<220>
   <221> source
   <223> /note="Description of Artificial Sequence: Synthetic oligonucleotide"
<400> 93
   gttagcgctt cggggagttc 20
<210> 94
   <211> 19
   <212> DNA
   <213> Artificial Sequence
<220>
   <221> source
   <223> /note="Description of Artificial Sequence: Synthetic oligonucleotide"
<400> 94
   cggctgcact tgcacttgg 19
<210> 95
   <211> 22
   <212> DNA
   <213> Artificial Sequence
<220>
   <221> source
   <223> /note="Description of Artificial Sequence: Synthetic oligonucleotide"
<400> 95
   tctgggatta accaagtgca ag 22
<210> 96
   <211> 21
   <212> DNA
   <213> Artificial Sequence
<220>
   <221> source
   <223> /note="Description of Artificial Sequence: Synthetic oligonucleotide"
<400> 96
   tgcgcatcgc cttggaaagt g 21
<210> 97
   <211> 20
   <212> DNA
   <213> Artificial Sequence
<220>
   <221> source
   <223> /note="Description of Artificial Sequence: Synthetic oligonucleotide"
<400> 97
   cgggcacgcc actttccaag 20
<210> 98
   <211> 21
   <212> DNA
   <213> Artificial Sequence
<220>
   <221> source
   <223> /note="Description of Artificial Sequence: Synthetic oligonucleotide"
<400> 98
   tagatcacca cattgagaaa g 21
<210> 99
   <211> 22
   <212> DNA
   <213> Artificial Sequence
<220>
   <221> source
   <223> /note="Description of Artificial Sequence: Synthetic oligonucleotide"
<400> 99
   tgtcggtagc tttctcaatg tg 22
<210> 100
   <211> 45
   <212> DNA
   <213> Artificial Sequence
<220>
   <221> source
   <223> /note="Description of Artificial Sequence: Synthetic oligonucleotide"
<400> 100
   ccgattggta tcacgcacgt cacgtgcgat cacatcggca tcgac 45
<210> 101
   <211> 45
   <212> DNA
   <213> Artificial Sequence
<220>
   <221> source
   <223> /note="Description of Artificial Sequence: Synthetic oligonucleotide"
<400> 101
   atcgcacgtg acgtgcgtga taccaatcgg gtcaaaaccg tagtg 45
<210> 102
   <211> 23
   <212> DNA
   <213> Artificial Sequence
<220>
   <221> source
   <223> /note="Description of Artificial Sequence: Synthetic oligonucleotide"
<400> 102
   tggacgtcga tacgcacggc tag 23
<210> 103
   <211> 22
   <212> DNA
   <213> Artificial Sequence
<220>
   <221> source
   <223> /note="Description of Artificial Sequence: Synthetic oligonucleotide"
<400> 103
   caactcgcta gccgtgcgta tc 22
<210> 104
   <211> 22
   <212> DNA
   <213> Artificial Sequence
<220>
   <221> source
   <223> /note="Description of Artificial Sequence: Synthetic oligonucleotide"
<400> 104
   tggggttaaa aattggaagg ag 22
<210> 105
   <211> 26
   <212> DNA
   <213> Artificial Sequence
<220>
   <221> source
   <223> /note="Description of Artificial Sequence: Synthetic oligonucleotide"
<400> 105
   tttaaagtct ccttccaatt tttaac 26
<210> 106
   <211> 21
   <212> DNA
   <213> Artificial Sequence
<220>
   <221> source
   <223> /note="Description of Artificial Sequence: Synthetic oligonucleotide"
<400> 106
   gccttaacct tgaccacact c 21
<210> 107
   <211> 22
   <212> DNA
   <213> Artificial Sequence
<220>
   <221> source
   <223> /note="Description of Artificial Sequence: Synthetic oligonucleotide"
<400> 107
   gccggcaggg agtgtggtca ag 22
<210> 108
   <211> 21
   <212> DNA
   <213> Artificial Sequence
<220>
   <221> source
   <223> /note="Description of Artificial Sequence: Synthetic oligonucleotide"
<400> 108
   tcagacaaca tgttcacgtt g 21
<210> 109
   <211> 22
   <212> DNA
   <213> Artificial Sequence
<220>
   <221> source
   <223> /note="Description of Artificial Sequence: Synthetic oligonucleotide"
<400> 109
   cggcggtgaa ggcaacgtga ac 22
<210> 110
   <211> 60
   <212> DNA
   <213> Artificial Sequence
<220>
   <221> source
   <223> /note="Description of Artificial Sequence: Synthetic oligonucleotide"
<400> 110
   ttgcatctaa actcgacctc tacatttttt atgtttatct ttcctcaccg atatttcgtg 60
<210> 111
   <211> 51
   <212> DNA
   <213> Artificial Sequence
<220>
   <221> source
   <223> /note="Description of Artificial Sequence: Synthetic oligonucleotide"
<400> 111
   cagattacgc gcagaaaaaa aggatctcaa gactttagcc ttgagatgat g 51
<210> 112
   <211> 53
   <212> DNA
   <213> Artificial Sequence
<220>
   <221> source
   <223> /note="Description of Artificial Sequence: Synthetic oligonucleotide"
<400> 112
   gccttctatc gccttcttga cgagttcttc tgattcctca ccgatatttc gtg 53
<210> 113
   <211> 56
   <212> DNA
   <213> Artificial Sequence
<220>
   <221> source
   <223> /note="Description of Artificial Sequence: Synthetic oligonucleotide"
<400> 113
   cagattacgc gcagaaaaaa aggatctcaa gctaaattgt aagcgttaat attttg 56
<210> 114
   <211> 20
   <212> DNA
   <213> Artificial Sequence
<220>
   <221> source
   <223> /note="Description of Artificial Sequence: Synthetic oligonucleotide"
<400> 114
   caggctaaaa cgcgcacctg 20
<210> 115
   <211> 20
   <212> DNA
   <213> Artificial Sequence
<220>
   <221> source
   <223> /note="Description of Artificial Sequence: Synthetic oligonucleotide"
<400> 115
   caggtgcgcg ttttagcctg 20
<210> 116
   <211> 20
   <212> DNA
   <213> Artificial Sequence
<220>
   <221> source
   <223> /note="Description of Artificial Sequence: Synthetic oligonucleotide"
<400> 116
   cagaccgtca ccacgatccg 20
<210> 117
   <211> 20
   <212> DNA
   <213> Artificial Sequence
<220>
   <221> source
   <223> /note="Description of Artificial Sequence: Synthetic oligonucleotide"
<400> 117
   cggatcgtgg tgacggtctg 20
<210> 118
   <211> 21
   <212> DNA
   <213> Artificial Sequence
<220>
   <221> source
   <223> /note="Description of Artificial Sequence: Synthetic oligonucleotide"
<400> 118
   ctcgatacga tgcgggatat c 21
<210> 119
   <211> 21
   <212> DNA
   <213> Artificial Sequence
<220>
   <221> source
   <223> /note="Description of Artificial Sequence: Synthetic oligonucleotide"
<400> 119
   gatatcccgc atcgtatcga g 21
<210> 120
   <211> 20
   <212> DNA
   <213> Artificial Sequence
<220>
   <221> source
   <223> /note="Description of Artificial Sequence: Synthetic oligonucleotide"
<400> 120
   cacggttggt cagctcattc 20
<210> 121
   <211> 20
   <212> DNA
   <213> Artificial Sequence
<220>
   <221> source
   <223> /note="Description of Artificial Sequence: Synthetic oligonucleotide"
<400> 121
   gaatgagctg accaaccgtg 20
<210> 122
   <211> 19
   <212> DNA
   <213> Artificial Sequence
<220>
   <221> source
   <223> /note="Description of Artificial Sequence: Synthetic oligonucleotide"
<400> 122
   cagcggacgg aaatcctcc 19
<210> 123
   <211> 19
   <212> DNA
   <213> Artificial Sequence
<220>
   <221> source
   <223> /note="Description of Artificial Sequence: Synthetic oligonucleotide"
<400> 123
   ggaggatttc cgtccgctg 19
<210> 124
   <211> 20
   <212> DNA
   <213> Artificial Sequence
<220>
   <221> source
   <223> /note="Description of Artificial Sequence: Synthetic oligonucleotide"
<400> 124
   ttacctcctt aaagatcttc 20
<210> 125
   <211> 20
   <212> DNA
   <213> Artificial Sequence
<220>
   <221> source
   <223> /note="Description of Artificial Sequence: Synthetic oligonucleotide"
<400> 125
   gaagatcttt aaggaggtaa 20
<210> 126
   <211> 19
   <212> DNA
   <213> Artificial Sequence
<220>
   <221> source
   <223> /note="Description of Artificial Sequence: Synthetic oligonucleotide"
<400> 126
   cgacggtttc gaaccaaac 19
<210> 127
   <211> 19
   <212> DNA
   <213> Artificial Sequence
<220>
   <221> source
   <223> /note="Description of Artificial Sequence: Synthetic oligonucleotide"
<400> 127
   gtttggttcg aaaccgtcg 19
<210> 128
   <211> 54
   <212> DNA
   <213> Artificial Sequence
<220>
   <221> source
   <223> /note="Description of Artificial Sequence: Synthetic oligonucleotide"
<400> 128
   gccttctatc gccttcttga cgagttcttc tgattagcgc ttggccgcga aaac 54
<210> 129
   <211> 24
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-A base
<400> 129
   tcagaagaac tcgtcaagaa ggcg 24
<210> 130
   <211> 24
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-T base
<220>
   <221> modified_base
   <222> (17)..(17)
   <223> Phosphorothioate-T base
<400> 130
   cttgagatcc tttttttctg cgcg 24
<210> 131
   <211> 24
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-A base
<400> 131
   tcaagaaggc gatagaaggc gatg 24
<210> 132
   <211> 28
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-T base
<400> 132
   ttttttctgc gcgtaatctg ctgcttgc 28
<210> 133
   <211> 40
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-G base
<400> 133
   tacgcgcaga aaaaaaggat ctcaagactt tagccttgag 40
<210> 134
   <211> 29
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-C base
<220>
   <221> modified_base
   <222> (17)..(17)
   <223> Phosphorothioate-C base
<400> 134
   tcgggcaccg aactccccga agcgctaac 29
<210> 135
   <211> 28
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-G base
<220>
   <221> modified_base
   <222> (17)..(17)
   <223> Phosphorothioate-G base
<400> 135
   gagttcggtg cccgaggcgc tgcttgag 28
<210> 136
   <211> 29
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-T base
<220>
   <221> modified_base
   <222> (17)..(17)
   <223> Phosphorothioate-T base
<400> 136
   cggctgcact tgcacttggt taatcccag 29
<210> 137
   <211> 34
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-A base
<400> 137
   gtgcaagtgc agccgaaaat cggctttggc tttg 34
<210> 138
   <211> 30
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-A base
<400> 138
   tgcgcatcgc cttggaaagt ggcgtgcccg 30
<210> 139
   <211> 33
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-C base
<220>
   <221> modified_base
   <222> (17)..(17)
   <223> Phosphorothioate-C base
<400> 139
   ccaaggcgat gcgcaccgcc agcgcccatt ttg 33
<210> 140
   <211> 29
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-G base
<400> 140
   tagatcacca cattgagaaa gctaccgac 29
<210> 141
   <211> 34
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-T base
<220>
   <221> modified_base
   <222> (17)..(17)
   <223> Phosphorothioate-C base
<400> 141
   caatgtggtg atctatcgct tacccaaaat catg 34
<210> 142
   <211> 33
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-C base
<220>
   <221> modified_base
   <222> (17)..(17)
   <223> Phosphorothioate-A base
<400> 142
   ccgattggta tcacgcacgt cacgtgcgat cac 33
<210> 143
   <211> 30
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-G base
<220>
   <221> modified_base
   <222> (17)..(17)
   <223> Phosphorothioate-T base
<400> 143
   cgtgatacca atcgggtcaa aaccgtagtg 30
<210> 144
   <211> 30
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-C base
<400> 144
   tggacgtcga tacgcacggc tagcgagttg 30
<210> 145
   <211> 37
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-G base
<220>
   <221> modified_base
   <222> (17)..(17)
   <223> Phosphorothioate-A base
<400> 145
   gcgtatcgac gtccagactg gatgttggtg gatattg 37
<210> 146
   <211> 31
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-G base
<220>
   <221> modified_base
   <222> (17)..(17)
   <223> Phosphorothioate-A base
<400> 146
   tggggttaaa aattggaagg agactttaaa g 31
<210> 147
   <211> 33
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-G base
<220>
   <221> modified_base
   <222> (17)..(17)
   <223> Phosphorothioate-C base
<400> 147
   caatttttaa ccccagcctc taacccgcaa gag 33
<210> 148
   <211> 35
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-C base
<400> 148
   gccttaacct tgaccacact ccctgccggc gtttg 35
<210> 149
   <211> 34
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-G base
<220>
   <221> modified_base
   <222> (17)..(17)
   <223> Phosphorothioate-G base
<400> 149
   ggtcaaggtt aaggcgggtc aatatgtcgg aatc 34
<210> 150
   <211> 36
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-C base
<400> 150
   tcagacaaca tgttcacgtt gccttcaccg ccgatc 36
<210> 151
   <211> 34
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-A base
<220>
   <221> modified_base
   <222> (17)..(17)
   <223> Phosphorothioate-C base
<400> 151
   gaacatgttg tctgaacgag gttcgatcag catc 34
<210> 152
   <211> 31
   <212> DNA
   <213> Artificial Sequence
<220>
   <221> source
   <223> /note="Description of Artificial Sequence: Synthetic oligonucleotide"
<220>
   <221> modified_base
   <222> (16)..(16)
   <223> Phosphorothioate-C base
<220>
   <221> modified_base
   <222> (17)..(17)
   <223> Phosphorothioate-G base
<400> 152
   ctatcgcctt cttgacgagt tcttctgatt c 31
<210> 153
   <211> 2848
   <212> DNA
   <213> Artificial Sequence
<220>
   <221> source
   <223> /note="Description of Artificial Sequence: Synthetic polynucleotide"
<400> 153
   aaaaaatgta gaggtcgagt ttagatgcaa gttcaaggag cgaaaggtgg atgggtaggt       60
   tatataggga tatagcacag agatatatag caaagagata cttttgagca atgtttgtgg      120
   aagcggtatt cgcaatggga agctccaccc cggttgataa tcagaaagct tcaaccaaag      180
   cttcagggga taacgcagga aagaacatgt gagcaaaagg ccagcaaaag cccaggaacc 240
   gtaaaaaggc cgcgttgctg gcgtttttcc ataggctccg cccccctgac gagcatcaca 300
   aaaatcgacg ctcaagtcag aggtggcgaa acccgacagg actataaaga taccaggcgt 360
   ttccccctgg aagctccctc gtgcgctctc ctgttccgac cctgccgctt accggatacc 420
   tgtccgcctt tctcccttcg ggaagcgtgg cgctttctca tagctcacgc tgtaggtatc 480
   tcagttcggt gtaggtcgtt cgctccaagc tgggctgtgt gcacgaaccc cccgttcagc 540
   ccgaccgctg cgccttatcc ggtaactatc gtcttgagtc caacccggta agacacgact 600
   tatcgccact ggcagcagcc actggtaaca ggattagcag agcgaggtat gtaggcggtg 660
   ctacagagtt cttgaagtgg tggcctaact acggctacac tagaaggaca gtatttggta 720
   tctgcgctct gctgaagcca gttaccttcg gaaaaagagt tggtagctct tgatccggca 780
   aacaaaccac cgctggtagc ggtggttttt ttgtttgcaa gcagcagatt acgcgcagaa 840
   aaaaaggatc tcaagaagat cctttgatct tttctacggg gtctgacgct cagtggaacg 900
   aaaactcacg ttaagggatt ttggtcatga gattatcaaa aaggatcttc acctagatcc 960
   ttttaaatta aaaatgaagt tttaaatcaa tctaaagtat atatgagtaa acttggtctg 1020
   acagttacca atgcttaatc agtgaggcac ctatctcagc gatctgtcta tttcgttcat 1080
   ccatagttgc ctgactcccc gtcgtgtaga taactacgat acgggagcgc ttaccatctg 1140
   gccccagtgc tgcaatgata ccgcgagacc cacgctcacc ggctccagat ttatcagcaa 1200
   taaaccagcc agccggaagg gccgagcgca gaagtggtcc tgcaacttta tccgcctcca 1260
   tccagtctat taattgttgc cgggaagcta gagtaagtag ttcgccagtt aatagtttgc 1320
   gcaacgttgt tgccattgct acaggcatcg tggtgtcacg ctcgtcgttt ggtatggctt 1380
   cattcagctc cggttcccaa cgatcaaggc gagttacatg atcccccatg ttgtgcaaaa 1440
   aagcggttag ctccttcggt cctccgatcg ttgtcagaag taagttggcc gcagtgttat 1500
   cactcatggt tatggcagca ctgcataatt ctcttactgt catgccatcc gtaagatgct 1560
   tttctgtgac tggtgagtac tcaaccaagt cattctgaga atagtgtatg cggcgaccga 1620
   gttgctcttg cccggcgtca acacgggata ataccgcgcc acatagcaga actttaaaag 1680
   tgctcatcat tggaaaacgt tcttcggggc gaaaactctc aaggatctta ccgctgttga 1740
   gatccagttc gatgtaaccc actcgtgcac ccaactgatc ttcagcatct tttactttca 1800
   ccagcgtttc tgggtgagca aaaacaggaa ggcaaaatgc cgcaaaaaag ggaataaggg 1860
   cgacacggaa atgttgaata ctcatactct tcctttttca atattattga agcatttatc 1920
   agggttattg tctcatgagc ggatacatat ttgaatgtat ttagaaaaat aaacaaatag 1980
   gggttccgcg cacatttccc cgaaaagtgc cacctgacgt ctaagaaacc attattatca 2040
   tgacattaac ctataaaaat aggcgtatca cgaggccctt tcgtcttcaa gaaattcggt 2100
   cgaaaaaaga aaaggagagg gccaagaggg agggcattgg tgactattga gcacgtgagt 2160
   atacgtgatt aagcacacaa aggcagcttg gagtatgtct gttattaatt tcacaggtag 2220
   ttctggtcca ttggtgaaag tttgcggctt gcagagcaca gaggccgcag aatgtgctct 2280
   agattccgat gctgacttgc tgggtattat atgtgtgccc aatagaaaga gaacaattga 2340
   cccggttatt gcaaggaaaa tttcaagtct tgtaaaagca tataaaaata gttcaggcac 2400
   tccgaaatac ttggttggcg tgtttcgtaa tcaacctaag gaggatgttt tggctctggt 2460
   caatgattac ggcattgata tcgtccaact gcacggagat gagtcgtggc aagaatacca 2520
   agagttcctc ggtttgccag ttattaaaag actcgtattt ccaaaagact gcaacatact 2580
   actcagtgca gcttcacaga aacctcattc gtttattccc ttgtttgatt cagaagcagg 2640
   tgggacaggt gaacttttgg attggaactc gatttctgac tgggttggaa ggcaagagag 2700
   ccccgagagc ttacatttta tgttagctgg tggactgacg ccagaaaatg ttggtgatgc 2760
   gcttagatta aatggcgtta ttggtgttga tgtaagcgga ggtgtggaga caaatggtgt 2820
   aaaagactct aacaaaatag caaatttc 2848
<210> 154
   <211> 4255
   <212> DNA
   <213> Artificial Sequence
<220>
   <221> source
   <223> /note="Description of Artificial Sequence: Synthetic polynucleotide"
<400> 154
   tatttaagta ttgtttgtgc acttgcccta gcttatcgat gataagctgt caaagatgag 60
   aattaattcc acggactata gactatacta gatactccgt ctactgtacg atacacttcc 120
   gctcaggtcc ttgtccttta acgaggcctt accactcttt tgttactcta ttgatccagc 180
   tcagcaaagg cagtgtgatc taagattcta tcttcgcgat gtagtaaaac tagctagacc 240
   gagaaagaga ctagaaatgc aaaaggcact tctacaatgg ctgccatcat tattatccga 300
   tgtgacgctg cagcttctca atgatattcg aatacgcttt gaggagatac agcctaatat 360
   ccgacaaact gttttacaga tttacgatcg tacttgttac ccatcattga attttgaaca 420
   tccgaacctg ggagttttcc ctgaaacaga tagtatattt gaacctgtat aataatatat 480
   agtctagcgc tttacggaag acaatgtatg tatttcggtt cctggagaaa ctattgcatc 540
   tattgcatag gtaatcttgc acgtcgcatc cccggttcat tttctgcgtt tccatcttgc 600
   acttcaatag catatctttg ttaacgaagc atctgtgctt cattttgtag aacaaaaatg 660
   caacgcgaga gcgctaattt ttcaaacaaa gaatctgagc tgcattttta cagaacagaa 720
   atgcaacgcg aaagcgctat tttaccaacg aagaatctgt gcttcatttt tgtaaaacaa      780
   aaatgcaacg cgacgagagc gctaattttt caaacaaaga atctgagctg catttttaca      840
   gaacagaaat gcaacgcgag agcgctattt taccaacaaa gaatctatac ttcttttttg      900
   ttctacaaaa atgcatcccg agagcgctat ttttctaaca aagcatctta gattactttt      960
   tttctccttt gtgcgctcta taatgcagtc tcttgataac tttttgcact gtaggtccgt     1020
   taaggttaga agaaggctac tttggtgtct attttctctt ccataaaaaa agcctgactc     1080
   cacttcccgc gtttactgat tactagcgaa gctgcgggtg cattttttca agataaaggc     1140
   atccccgatt atattctata ccgatgtgga ttgcgcatac tttgtgaaca gaaagtgata     1200
   gcgttgatga ttcttcattg gtcagaaaat tatgaacggt ttcttctatt ttgtctctat     1260
   atactacgta taggaaatgt ttacattttc gtattgtttt cgattcactc tatgaatagt     1320
   tcttactaca atttttttgt ctaaagagta atactagaga taaacataaa aaatgtagag     1380
   gtcgagttta gatgcaagtt caaggagcga aggtggatgg ggtaggttat atagggatat     1440
   agcacagaga tatatagcaa agagatactt ttgagcaatg tttgtggaag cggtattcgc     1500
   aatgggaagc tccaccccgg ttgataatca gaaagcttca accaaagctt caggggataa     1560
   cgcaggaaag aacatgtgag caaaaggcca gcaaaagccc aggaaccgta aaaaggccgc     1620
   gttgctggcg tttttccata ggctccgccc ccctgacgag catcacaaaa atcgacgctc     1680
   aagtcagagg tggcgaaacc cgacaggact ataaagatac caggcgtttc cccctggaag     1740
   ctccctcgtg cgctctcctg ttccgaccct gccgcttacc ggatacctgt ccgcctttct     1800
   cccttcggga agcgtggcgc tttctcatag ctcacgctgt aggtatctca gttcggtgta     1860
   ggtcgttcgc tccaagctgg gctgtgtgca cgaacccccc gttcagcccg accgctgcgc     1920
   cttatccggt aactatcgtc ttgagtccaa cccggtaaga cacgacttat cgccactggc     1980
   agcagccact ggtaacagga ttagcagagc gaggtatgta ggcggtgcta cagagttctt     2040
   gaagtggtgg cctaactacg gctacactag aaggacagta tttggtatct gcgctctgct     2100
   gaagccagtt accttcggaa aaagagttgg tagctcttga tccggcaaac aaaccaccgc     2160
   tggtagcggt ggtttttttg tttgcaagca gcagattacg cgcagaaaaa aaggatctca     2220
   agaagatcct ttgatctttt ctacggggtc tgacgctcag tggaacgaaa actcacgtta     2280
   agggattttg gtcatgagat tatcaaaaag gatcttcacc tagatccttt taaattaaaa     2340
   atgaagtttt aaatcaatct aaagtatata tgagtaaact tggtctgaca gttaccaatg     2400
   cttaatcagt gaggcaccta tctcagcgat ctgtctattt cgttcatcca tagttgcctg     2460
   actccccgtc gtgtagataa ctacgatacg ggagcgctta ccatctggcc ccagtgctgc     2520
   aatgataccg cgagacccac gctcaccggc tccagattta tcagcaataa accagccagc     2580
   cggaagggcc gagcgcagaa gtggtcctgc aactttatcc gcctccatcc agtctattaa     2640
   ttgttgccgg gaagctagag taagtagttc gccagttaat agtttgcgca acgttgttgc 2700
   cattgctaca ggcatcgtgg tgtcacgctc gtcgtttggt atggcttcat tcagctccgg 2760
   ttcccaacga tcaaggcgag ttacatgatc ccccatgttg tgcaaaaaag cggttagctc 2820
   cttcggtcct ccgatcgttg tcagaagtaa gttggccgca gtgttatcac tcatggttat 2880
   ggcagcactg cataattctc ttactgtcat gccatccgta agatgctttt ctgtgactgg 2940
   tgagtactca accaagtcat tctgagaata gtgtatgcgg cgaccgagtt gctcttgccc 3000
   ggcgtcaaca cgggataata ccgcgccaca tagcagaact ttaaaagtgc tcatcattgg 3060
   aaaacgttct tcggggcgaa aactctcaag gatcttaccg ctgttgagat ccagttcgat 3120
   gtaacccact cgtgcaccca actgatcttc agcatctttt actttcacca gcgtttctgg 3180
   gtgagcaaaa acaggaaggc aaaatgccgc aaaaaaggga ataagggcga cacggaaatg 3240
   ttgaatactc atactcttcc tttttcaata ttattgaagc atttatcagg gttattgtct 3300
   catgagcgga tacatatttg aatgtattta gaaaaataaa caaatagggg ttccgcgcac 3360
   atttccccga aaagtgccac ctgacgtcta agaaaccatt attatcatga cattaaccta 3420
   taaaaatagg cgtatcacga ggccctttcg tcttcaagaa attcggtcga aaaaagaaaa 3480
   ggagagggcc aagagggagg gcattggtga ctattgagca cgtgagtata cgtgattaag 3540
   cacacaaagg cagcttggag tatgtctgtt attaatttca caggtagttc tggtccattg 3600
   gtgaaagttt gcggcttgca gagcacagag gccgcagaat gtgctctaga ttccgatgct 3660
   gacttgctgg gtattatatg tgtgcccaat agaaagagaa caattgaccc ggttattgca 3720
   aggaaaattt caagtcttgt aaaagcatat aaaaatagtt caggcactcc gaaatacttg 3780
   gttggcgtgt ttcgtaatca acctaaggag gatgttttgg ctctggtcaa tgattacggc 3840
   attgatatcg tccaactgca cggagatgag tcgtggcaag aataccaaga gttcctcggt 3900
   ttgccagtta ttaaaagact cgtatttcca aaagactgca acatactact cagtgcagct 3960
   tcacagaaac ctcattcgtt tattcccttg tttgattcag aagcaggtgg gacaggtgaa 4020
   cttttggatt ggaactcgat ttctgactgg gttggaaggc aagagagccc cgagagctta 4080
   cattttatgt tagctggtgg actgacgcca gaaaatgttg gtgatgcgct tagattaaat 4140
   ggcgttattg gtgttgatgt aagcggaggt gtggagacaa atggtgtaaa agactctaac 4200
   aaaatagcaa atttcgtcaa aaatgctaag aaataggtta ttactgagta gtatt 4255
<210> 155
   <211> 2764
   <212> DNA
   <213> Artificial Sequence
<220>
   <221> source
   <223> /note="Description of Artificial Sequence: Synthetic polynucleotide"
<400> 155
   atgtgagcaa aaggccagca aaagcccagg aaccgtaaaa aggccgcgtt gctggcgttt 60
   ttccataggc tccgcccccc tgacgagcat cacaaaaatc gacgctcaag tcagaggtgg 120
   cgaaacccga caggactata aagataccag gcgtttcccc ctggaagctc cctcgtgcgc 180
   tctcctgttc cgaccctgcc gcttaccgga tacctgtccg cctttctccc ttcgggaagc 240
   gtggcgcttt ctcatagctc acgctgtagg tatctcagtt cggtgtaggt cgttcgctcc 300
   aagctgggct gtgtgcacga accccccgtt cagcccgacc gctgcgcctt atccggtaac 360
   tatcgtcttg agtccaaccc ggtaagacac gacttatcgc cactggcagc agccactggt 420
   aacaggatta gcagagcgag gtatgtaggc ggtgctacag agttcttgaa gtggtggcct 480
   aactacggct acactagaag gacagtattt ggtatctgcg ctctgctgaa gccagttacc 540
   ttcggaaaaa gagttggtag ctcttgatcc ggcaaacaaa ccaccgctgg tagcggtggt 600
   tttttgtttt gcaagcagca gattacgcgc agaaaaaaca agaattctta ctacgcacca 660
   ccctgccact cgtcgcaata ctgttgcagt tcattcagca tacgaccaac gtggaaacca 720
   tcgcaaaccg cgtggtgaac ctggatcgcc agcggcatca gaactttgtc accctgggtg 780
   tagtatttac ccatggtgaa gaccggtgcg aaaaagttgt ccatgttcgc tacattcagg 840
   tcgaagctag tgaaggatac ccaagggttc gcagatacga agaacatatt ttcgatgaag 900
   ccttttggga aatacgcgag attctcaccg taacacgcaa cgtcctgaga gtagatgtgc 960
   aggaactgac ggaagtcgtc gtggtattcg ctccacaggc tagagaaggt ttcggtctgt 1020
   tcgtgaaaaa cggtgtagca cgggtgaaca gagtcccaga taaccagttc gccgtctttc 1080
   atcgccatac gaaattccgg atgggcgttc atgagacgcg ccaggatgtg gatgaaggcc 1140
   gggtagaatt tgtgcttgtt tttcttgaca gtcttgagga atgcggtgat gtcgagctga 1200
   acagtttggt tgtaggtgca ctgcgcaacg gactggaacg cttcaaagtg ctctttacga 1260
   tgccactgag agatgtcaac ggtcgtgtag ccggtgatct ttttttccat tttagcttcc 1320
   ttagctcctg aaaatctcga taactcaaaa aatacgccct catactagat atctagatcc 1380
   ggcccgatgc gtccggcgta gaggatctga agatcagcag ttcaacctgt tgatagtacg 1440
   tactaagctc tcatgtttca cgtactaagc tctcatgttt aacgtactaa gctctcatgt 1500
   ttaacgaact aaaccctcat ggctaacgta ctaagctctc atggctaacg tactaagctc 1560
   tcatgtttca cgtactaagc tctcatgttt gaacaataaa attaatataa atcagcaact 1620
   taaatagcct ctaaggtttt aagttttata agaaaaaaaa gaatatataa ggcttttaaa 1680
   gcttttaagg tttaacggtt gtggacaaca agccagggat gtaacgcact gagaagccct 1740
   tagagcctct caaagcaatt ttcagtgaca caggaacact taacggctga catgggaatt 1800
   ctactgtttg ggtgtatgag ctcaaagaag gcgatagaag gcgatgcgct gcgaatcggg 1860
   agcggcgata ccgtaaagca cgaggaagcg gtcagcccat tcgccgccaa gctcttcagc 1920
   aatatcacgg gtagccaacg ctatgtcctg atagcggtcc gccacaccca gccggccaca 1980
   gtcgatgaat ccagaaaagc ggccattttc caccatgata ttcggcaagc aggcatcgcc 2040
   atgggtcacg acgagatcct cgccgtcggg catgctcgcc ttgagcctgg cgaacagttc 2100
   ggctggcgcg agcccctgat gctcttcgtc cagatcatcc tgatcgacaa gaccggcttc 2160
   catccgagta cgtgctcgct cgatgcgatg tttcgcttgg tggtcgaatg ggcaggtagc 2220
   cggatcaagc gtatgcagcc gccgcattgc atcagccatg atggatactt tctcggcagg 2280
   agcaaggtga gatgacagga gatcctgccc cggcacttcg cccaatagca gccagtccct 2340
   tcccgcttca gtgacaacgt cgagcacagc tgcgcaagga acgcccgtcg tggccagcca 2400
   cgatagccgc gctgcctcgt cttgcagttc attcagggca ccggacaggt cggtcttgac 2460
   aaaaagaacc gggcgcccct gcgctgacag ccggaacacg gcggcatcag agcagccgat 2520
   tgtctgttgt gcccagtcat agccgaatag cctctccacc caagcggccg gagaacctgc 2580
   gtgcaatcca tcttgttcaa tcatgcgaaa cgatcctcat cctgtctctt gatcagagct 2640
   tgatcccctg cgccatcaga tccttggcgg cgagaaagcc atccagttta ctttgcaggg 2700
   cttcccaacc ttaccagagg gcgccccagc tggcaattcc ggttgaaaag tgccacctga 2760
   cgtc 2764
<210> 156
   <211> 1604
   <212> DNA
   <213> Artificial Sequence
<220>
   <221> source
   <223> /note="Description of Artificial Sequence: Synthetic polynucleotide"
<400> 156
   tcagaagaac tcgtcaagaa ggcgatagaa ggcgatgcgc tgcgaatcgg gagcggcgat 60
   accgtaaagc acgaggaagc ggtcagccca ttcgccgcca agctcttcag caatatcacg 120
   ggtagccaac gctatgtcct gatagcggtc cgccacaccc agccggccac agtcgatgaa 180
   tccagaaaag cggccatttt ccaccatgat attcggcaag caggcatcgc catgggtcac 240
   gacgagatcc tcgccgtcgg gcatgctcgc cttgagcctg gcgaacagtt cggctggcgc 300
   gagcccctga tgctcttcgt ccagatcatc ctgatcgaca agaccggctt ccatccgagt 360
   acgtgctcgc tcgatgcgat gtttcgcttg gtggtcgaat gggcaggtag ccggatcaag 420
   cgtatgcagc cgccgcattg catcagccat gatggatact ttctcggcag gagcaaggtg 480
   agatgacagg agatcctgcc ccggcacttc gcccaatagc agccagtccc ttcccgcttc 540
   agtgacaacg tcgagcacag ctgcgcaagg aacgcccgtc gtggccagcc acgatagccg 600
   cgctgcctcg tcttgcagtt cattcagggc accggacagg tcggtcttga caaaaagaac 660
   cgggcgcccc tgcgctgaca gccggaacac ggcggcatca gagcagccga ttgtctgttg 720
   tgcccagtca tagccgaata gcctctccac ccaagcggcc ggagaacctg cgtgcaatcc 780
   atcttgttca atcatgcgaa acgatcctca tcctgtctct tgatcagagc ttgatcccct 840
   gcgccatcag atccttggcg gcgagaaagc catccagttt actttgcagg gcttcccaac 900
   cttaccagag ggcgccccag ctggcaattc cggttgaaaa gtgccacctg acgtcatgtg 960
   agcaaaaggc cagcaaaagc ccaggaaccg taaaaaggcc gcgttgctgg cgtttttcca 1020
   taggctccgc ccccctgacg agcatcacaa aaatcgacgc tcaagtcaga ggtggcgaaa 1080
   cccgacagga ctataaagat accaggcgtt tccccctgga agctccctcg tgcgctctcc 1140
   tgttccgacc ctgccgctta ccggatacct gtccgccttt ctcccttcgg gaagcgtggc 1200
   gctttctcat agctcacgct gtaggtatct cagttcggtg taggtcgttc gctccaagct 1260
   gggctgtgtg cacgaacccc ccgttcagcc cgaccgctgc gccttatccg gtaactatcg 1320
   tcttgagtcc aacccggtaa gacacgactt atcgccactg gcagcagcca ctggtaacag 1380
   gattagcaga gcgaggtatg taggcggtgc tacagagttc ttgaagtggt ggcctaacta 1440
   cggctacact agaaggacag tatttggtat ctgcgctctg ctgaagccag ttaccttcgg 1500
   aaaaagagtt ggtagctctt gatccggcaa acaaaccacc gctggtagcg gtggtttttt 1560
   tgtttgcaag cagcagatta cgcgcagaaa aaaaggatct caag 1604
<210> 157
   <211> 742
   <212> DNA
   <213> Artificial Sequence
<220>
   <221> source
   <223> /note="Description of Artificial Sequence: Synthetic polynucleotide"
<400> 157
   gttaggcgtt ttgcgctgct tcgcgatgta cgggccagat atacgcgttg acattgatta      60
   ttgactagtt attaatagta atcaattacg gggtcattag ttcatagccc atatatggag     120
   ttccgcgtta cataacttac ggtaaatggc ccgcctggct gaccgcccaa cgacccccgc     180
   ccattgacgt caataatgac gtatgttccc atagtaacgc caatagggac tttccattga     240
   cgtcaatggg tggagtattt acggtaaact gcccacttgg cagtacatca agtgtatcat     300
   atgccaagta cgccccctat tgacgtcaat gacggtaaat ggcccgcctg gcattatgcc     360
   cagtacatga ccttatggga ctttcctact tggcagtaca tctacgtatt agtcatcgct     420
   attaccatgg tgatgcggtt ttggcagtac atcaatgggc gtggatagcg gtttgactca     480
   cggggatttc caagtctcca ccccattgac gtcaatggga gtttgttttg gcaccaaaat     540
   caacgggact ttccaaaatg tcgtaacaac tccgccccat tgacgcaaat gggcggtagg     600
   cgtgtacggt gggaggtcta tataagcaga gctcgtttag tgaaccgtca gatcgcctgg     660
   agacgccatc cacgctgttt tgacctccat agaagacacc gggaccgatc cagcctccgg     720
   actctagagg atcgaatggc aa                                              742
<210> 158
   <211> 897
   <212> DNA
   <213> Artificial Sequence
<220>
   <221> source
   <223> /note="Description of Artificial Sequence: Synthetic polynucleotide"
<400> 158
   tgcagatgca gcttgaagca aatgcagata cttcagtgga agaagaaagc tttggcccac      60
   aacccatttc acggttagag cagtgtggca taaatgccaa cgatgtgaag aaattggaag     120
   aagctggatt ccatactgtg gaggctgttg cctatgcgcc aaagaaggag ctaataaata     180
   ttaagggaat tagtgaagcc aaagctgata aaattctggc tgaggcagct aaattagttc     240
   caatgggttt caccactgca actgaattcc accaaaggcg gtcagagatc atacagatta     300
   ctactggctc caaagagctt gacaaactac ttcaaggtgg aattgagact ggatctatca     360
   cagaaatgtt tggagaattc cgaactggga agacccagat ctgtcatacg ctagctgtca     420
   cctgccagct tcccattgac cggggtggag gtgaaggaaa ggccatgtac attgacactg     480
   agggtacctt taggccagaa cggctgctgg cagtggctga gaggtatggt ctctctggca     540
   gtgatgtcct ggataatgta gcatatgctc gagcgttcaa cacagaccac cagacccagc     600
   tcctttatca agcatcagcc atgatggtag aatctaggta tgcactgctt attgtagaca     660
   gtgccaccgc cctttacaga acagactact cgggtcgagg tgagctttca gccaggcaga     720
   tgcacttggc caggtttctg cggatgcttc tgcgactcgc tgatgagttt ggtgtagcag     780
   tggtaatcac taatcaggtg gtagctcaag tggatggagc agcgatgttt gctgctgatc     840
   ccaaaaaacc tattggagga aatatcatcg cccatgcatc aacaaccaga ttgtatc        897
<210> 159
   <211> 908
   <212> DNA
   <213> Artificial Sequence
<220>
   <221> source
   <223> /note="Description of Artificial Sequence: Synthetic polynucleotide"
<400> 159
   tgaggaaagg aagaggggaa accagaatct gcaaaatcta cgactctccc tgtcttcctg 60
   aagctgaagc tatgttcgcc attaatgcag atggagtggg agatgccaaa gacggaagcg 120
   gagctactaa cttcagcctg ctgaagcagg ctggagacgt ggaggagaac cctggaccta 180
   tggctgctgt tcctcaaaat aatctacagg agcaactaga acgtcactca gccagaacac 240
   ttaataataa attaagtctt tcaaaaccaa aattttcagg tttcactttt aaaaagaaaa 300
   catcttcaga taacaatgta tctgtaacta atgtgtcagt agcaaaaaca cctgtattaa 360
   gaaataaaga tgttaatgtt accgaagact tttccttcag tgaacctcta cccaacacca 420
   caaatcagca aagggtcaag gacttcttta aaaatgctcc agcaggacag gaaacacaga 480
   gaggtggatc aaaatcatta ttgccagatt tcttgcagac tccgaaggaa gttgtatgca 540
   ctacccaaaa cacaccaact gtaaagaaat cccgggatac tgctctcaag aaattagaat 600
   ttagttcttc accagattct ttaagtacca tcaatgattg ggatgatatg gatgactttg 660
   atacttctga gacttcaaaa tcatttgtta caccacccca aagtcacttt gtaagagtaa 720
   gcactgctca gaaatcaaaa aagggtaaga gaaacttttt taaagcacag ctttatacaa 780
   caaacacagt aaagactgat ttgcctccac cctcctctga aagcgagcaa atagatttga 840
   ctgaggaaca gaaggatgac tcagaatggt taagcagcga tgtgatttgc atcgatgatg 900
   gccccatt 908
<210> 160
   <211> 3520
   <212> DNA
   <213> Artificial Sequence
<220>
   <221> source
   <223> /note="Description of Artificial Sequence: Synthetic polynucleotide"
<400> 160
   gctgaagtgc atataaatga agatgctcag gaaagtgact ctctgaaaac tcatttggaa 60
   gatgaaagag ataatagcga aaagaagaag aatttggaag aagctgaatt acattcaact 120
   gagaaagttc catgtattga atttgatgat gatgattatg atacggattt tgttccacct 180
   tctccagaag aaattatttc tgcttcttct tcctcttcaa aatgccttag tacgttaaag 240
   gaccttgaca catctgacag aaaagaggat gttcttagca catcaaaaga tcttttgtca 300
   aaacctgaga aaatgagtat gcaggagctg aatccagaaa ccagcacaga ctgtgacgct 360
   agacagataa gtttacagca gcagcttatt catgtgatgg agcacatctg taaattaatt 420
   gatactattc ctgatgataa actgaaactt ttggattgtg ggaacgaact gcttcagcag 480
   cggaacataa gaaggaaact tctaacggaa gtagatttta ataaaagtga tgccagtctt      540
   cttggctcat tgtggagata caggcctgat tcacttgatg gccctatgga gggtgattcc      600
   tgccctacag ggaattctat gaaggagtta aatttttcac accttccctc aaattctgtt      660
   tctcctgggg actgtttact gactaccacc ctaggaaaga caggattctc tgccaccagg      720
   aagaatcttt ttgaaaggcc tttattcaat acccatttac agaagtcctt tgtaagtagc      780
   aactgggctg aaacaccaag actaggaaaa aaaaatgaaa gctcttattt cccaggaaat      840
   gttctcacaa gcactgctgt gaaagatcag aataaacata ctgcttcaat aaatgactta      900
   gaaagagaaa cccaaccttc ctatgatatt gataattttg acatagatga ctttgatgat      960
   gatgatgact gggaagacat aatgcataat ttagcagcca gcaaatcttc cacagctgcc     1020
   tatcaaccca tcaaggaagg tcggccaatt aaatcagtat cagaaagact ttcctcagcc     1080
   aagacagact gtcttccagt gtcatctact gctcaaaata taaacttctc agagtcaatt     1140
   cagaattata ctgacaagtc agcacaaaat ttagcatcca gaaatctgaa acatgagcgt     1200
   ttccaaagtc ttagttttcc tcatacaaag gaaatgatga agatttttca taaaaaattt     1260
   ggcctgcata attttagaac taatcagcta gaggcgatca atgctgcact gcttggtgaa     1320
   gactgtttta tcctgatgcc gactggaggt ggtaagagtt tgtgttacca gctccctgcc     1380
   tgtgtttctc ctggggtcac tgttgtcatt tctcccttga gatcacttat cgtagatcaa     1440
   gtccaaaagc tgacttcctt ggatattcca gctacatatc tgacaggtga taagactgac     1500
   tcagaagcta caaatattta cctccagtta tcaaaaaaag acccaatcat aaaacttcta     1560
   tatgtcactc cagaaaagat ctgtgcaagt aacagactca tttctactct ggagaatctc     1620
   tatgagagga agctcttggc acgttttgtt attgatgaag cacattgtgt cagtcagtgg     1680
   ggacatgatt ttcgtcaaga ttacaaaaga atgaatatgc ttcgccagaa gtttccttct     1740
   gttccggtga tggctcttac ggccacagct aatcccaggg tacagaagga catcctgact     1800
   cagctgaaga ttctcagacc tcaggtgttt agcatgagct ttaacagaca taatctgaaa     1860
   tactatgtat taccgaaaaa gcctaaaaag gtggcatttg attgcctaga atggatcaga     1920
   aagcaccacc catatgattc agggataatt tactgcctct ccaggcgaga atgtgacacc     1980
   atggctgaca cgttacagag agatgggctc gctgctcttg cttaccatgc tggcctcagt     2040
   gattctgcca gagatgaagt gcagcagaag tggattaatc aggatggctg tcaggttatc     2100
   tgtgctacaa ttgcatttgg aatggggatt gacaaaccgg acgtgcgatt tgtgattcat     2160
   gcatctctcc ctaaatctgt ggagggttac taccaagaat ctggcagagc tggaagagat     2220
   ggggaaatat ctcactgcct gcttttctat acctatcatg atgtgaccag actgaaaaga     2280
   cttataatga tggaaaaaga tggaaaccat catacaagag aaactcactt caataatttg     2340
   tatagcatgg tacattactg tgaaaatata acggaatgca ggagaataca gcttttggcc 2400
   tactttggtg aaaatggatt taatcctgat ttttgtaaga aacacccaga tgtttcttgt 2460
   gataattgct gtaaaacaaa ggattataaa acaagagatg tgactgacga tgtgaaaagt 2520
   attgtaagat ttgttcaaga acatagttca tcacaaggaa tgagaaatat aaaacatgta 2580
   ggtccttctg gaagatttac tatgaatatg ctggtcgaca ttttcttggg gagtaagagt 2640
   gcaaaaatcc agtcaggtat atttggaaaa ggatctgctt attcacgaca caatgccgaa 2700
   agacttttta aaaagctgat acttgacaag attttggatg aagacttata tatcaatgcc 2760
   aatgaccagg cgatcgctta tgtgatgctc ggaaataaag cccaaactgt actaaatggc 2820
   aatttaaagg tagactttat ggaaacagaa aattccagca gtgtgaaaaa acaaaaagcg 2880
   ttagtagcaa aagtgtctca gagggaagag atggttaaaa aatgtcttgg agaacttaca 2940
   gaagtctgca aatctctggg gaaagttttt ggtgtccatt acttcaatat ttttaatacc 3000
   gtcactctca agaagcttgc agaatcttta tcttctgatc ctgaggtttt gcttcaaatt 3060
   gatggtgtta ctgaagacaa actggaaaaa tatggtgcgg aagtgatttc agtattacag 3120
   aaatactctg aatggacatc gccagctgaa gacagttccc cagggataag cctgtccagc 3180
   agcagaggcc ccggaagaag tgccgctgag gagcttgacg aggaaatacc cgtatcttcc 3240
   cactactttg caagtaaaac cagaaatgaa aggaagagga aaaagatgcc agcctcccaa 3300
   aggtctaaga ggagaaaaac tgcttccagt ggttccaagg caaagggggg gtctgccaca 3360
   tgtagaaaga tatcttccaa aacgaaatcc tccagcatca ttggatccag ttcagcctca 3420
   catacttctc aagcgacatc aggagccaat agcaaattgg ggattatggc tccaccgaag 3480
   cctataaata gaccgtttct taagccttca tatgcattct 3520
<210> 161
   <211> 1954
   <212> DNA
   <213> Artificial Sequence
<220>
   <221> source
   <223> /note="Description of Artificial Sequence: Synthetic polynucleotide"
<400> 161
   cataaggggg aggctaactg aaacacggaa ggagacaata ccggaaggaa cccgcgctat        60
   gacggcaata aaaagacaga ataaaacgca cgggtgttgg gtcgtttgtt cataaacgcg       120
   gggttcggtc ccagggctgg cactctgtcg ataccccacc gagaccccat tggggccaat       180
   acgcccgcgt ttcttccttt tccccacccc accccccaag ttcgggtgaa ggcccagggc        240
   tcgcagccaa cgtcggggcg gcaggccctg ccatagcaga tctgcgcagc tggggctcta       300
   gggggtatcc ccacgcgccc tgtagcggcg cattaagcgc ggcgggtgtg gtggttacgc       360
   gcagcgtgac cgctacactt gccagcgccc tagcgcccgc tcctttcgct ttcttccctt 420
   cctttctcgc cacgttcgcc ggctttcccc gtcaagctct aaatcggggc atccctttag 480
   ggttccgatt tagtgcttta cggcacctcg accccaaaaa acttgattag ggtgatggtt 540
   cacgtagtgg gccatcgccc tgatagacgg tttttcgccc tttgacgttg gagtccacgt 600
   tctttaatag tggactcttg ttccaaactg gaacaacact caaccctatc tcggtctatt 660
   cttttgattt ataagggatt ttggggattt cggcctattg gttaaaaaat gagctgattt 720
   aacaaaaatt taacgcgaat taattctgtg gaatgtgtgt cagttagggt gtggaaagtc 780
   cccaggctcc ccagcaggca gaagtatgca aagcatgcat ctcaattagt cagcaaccag 840
   gtgtggaaag tccccaggct ccccagcagg cagaagtatg caaagcatgc atctcaatta 900
   gtcagcaacc atagtcccgc ccctaactcc gcccatcccg cccctaactc cgcccagttc 960
   cgcccattct ccgccccatg gctgactaat tttttttatt tatgcagagg ccgaggccgc 1020
   ctctgcctct gagctattcc agaagtagtg aggaggcttt tttggaggcc taggcttttg 1080
   caaaaagctc ccgggagctt gtatatccat tttcggatct gatcaagaga caggatgagg 1140
   atcgtttcgc atgattgaac aagatggatt gcacgcaggt tctccggccg cttgggtgga 1200
   gaggctattc ggctatgact gggcacaaca gacaatcggc tgctctgatg ccgccgtgtt 1260
   ccggctgtca gcgcaggggc gcccggttct ttttgtcaag accgacctgt ccggtgccct 1320
   gaatgaactg caggacgagg cagcgcggct atcgtggctg gccacgacgg gcgttccttg 1380
   cgcagctgtg ctcgacgttg tcactgaagc gggaagggac tggctgctat tgggcgaagt 1440
   gccggggcag gatctcctgt catctcacct tgctcctgcc gagaaagtat ccatcatggc 1500
   tgatgcaatg cggcggctgc atacgcttga tccggctacc tgcccattcg accaccaagc 1560
   gaaacatcgc atcgagcgag cacgtactcg gatggaagcc ggtcttgtcg atcaggatga 1620
   tctggacgaa gagcatcagg ggctcgcgcc agccgaactg ttcgccaggc tcaaggcgcg 1680
   catgcccgac ggcgaggatc tcgtcgtgac ccatggcgat gcctgcttgc cgaatatcat 1740
   ggtggaaaat ggccgctttt ctggattcat cgactgtggc cggctgggtg tggcggaccg 1800
   ctatcaggac atagcgttgg ctacccgtga tattgctgaa gagcttggcg gcgaatgggc 1860
   tgaccgcttc ctcgtgcttt acggtatcgc cgctcccgat tcgcagcgca tcgccttcta 1920
   tcgccttctt gacgagttct tctgaatggg gata 1954
<210> 162
   <211> 5082
   <212> DNA
   <213> Artificial Sequence
<220>
   <221> source
   <223> /note="Description of Artificial Sequence: Synthetic polynucleotide"
<400> 162
   cagggattgc tacaatttat caaagaagct tcagaaccca tccatgtgag gaagtataaa      60
   gggcaggtag tagctgtgga tacatattgc tggcttcaca aaggagctat tgcttgtgct     120
   gaaaaactag ccaaaggtga acctactgat aggtatgtag gattttgtat gaaatttgta     180
   aatatgttac tatctcatgg gatcaagcct attctcgtat ttgatggatg tactttacct     240
   tctaaaaagg aagtagagag atctagaaga gaaagacgac aagccaatct tcttaaggga     300
   aagcaacttc ttcgtgaggg gaaagtctcg gaagctcgag agtgtttcac ccggtctatc     360
   aatatcacac atgccatggc ccacaaagta attaaagctg cccggtctca gggggtagat     420
   tgcctcgtgg ctccctatga agctgatgcg cagttggcct atcttaacaa agcgggaatt     480
   gtgcaagcca taattacaga ggactcggat ctcctagctt ttggctgtaa aaaggtaatt     540
   ttaaagatgg accagtttgg aaatggactt gaaattgatc aagctcggct aggaatgtgc     600
   agacagcttg gggatgtatt cacggaagag aagtttcgtt acatgtgtat tctttcaggt     660
   tgtgactacc tgtcatcact gcgtgggatt ggattagcaa aggcatgcaa agtcctaaga     720
   ctagccaata atccagatat agtaaaggtt atcaagaaaa ttggacatta tctcaagatg     780
   aatatcacgg taccagagga ttacatcaac gggtttattc gggccaacaa taccttcctc     840
   tatcagctag tttttgatcc catcaaaagg aaacttattc ctctgaacgc ctatgaagat     900
   gatgttgatc ctgaaacact aagctacgct gggcaatatg ttgatgattc catagctctt     960
   caaatagcac ttggaaataa agatataaat acttttgaac agatcgatga ctacaatcca    1020
   gacactgcta tgcctgccca ttcaagaagt cgtagttggg atgacaaaac atgtcaaaag    1080
   tcagctaatg ttagcagcat ttggcatagg aattactctc ccagaccaga gtcgggtact    1140
   gtttcagatg ccccacaatt gaaggaaaat ccaagtactg tgggagtgga acgagtgatt    1200
   agtactaaag ggttaaatct cccaaggaaa tcatccattg tgaaaagacc aagaagtgca    1260
   gagctgtcag aagatgacct gttgagtcag tattctcttt catttacgaa gaagaccaag    1320
   aaaaatagct ctgaaggcaa taaatcattg agcttttctg aagtgtttgt gcctgacctg    1380
   gtaaatggac ctactaacaa aaagagtgta agcactccac ctaggacgag aaataaattt    1440
   gcaacatttt tacaaaggaa aaatgaagaa agtggtgcag ttgtggttcc agggaccaga    1500
   agcaggtttt tttgcagttc agattctact gactgtgtat caaacaaagt gagcatccag    1560
   cctctggatg aaactgctgt cacagataaa gagaacaatc tgcatgaatc agagtatgga    1620
   gaccaagaag gcaagagact ggttgacaca gatgtagcac gtaattcaag tgatgacatt    1680
   ccgaataatc atattccagg tgatcatatt ccagacaagg caacagtgtt tacagatgaa    1740
   gagtcctact cttttaagag cagcaaattt acaaggacca tttcaccacc cactttggga    1800
   acactaagaa gttgttttag ttggtctgga ggtcttggag atttttcaag aacgccgagc 1860
   ccctctccaa gcacagcatt gcagcagttc cgaagaaaga gcgattcccc cacctctttg 1920
   cctgagaata atatgtctga tgtgtcgcag ttaaagagcg aggagtccag tgacgatgag 1980
   tctcatccct tacgagaagg ggcatgttct tcacagtccc aggaaagtgg agaattctca 2040
   ctgcagagtt caaatgcatc aaagctttct cagtgctcta gtaaggactc tgattcagag 2100
   gaatctgatt gcaatattaa gttacttgac agtcaaagtg accagacctc caagctatgt 2160
   ttatctcatt tctcaaaaaa agacacacct ctaaggaaca aggttcctgg gctatataag 2220
   tccagttctg cagactctct ttctacaacc aagatcaaac ctctaggacc tgccagagcc 2280
   agtgggctga gcaagaagcc ggcaagcatc cagaagagaa agcatcataa tgccgagaac 2340
   aagccggggt tacagatcaa actcaatgga gctctggaaa aactttggat ttaagcggga 2400
   ctctggggtt cgcgaaatga ccgaccaagc gacgcccaac ctgccatcac gagatttcga 2460
   ttccaccgcc gccttctatg aaaggttggg cttcggaatc gttttccggg acgccggctg 2520
   gatgatcctc cagcgcgggg atctcatgct ggagttcttc gcccacccca acttgtttat 2580
   tgcagcttat aatggttaca aataaagcaa tagcatcaca aatttcacaa ataaagcatt 2640
   tttttcactg cattctagtt gtggtttgtc caaactcatc aatgtatctt atcatgtctg 2700
   tataccgtcg acctctagct agagcttggc gtaatcatgg tcatagctgt ttcctgtgtg 2760
   aaattgttat ccgctcacaa ttccacacaa catacgagcc ggaagcataa agtgtaaagc 2820
   ctggggtgcc taatgagtga gctaactcac attaattgcg ttgcgctcac tgcccgcttt 2880
   ccagtcggga aacctgtcgt gccagctgca ttaatgaatc ggccaacgcg cggggagagg 2940
   cggtttgcgt attgggcgct cttccgcttc ctcgctcact gactcgctgc gctcggtcgt 3000
   tcggctgcgg cgagcggtat cagctcactc aaaggcggta atacggttat ccacagaatc 3060
   aggggataac gcaggaaaga acatgtgagc aaaaggccag caaaaggcca ggaaccgtaa 3120
   aaaggccgcg ttgctggcgt ttttccatag gctccgcccc cctgacgagc atcacaaaaa 3180
   tcgacgctca agtcagaggt ggcgaaaccc gacaggacta taaagatacc aggcgtttcc 3240
   ccctggaagc tccctcgtgc gctctcctgt tccgaccctg ccgcttaccg gatacctgtc 3300
   cgcctttctc ccttcgggaa gcgtggcgct ttctcaatgc tcacgctgta ggtatctcag 3360
   ttcggtgtag gtcgttcgct ccaagctggg ctgtgtgcac gaaccccccg ttcagcccga 3420
   ccgctgcgcc ttatccggta actatcgtct tgagtccaac ccggtaagac acgacttatc 3480
   gccactggca gcagccactg gtaacaggat tagcagagcg aggtatgtag gcggtgctac 3540
   agagttcttg aagtggtggc ctaactacgg ctacactaga aggacagtat ttggtatctg 3600
   cgctctgctg aagccagtta ccttcggaaa aagagttggt agctcttgat ccggcaaaca 3660
   aaccaccgct ggtagcggtg gtttttttgt ttgcaagcag cagattacgc gcagaaaaaa     3720
   aggatctcaa gaagatcctt tgatcttttc tacggggtct gacgctcagt ggaacgaaaa     3780
   ctcacgttaa gggattttgg tcatgagatt atcaaaaagg atcttcacct agatcctttt     3840
   aaattaaaaa tgaagtttta aatcaatcta aagtatatat gagtaaactt ggtctgacag     3900
   ttaccaatgc ttaatcagtg aggcacctat ctcagcgatc tgtctatttc gttcatccat     3960
   agttgcctga ctccccgtcg tgtagataac tacgatacgg gagggcttac catctggccc     4020
   cagtgctgca atgataccgc gagacccacg ctcaccggct ccagatttat cagcaataaa     4080
   ccagccagcc ggaagggccg agcgcagaag tggtcctgca actttatccg cctccatcca     4140
   gtctattaat tgttgccggg aagctagagt aagtagttcg ccagttaata gtttgcgcaa     4200
   cgttgttgcc attgctacag gcatcgtggt gtcacgctcg tcgtttggta tggcttcatt     4260
   cagctccggt tcccaacgat caaggcgagt tacatgatcc cccatgttgt gcaaaaaagc     4320
   ggttagctcc ttcggtcctc cgatcgttgt cagaagtaag ttggccgcag tgttatcact     4380
   catggttatg gcagcactgc ataattctct tactgtcatg ccatccgtaa gatgcttttc     4440
   tgtgactggt gagtactcaa ccaagtcatt ctgagaatag tgtatgcggc gaccgagttg     4500
   ctcttgcccg gcgtcaatac gggataatac cgcgccacat agcagaactt taaaagtgct     4560
   catcattgga aaacgttctt cggggcgaaa actctcaagg atcttaccgc tgttgagatc     4620
   cagttcgatg taacccactc gtgcacccaa ctgatcttca gcatctttta ctttcaccag     4680
   cgtttctggg tgagcaaaaa caggaaggca aaatgccgca aaaaagggaa taagggcgac     4740
   acggaaatgt tgaatactca tactcttcct ttttcaatat tattgaagca tttatcaggg     4800
   ttattgtctc atgagcggat acatatttga atgtatttag aaaaataaac aaataggggt     4860
   tccgcgcaca tttccccgaa aagtgccacc tgacgtcgac ggatcgggag atctcccgat     4920
   cccctatggt cgactctcag tacaatctgc tctgatgccg catagttaag ccagtatctg     4980
   ctccctgctt gtgtgttgga ggtcgctgag tagtgcgcga gcaaaattta agctacaaca     5040
   aggcaaggct tgaccgacaa ttgcatgaag aatctgctta gg                        5082